# EUROPEAN PATENT SPECIFICATION

(11) **EP 3 524 608 B1**
(45) Date of publication and mention of the grant of the patent: **30.12.2020**
(21) Application number: 17856618.8
(22) Date of filing: 15.09.2017
(51) Int. Cl.: C07D 487/22, C07D 487/04, A61K 31/5517, A61P 35/00

(54) **NOVEL PYRIMIDINE DERIVATIVE COMPOUND OR PHARMACEUTICALLY ACCEPTABLE SALTS THEREOF, PREPARATION METHOD THEREOF, AND PHARMACEUTICAL COMPOSITION USING SAME**
NEUARTIGE PYRIMIDINDERIVATVERBINDUNG ODER PHARMAZEUTISCH AKZEPTABLE SALZE DAVON, HERSTELLUNGSVERFAHREN DAFÜR UND PHARMAZEUTISCHE ZUSAMMENSETZUNG DAMIT
NOUVEAU COMPOSÉ DÉRIVÉ DE PYRIMIDINE OU SELS PHARMACEUTIQUEMENT ACCEPTABLES DE CELUI-CI, SON PROCÉDÉ DE PRODUCTION ET COMPOSITION PHARMACEUTIQUE LE COMPRENANT

(30) Priority: 30.09.2016 KR 20160127045
(43) Date of publication of application: 14.08.2019
(73) Proprietor: Spark Biopharma, Inc., Seoul 08826 (KR)
(72) Inventor: PARK, Seung Bum, Seoul 04427 (KR); KIM, Jong Hoon, Seoul 08799 (KR)
(74) Representative: Snaith, James Michael
(86) International application number: PCT/KR2017/010120
(87) International publication number: WO 2018/062732

(56) References cited:
- WO-A1-93/08171
- WO-A1-2013/064231
- WO-A2-2004/030635
- HEANEY, F. et al.: "Pyrimidine Annotated Heterocycles-synthesis and Cycloaddition of the First Pyrimido[1,4]diazepine N-oxides", Perkinl: An International Journal of Organic and Bio-organic Chemistry, vol. 6, 2001, pages 622-632, XP002254480,
- HUDDLESTON, N. E. et al.: "Synthesis of 2-amino-4-chloro-6,9-bis-(2,4-dimethoxy benzyl)-6,7,8,9-tetrahydro-5H-pyrimido[4,5 -e][1,4]diazepine. A Potentially Useful Intermediate to Pyrimido[4,5-e][1,4]diazepine-based Folates", Heterocyclic Communications, vol. 10, no. 6, 2004, pages 405-406, XP009513879, ISSN: 0793-0283, DOI: 10.1515/HC.2004.10.6.405
- KIM, J. et al.: "Diversity-oriented Synthetic Strategy for Developing a Chemical Modulator of Protein-protein Interaction", Nature Communications, vol. 7, 24 October 2016 (2016-10-24), pages 1-10, XP055498367,

## Description

### Technical Field

The present invention is related to a novel pyrimidine derivative compound or a pharmaceutically acceptable salt thereof, a processs for preparing the same, and a pharmaceutical composition using the same.

### Background Art

Leucyl-tRNA synthetase (LRS) catalyses the conjugation of Leucine(Leu) to its cognate tRNA to form an aminoacyl-tRNA, which serves as a precursor for protein biosynthesis. That is, LRS plays a canonical role in protein biosynthesis. Further, it was known that four types of Rag proteins are expressed in human wherein RagD alone is interacted with LRS.

To investigate specific modulators for targets such as protein-protein interaction (hereinafter, 'PPI') based on the mechanism, it was attempted to identify novel chemical inhibitors of the LRS-Ras-related GTP-binding protein D (RagD) interaction for the target regulation of rapamycin complex (mTORC1) pathway - a dominant effector that regulates cellular growth, metabolism, proliferation, and survival. Upon activation of mTORC1 by multiple upstream inputs such as growth factors, energy status, and amino acids, particularly the branched-chain amino acid, leucine (Leu), mTORC1 plays a crucial role in triggering eukaryotic cell growth and proliferation through stimulating protein biosynthesis and other anabolic processes while suppressing a catabolic process, for example, autophagy. Consequently, the dysregulation of mTORC1 accelerates biological pathways that could lead to cancer cell growth, survival, and proliferation. Accordingly, to find a specific and selective inhibitor of mTORC1 is a very important purpose for treating a cancer.

Currently, the LRS-RagD interaction and human LRS and RagD have not yet been fully characterized while neither method nor material for inhibiting the binding between LRS and RagD was known. Given this, the discovery of novel small-molecule PPI inhibitors of LRS and RagD could shed light on the molecular mechanism of mTORC1 activation in a nutrition-dependent manner.

Traditional synthetic organic chemistry has been advanced with the development of target-oriented synthesis. Many organic chemists have conceived and developed various new reactions through the synthesis of diverse natural products. Since the target-oriented synthesis is configured to target and synthesize a specific natural product, the obtained compound may be represented as one point in view of a compound structure space. Thus, target oriented synthesis seems to be very limited in terms of the diversity of compound.

Many efforts have thus been made to track down more phisiologically active compounds by improving specific chemical properties of the obtained compound, resulting in the advent and development of combinatorial chemical synthesis.

Combinatorial chemical synthesis is a new synthetic method for the development of new materials. Whereas conventional organic synthesis methods can require a single reaction for the synthesis of one kind of compound, combinatorial chemical synthesis is a highly efficient chemical synthesis to synthesize more various and numerous compounds at the same time or to automate the multi-step synthetic process. With the introduction of combinatorial chemical synthesis, it has become easier to screen biological hit and lead compounds of new structures and to optimize the structure and activity thereof. Combinatorial chemical synthesis has been mainly studied in medicinal chemistry, particularly greatly contributing to the study of structure-activity relationship, and also has allowed for various substitution reactions in a specific skeletal structure, ensuring diversity of compounds.

Developed as a different concept, diversity-oriented synthesis is configured to synthesize a collection of structurally-diverse compounds distributed in a wide range of the compound structure space and then to search for new various physiologically active compounds through high throughput screening (HTS).

In diversity-oriented synthesis, compounds having different core skeletons can be prepared at the same time, and can be constructed into a library, whereby various different active compounds can be identified by various screening methods.

Korean patent publication No. 10-2012-0093940 discloses a compound having a structure of pyrimidodiazepine. However, this is silent on the inhibition activity of the binding between LRS and RagD.

Accordingly, based on diversity-oriented synthesis, the present inventors have synthesized a new pyrimidine derivative compound having an activity inhibiting the binding between LRS and RagD to arrive in the present invention.

HEANEet al.: "Pyrimidine Annotated Heterocycles-synthesis and Cycloaddition of the First Pyrimido[1,4]diazepine N-oxides", Perkin Trans J, vol. 6, 2001, pages 622-632 discloses pyrimido[1,4]diazepine *N*-oxides.

### Disclosure of Invention

### Technical Problem

The purpose of the present invention is to provide a novel pyrimidine derivative compound or a pharmaceutically acceptable salt thereof.

The purpose of the present invention is to provide a process for preparing the novel pyrimidine derivative compound or a pharmaceutically acceptable salt thereof.

The purpose of the present invention is to provide a pharmaceutical composition using the novel pyrimidine derivative compound or a pharmaceutically acceptable salt thereof.

### Solution to Problem

The present invention provides a pyrimidine derivative compound represented by any one of formulas I to IX: 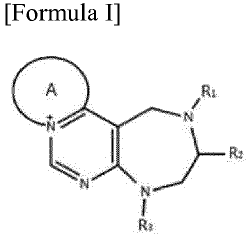
wherein A forms a heterobicyclic ring together with adjacent N and C atoms wherein the heterobicyclic ring is an 8- to 9-membered bicyclic ring which may contain 1 to 3 nitrogen atoms; R₁ is -Boc (tert-butyloxycarbonyl) or a benzyl group (Bn); R₂ is -CH₂-O-TIPS; and R₃ is hydrogen, a C₁ to C₃ alkyl group, an allyl group, -Tf, a C₁ to C₃ alkyl benzyl, or 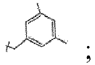 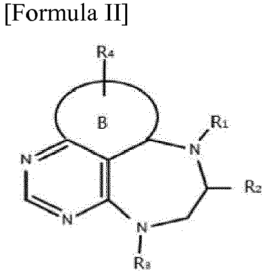
wherein B forms a 6- to 10-membered ring together with adjacent 2 to 3 C atoms wherein the ring may contain 1 to 3 nitrogen atoms; R₁ is -Boc or a benzyl group; R₂ is -CH₂-O-TIPS; and R₃ is hydrogen, a C₁ to C₃ alkyl group, an allyl group, -Tf, a C₁ to C₃ alkyl benzyl group, or 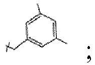 and R₄ is a C₁ to C₃ alkyl group or -CH₂-PMB; 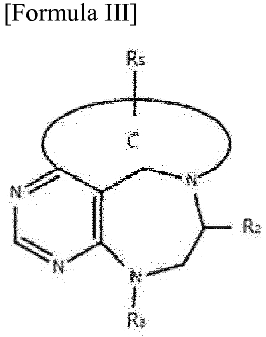
wherein C forms a 10- to 14-membered ring together with adjacent N and C atoms wherein the ring may contain 1 to 3 nitrogen atoms; R₂ is -CH₂-O-TIPS; R₃ is hydrogen, a C₁ to C₃ alkyl group, an allyl group, -Tf, a C₁ to C₃ alkyl benzyl group, or 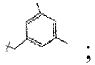 and R₅ is a C₁ to C₃ alkyl group or -CH₂-PMB; 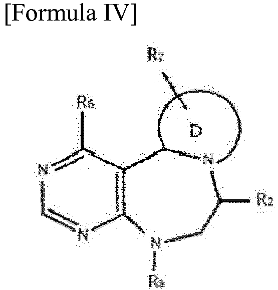
wherein D forms a 4- to 6-membered cyclic imide together with adjacent N and C atoms wherein the cyclic imide is a ring which may contain 1 to 3 nitrogen atoms; R₂ is - CH₂-O-TIPS; R₃ is hydrogen, a -C₁ to C₃ alkyl group, an allyl group, -Tf, a C₁ to C₃ alkyl benzyl group, or 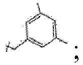 R₆ is 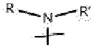 wherein R and R' are each independently a benzyl group or a C₁ to C₃ alkyl group; and R₇ is a benzyl group, a phenyl group, or 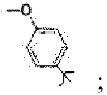 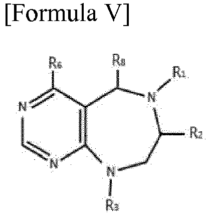
wherein R₁ is -Boc or a benzyl group; R₂ is -CH₂-O-TIPS; and R₃ is hydrogen, a C₁ to C₃ alkyl group, an allyl group, -Tf, a C₁ to C₃ alkyl benzyl group, or 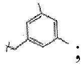 R₆ is 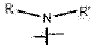 wherein R and R' are each independently a benzyl group or a C₁ to C₃ alkyl group; and R₈ is an acetylene group; 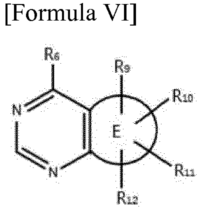
wherein E forms an 8- to 10-membered heterocyclic ring together with adjacent two C atoms wherein the heterocyclic ring is a saturated or unsaturated ring which contains at least two of 2 to 4 nitrogen atoms and 1 to 3 oxigen atoms; R₆ is 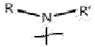 wherein R and R' are each independently a benzyl group or a C₁ to C₃ alkyl group; and R₉ to R₁₂ are each independently hydrogen, a methyl group, an azide group, a benzyl group, -CH₂-O-TIPS or-Tf; 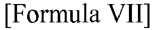 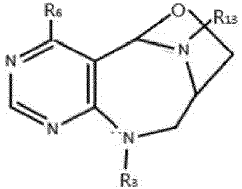
wherein R₃ is hydrogen, a -C₁ to C₃ alkyl group, an allyl group, -Tf, a C₁ to C₃ alkyl benzyl group, or 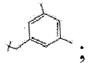 R₆ is 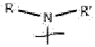 wherein R and R' are each independently a benzyl group or a C₁ to C₃ alkyl group; and R₁₃ is -Boc, -CONHBn, -m-Ns; 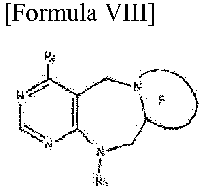
wherein F is -X-(CH₂)n-(O-CH₂)m- and forms a 3- to 6-membered heterocyclic ring together with adjacent N and C atoms wherein X is 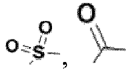 or -CH₂-, and n and m are respectively an integer from 0 to 2; R₃ is hydrogen, a -C₁ to C₃ alkyl group, an allyl group,-Tf, a C₁ to C₃ alkyl benzyl group, or 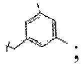 and R₆ is 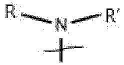 wherein R and R' are each independently a benzyl group or a C₁ to C₃ alkyl group; 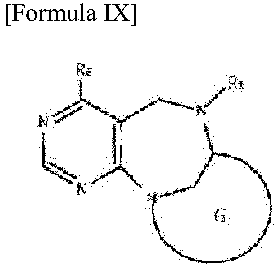
wherein G forms a 6- to 9-membered heterocyclic ring together with adjacent N and C atoms wherein the heterocyclic ring is an unsaturated heterocyclic ring which contains 2 to 3 nitrogen atoms; R₁ is -Boc or a benzyl group ; and R₆ is 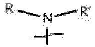 wherein R and R' are each independently a benzyl group or a C₁ to C₃ alkyl group;
or a pharmaceutically acceptable salt thereof.

The present invention provides a process for preparing the pyrimidine derivative compound of formulas I to IX or a pharmaceutically acceptable salt thereof, characterized in comprising:
step 1 for preparing an intermediate substance, tert-(S)-(1-(6-chloro-5-formylpyrimidin-4-yl)amino)-3-((triisopropylsilyl)oxy)propan-2-yl)carbamate;
step 1a for preparing a starting material represented by formula 1 by reacting the intermediate substance prepared in step 1 with cyclic amine or tetrakis palladium; and 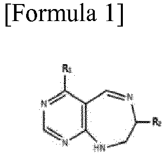 wherein R₁ is 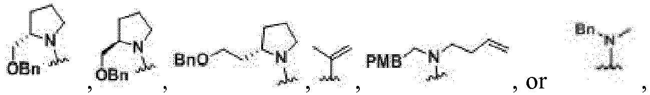 R₂ is - CH₂-O-TIPS;
step 2 for preparing a desired compound from the starting material prepared in step 1a.

The present invention provides a composition for treating cancer comprising the pyrimidine derivative compound of formulas I to IX or a pharmaceutically acceptable salt thereof as an active ingredient.

### Effect of Invention

The novel pyrimidine derivative compound of the present invention exerts excellent toxicity against cencer cells, based on which it can be used as a treating agent for cancer diseases. Further, the compound can be synthesized based on the preparation process according to the present invention in a relatively simple manner.

### Brief Description of the Drawings

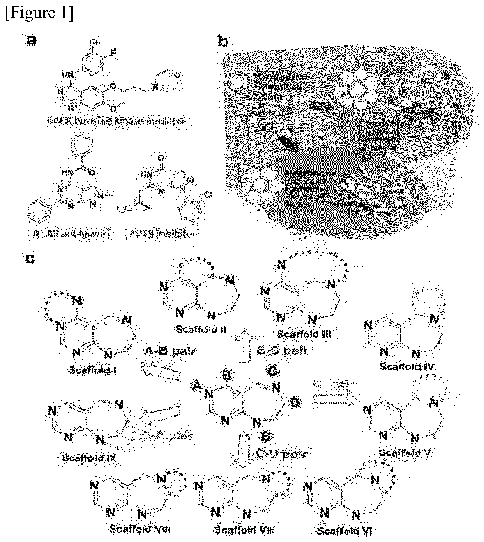

Fig. 1 shows diversity-oriented synthetic strategy with pyrimidine as a privileged structure.
   a) shows pyrimidine-containing bioactive compounds.
   b) shows chemical space occupied by 3-D space structure of pyrimidine and pyrimidine-containing tricyclic 6/6/6 and 6/7/6 systems in terms of diversity and complexity of 3-D space structure.
   c) shows synthetic strategy of pyrimidodiazepine- or pyrimidine-containing pyrimidine derivative.

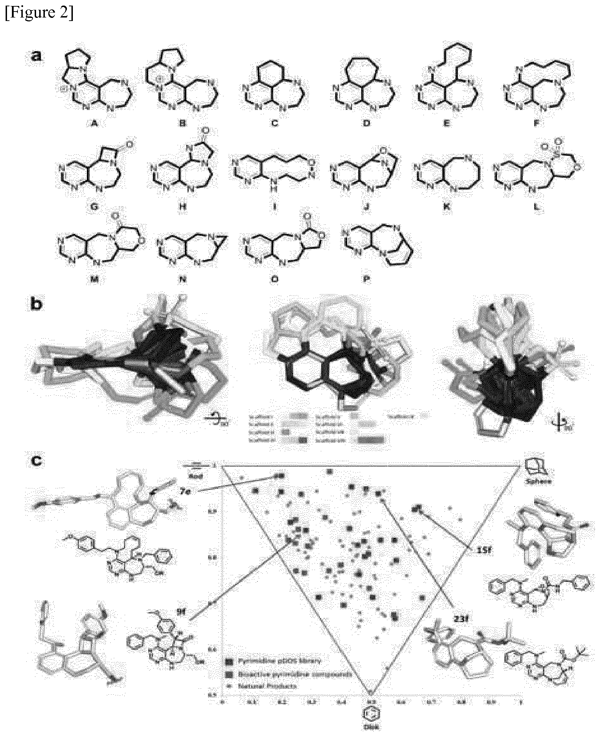

Fig. 2 shows chemoinformatic analysis for structural diversity and complexity of 3D of pDOS library.
   a) depicts 16 core skeletons containing pyrimidine- or pyrimidodiazepine.
   b) depicts overlay of energy-minimized conformers of 16 core skeletons containing the pyrimidine structure.
   c) is a PMI analysis drawing. The 3D structure of pDOS library in red squares was quantitatively compared with that of 15 FDA-approved drugs containing pyrimidine moiety in blue squares and bioactive natural products in grey dots.
Fig. 3 shows X-ray structure of compound 16f (Deposition number in Cambridge Structural Database: CCDC1500596).

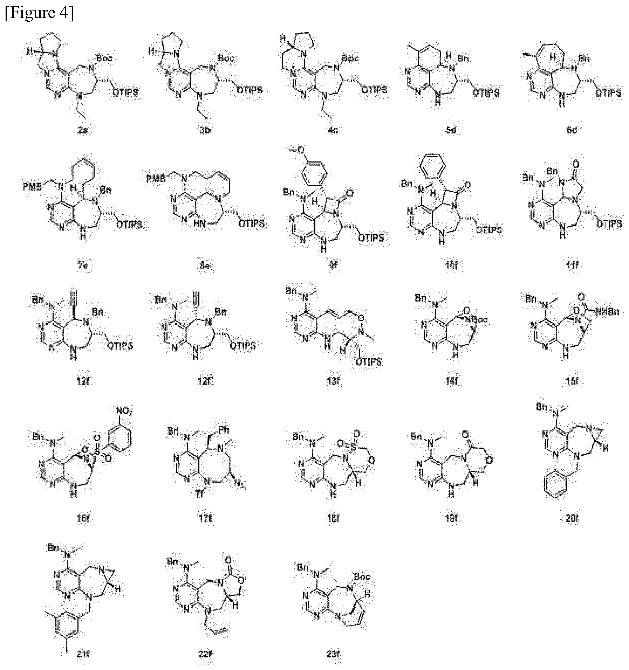

Fig. 4 shows pyrimidine diversity-oriented synthesis (pDOS) library used in PMI analysis.
Fig. 5 shows FDA-approved drugs embedded with pyrimidine moieties.
Figs. 6 to 14 depict analysis on COSY, HSQC and NOE spectra for some of desired compounds of Fig. 14 (i.e., compounds 5d, 6d, 7e, 9f, 10f, 12f, 12f, 17f, 17f).
Fig. 15 relate to experiments on discovery of chemical modulator for LRS-RagD interaction
   a) is a diagram relevant to experiments on discovery of chemical modulator for LRS-RagD interaction. It shows noncanonical role of LRS and Leucine-loaded LRS binds to RagD, which promotes the translocation of mTORC1 to lysosomal surface and subsequent activation.
   b) displays how LRS-RagD interaction affects mTORC1, showing the dose-response curves in ELISA of 20f and 21f wherein the results represent the mean of three replicates. This means that 20f and 21f inhibit the LRS-RagD interaction in a dose-dependent manner.
   c) Effects of 20f and 21f on mTORC1 signalling pathway. HEK293T cells were treated with 20 µM of 20f and 21f for 3 h, followed by western blotting to obtain the result as depicted. Cells deprived of leucine for 3 h was used as a control. The graph shows a relative level of phospho-T389 S6K1 to a DMSO-treated experimental group. The result represents the mean of five replicates. It was found that the cells treated with 21f showed a reduced amount of phospho-T389 S6K1, similarly to cells deprived of leucine, to inactivate mTORC1. The reduction degree of 20f is less than that of 21f.
   d) Effects of 21f on mTORC1 signalling pathway. HEK293Tcells were treated with 20, 10, 5 and 1 µM of compound 21f for 3 h. Cells deprived of leucine for 3 h and cells treated with 200 nM of rapamycin for 3h were used as a control. Phospho-T389 S6K1, phospho-S757 ULK1, and phospho-S65 4E-BP1 were reduced in cells treated with 21f in a dose-dependent manner, which shows that 21f well inhibits mTORC1. Contrary to this, no effects were found on phospho-T172 AMPKα and phospho-S473 Akt, from which it was confirmed that the compound selectively affects only mTORC1, without any effects on AMPK signaling pathways or mTORC2 signaling pathways.
   e) Effects of 21f on mTORC1 within cells were analyzed for 0-24 h. HEK293T cells were treated with 10 µM of 21f. Phospho-T389 S6K1, phospho-S757 ULK1, and phospho-S65 4E-BP1 were reduced in cells treated with 21f as time passed. The effect lasted for at least about 12 h. Further, no effects were found on phospho-T172 AMPKα and phospho-S473 Akt, from which it was confirmed that the compound selectively affects only mTORC1, without any effects on AMPK signaling pathways or mTORC2 signaling pathways.
   f) Analysis results of surface plasmon resonance (SPR) spectroscopy. 21f showed its concentration-dependent binding to LRS. The concentration of 21f as experimented were 1, 2.5, 5, 10, 12.5, 15, 17.5 and 20 µM. The curve fittings are shown in black. The results represent the mean of two replicates.

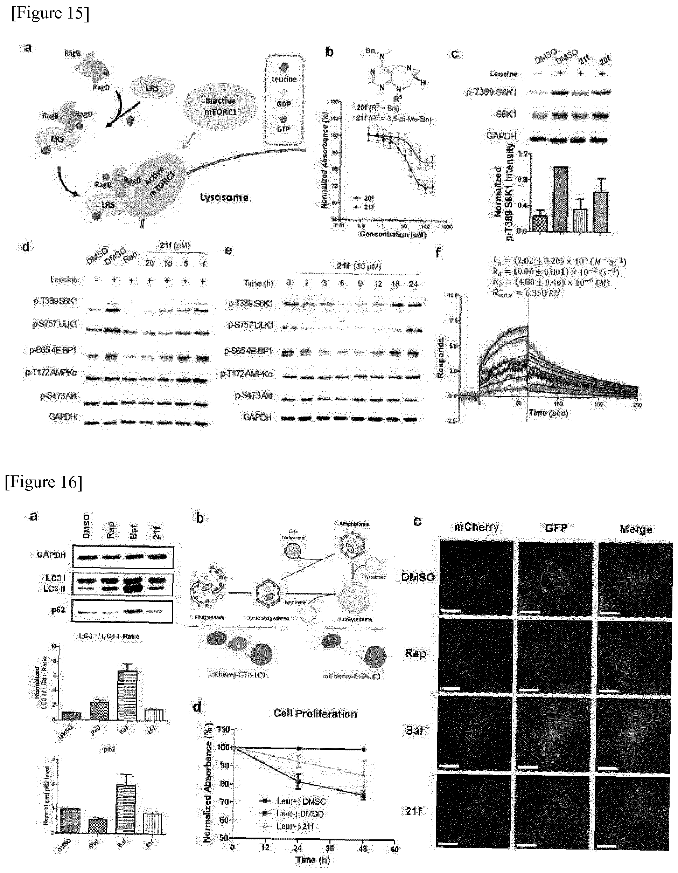

Fig. 16 shows autophagic activation of compound 21f and its inhibition activity of cell division.
   a) Western blot analysis of LC3 and p62. HeLa cells were treated with 200nM of rapamycin (Rap), 10nM of bafilomycin (Baf), or 20 µM of 21f for 6 h, followed by western blotting. The graph shows analysis results of western blotting of experimental and control groups based on LC3 I/LC3 II ratio and p62 level of the DMSO group. The results represent the mean of at least three replicates. Comparing with the DMSO group, rapamycin activates autophagic activity, lowering LC3 I/LC3 II ratio and reducing p62 level. Bafilomycin is a compound inhibiting autophagic activity by preventing formation of autolysosome. Similarly to rapamycin, 21f inhibits autophagic activity, lowering LC3 I/LC3 II ratio and reducing p62 level.
   b) Schematic depiction of mCherry-GFP-LC3 fluorescence image system. The pH decreases with lysosome fusion to autophagosome, thereby quenching GFP fluorescence while mCherry fluorescence not sensitive to pH is maintained.
   c) depicts mCherry and GFP fluorescence images. mCherry-GFP-LC3 genes are expressed in HeLa cells, treated with 200 nM of Rap, 10 nM of Baf, or 20 µM of 21f for 6 h, followed by obtaining an image through fluorescence microscope. Scale bar is 15 mm. Rapamycin inhibits autophagic activity, which results in combining lysosome with autophagosome to lower pH and lose GFP fluorescence, whereby red fluorescence of mCherry fluorescence alone remained. Bafilomycin inhibits combining lysosome with autophagosome, which results in maintaining pH and then GFP fluorescence, whereby mCherry fluorescence overlaps with GFP fluorescence to exert yellow color. Similarly to rapamycin, 21f exerts red fluorescence.
   d) shows the results where cellular division level in HEK293T cells was measured by BrdU assay. The Leu-deprived cells were used as a control. In an experimental group treated with 21f, cellular devision was inhibited as treatment time passed.

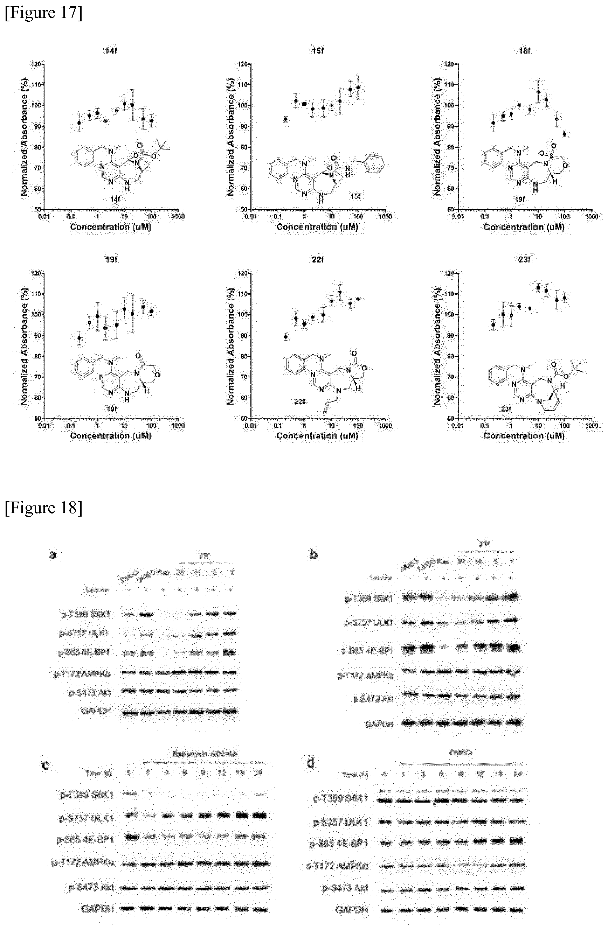

Fig. 17 shows dose-dependent graphs in ELISA with compounds 14f, 15f, 18f, 19f, 22f and 23f. Compounds embedded with pyrimidodiazepine skeleton other than 20f and 21f did not inhibit the LRS-RagD interaction in a dose-dependent manner.
Fig. 18 shows the results of western blotting of DMSO and Rapamycin (Rap) as a control and compound 21f.
   a) shows the results based on the experiment procedure of Fig. 15d, provided that it was carried out in Ca Ski cells.
   b) shows the results based on the experiment procedure of Fig. 15d, provided that it was carried out in DU145 cells.
      The results from the two cancer cell lines were same as those from HEK293T cells.
   c) shows the results based on the experiment procedure of Fig. 15e, provided that being treated with 500 nM of rapamycin.
   d) shows the results based on the experiment procedure of Fig. 15e, provided that being treated with DMSO.

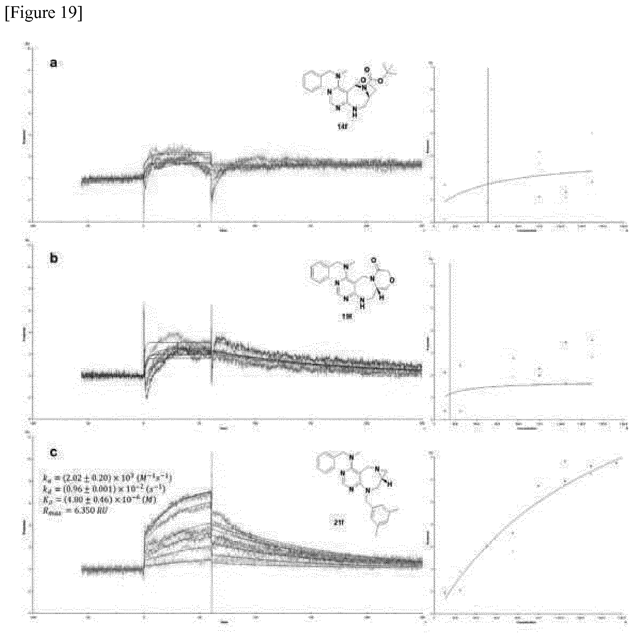

Fig. 19 depicts SPR analysis results showing concentration-dependent binding of compounds 14f, 19f, and 21f to purified LRS. 14f and 19f did not bind to LRS in a concentration-dependent manner, which is different from 21f.

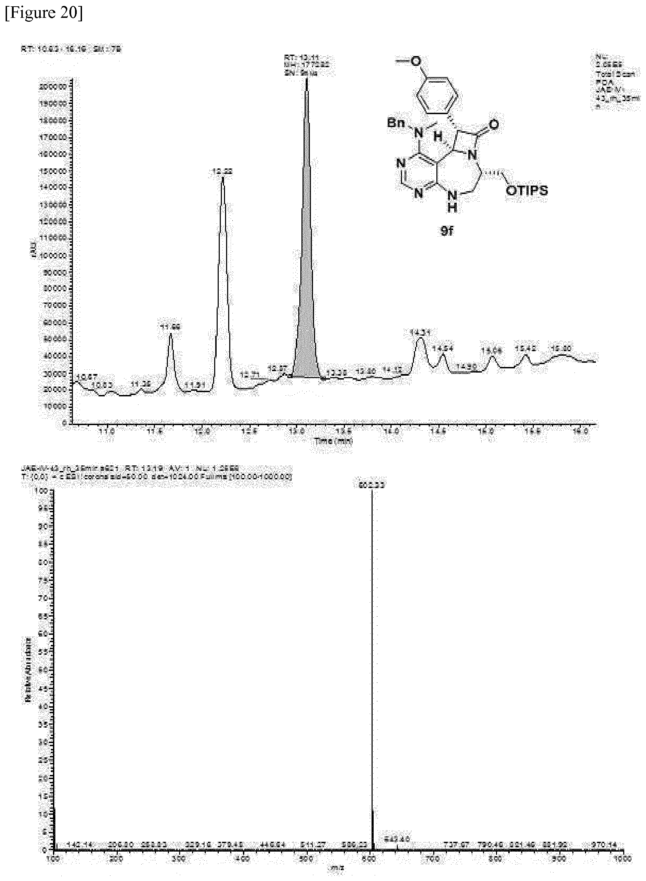

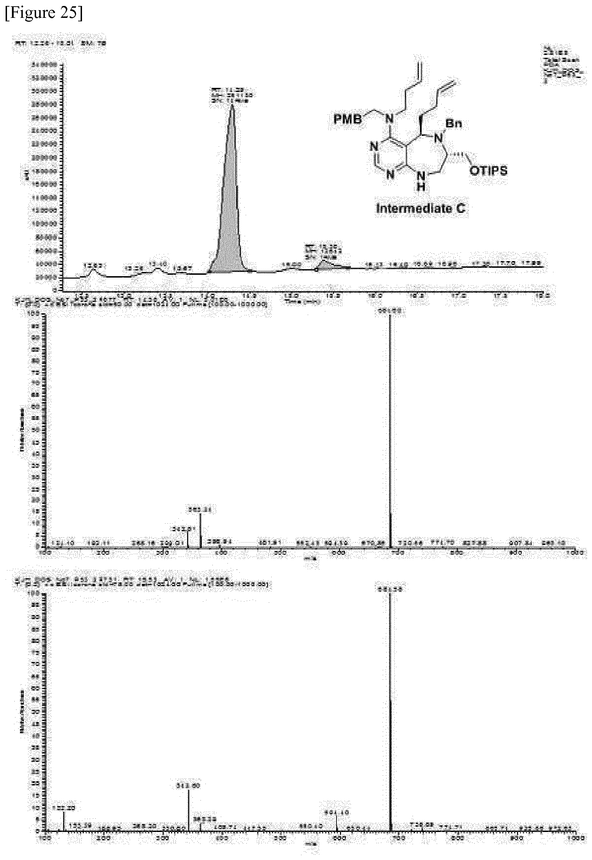

Figs. 20 and 25 depict LC-MS analysis results on compounds 9f and 10f, intermediate B (n=1 or 2),13f, intermediate C.

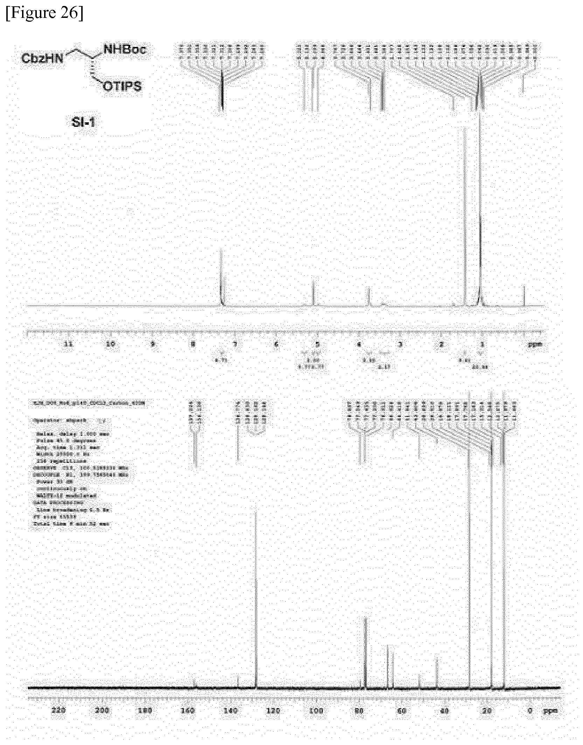

Figs. 26 to 62 depict ¹H and ¹³C NMR analysis results on desired compounds of the present invention (i.e., compound 2a to 23f), intermediate substance, or intermediate.

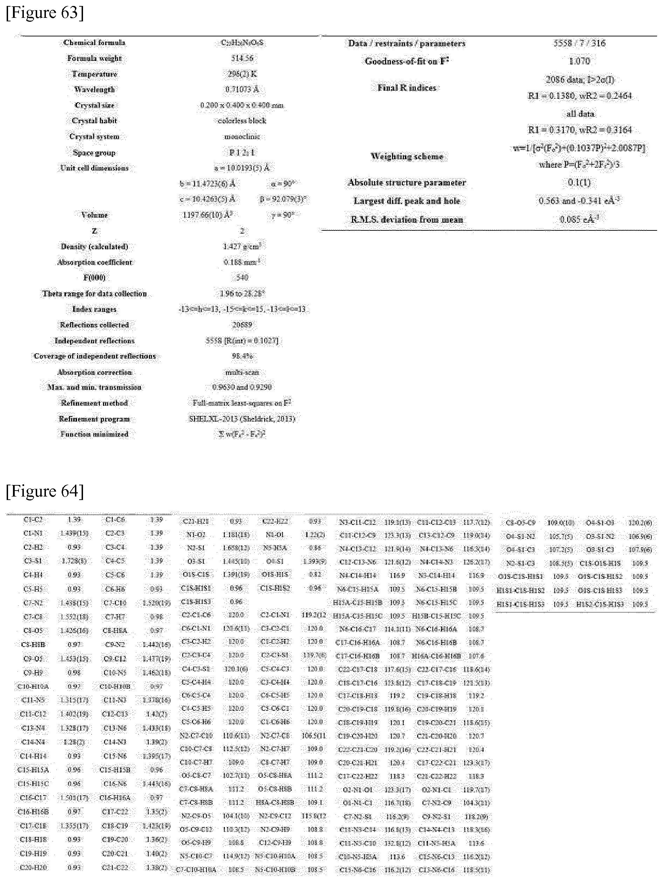

Fig. 63 shows crystal structure data of compound 16f.
Fig. 64 shows bond lengths (Å) and bond angles (°) for compound 16f.

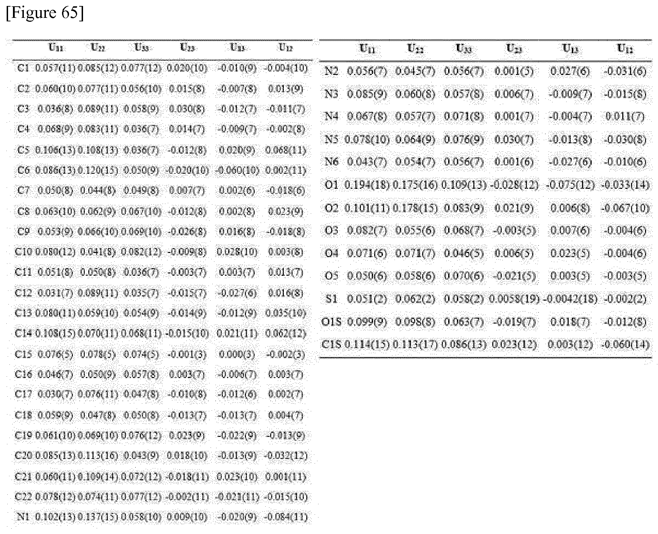

Fig. 65 shows anisotropic atomic displacement parameters (Å²) for compound 16f. The anisotropic atomic displacement factor was applied for the function: -2π²[h² a^{*2} U₁₁ + ... + 2 h k a^{*} b^{*} U₁₂].

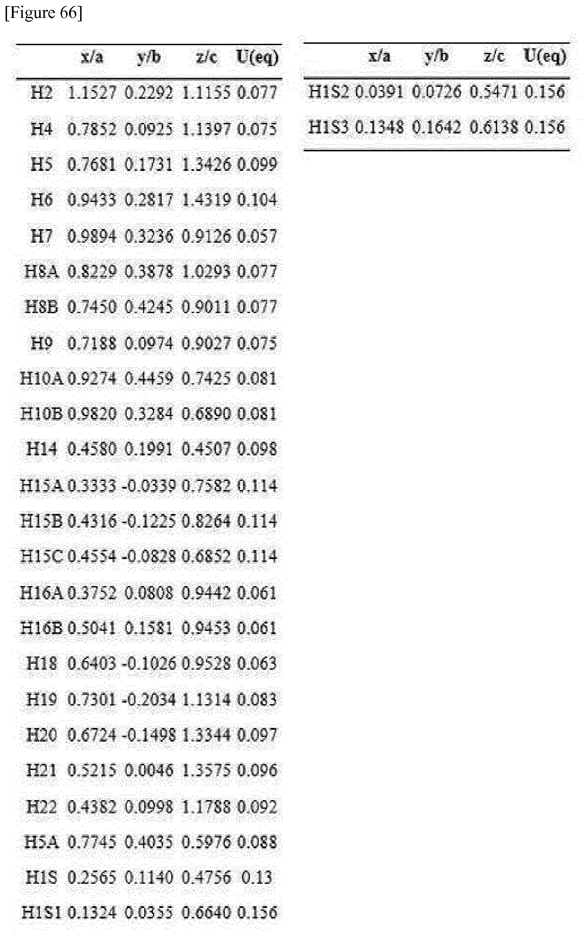

Fig. 66 shows hydrogen atomic coordinates and isotropic atomic displacement parameters (Å²) for compound 16f.

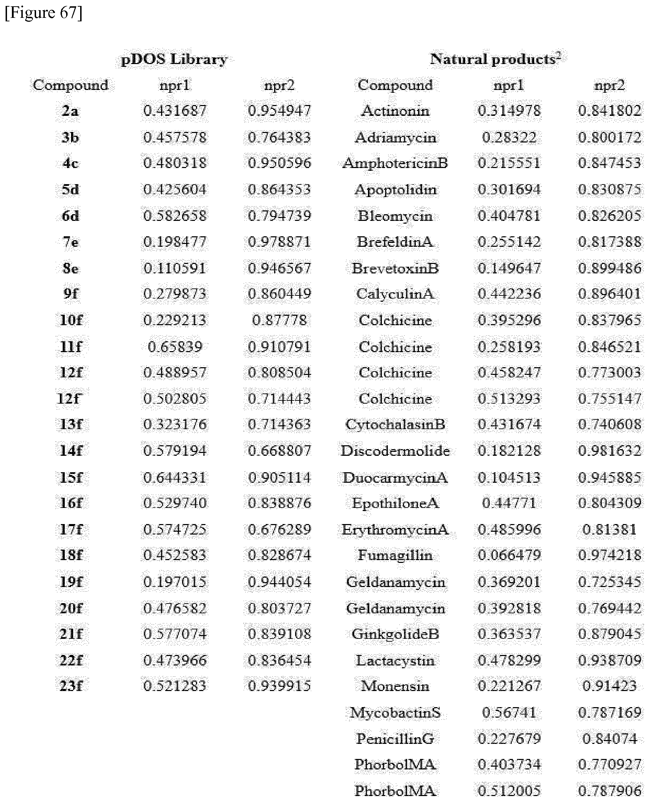

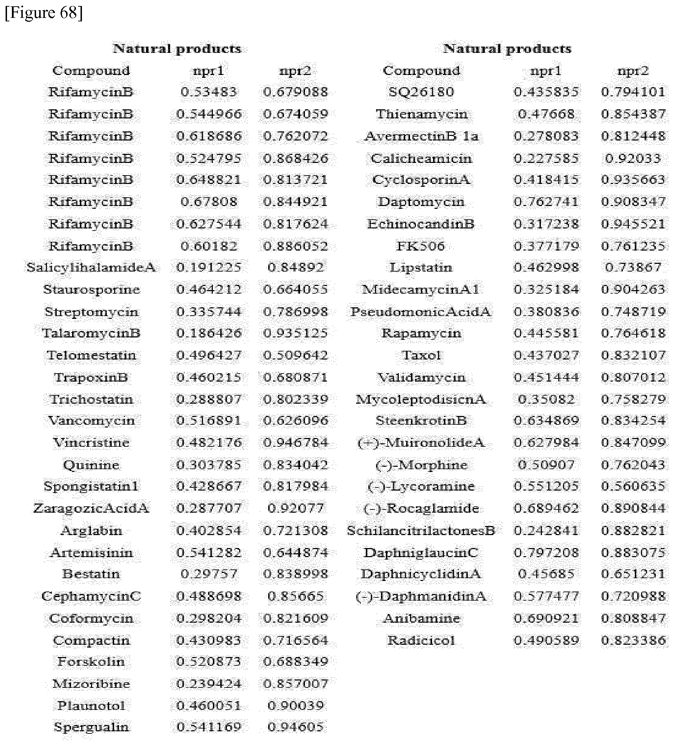

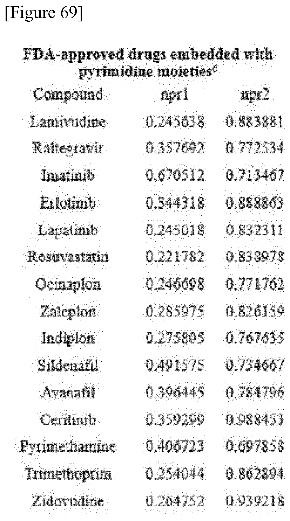

Figs. 67 to 69 show normalized PMI ratios of pDOS library, natural products and FDA-approved drugs embedded with pyrimidine moieties.

### Detailed Description of the Invention

The present invention is related to a novel pyrimidine derivative compound, a process for preparing the novel pyrimidine derivative compound, and a pharmaceutical composition using the novel pyrimidine derivative compound.

Specifically, a pyrimidine derivative compound of the present invention comprises a pyrimidine derivative compound represented by any one of formulas I to IX: 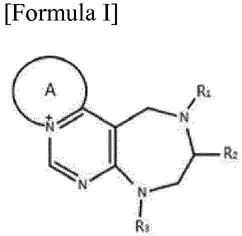
wherein A forms a heterobicyclic ring together with adjacent N and C atoms wherein the heterobicyclic ring is an 8- to 9-membered bicyclic ring which may contain 1 to 3 nitrogen atoms; R₁ is -Boc (tert-butyloxycarbonyl) or a benzyl group (Bn); R₂ is -CH₂-O-TIPS; and R₃ is hydrogen, a C₁ to C₃ alkyl group, an allyl group, -Tf, a C₁ to C₃ alkyl benzyl, or 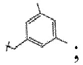 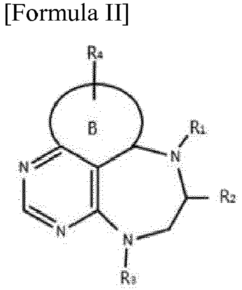
wherein B forms a 6- to 10-membered ring together with adjacent 2 to 3 C atoms wherein the ring may contain 1 to 3 nitrogen atoms; R₁ is -Boc or a benzyl group; R₂ is -CH₂-O-TIPS; and R₃ is hydrogen, a C₁ to C₃ alkyl group, an allyl group, -Tf, a C₁ to C₃ alkyl benzyl group, or 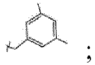 and R₄ is a C₁ to C₃ alkyl group or -CH₂-PMB; 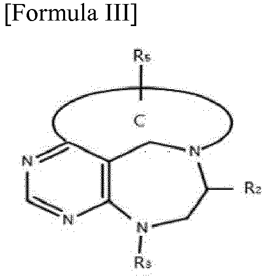
wherein C forms a 10- to 14-membered ring together with adjacent N and C atoms wherein the ring may contain 1 to 3 nitrogen atoms; R₂ is -CH₂-O-TIPS; R₃ is hydrogen, a C₁ to C₃ alkyl group, an allyl group, -Tf, a C₁ to C₃ alkyl benzyl group, or 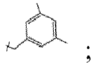 and R₅ is a C₁ to C₃ alkyl group or -CH₂-PMB; 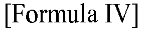 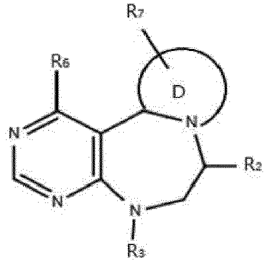
wherein D forms a 4- to 6-membered cyclic imide together with adjacent N and C atoms wherein the cyclic imide is a ring which may contain 1 to 3 nitrogen atoms; R₂ is - CH₂-O-TIPS; R₃ is hydrogen, a -C₁ to C₃ alkyl group, an allyl group, -Tf, a C₁ to C₃ alkyl benzyl group, or 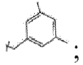 R₆ is 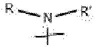 wherein R and R' are each independently a benzyl group or a C₁ to C₃ alkyl group; and R₇ is a benzyl group, a phenyl group, or 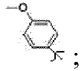 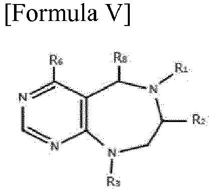
wherein R₁ is -Boc or a benzyl group; R₂ is -CH₂-O-TIPS; and R₃ is hydrogen, a C₁ to C₃ alkyl group, an allyl group, -Tf, a C₁ to C₃ alkyl benzyl group, or 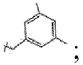 R₆ is 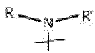 wherein R and R' arc each independently a benzyl group or a C₁ to C₃ alkyl group; and R₈ is an acetylene group; 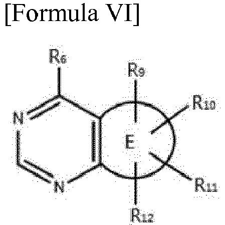
wherein E forms an 8- to 10-membered heterocyclic ring together with adjacent two C atoms wherein the heterocyclic ring is a saturated or unsaturated ring which contains at least two of 2 to 4 nitrogen atoms and 1 to 3 oxigen atoms; R₆ is 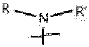 wherein R and R' are each independently a benzyl group or a C₁ to C₃ alkyl group; and R₉ to R₁₂ are each independently hydrogen, a methyl group, an azide group, a benzyl group, -CH₂-O-TIPS or-Tf; 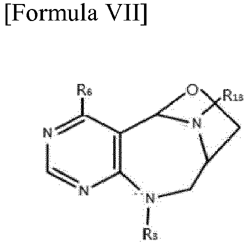
wherein R₃ is hydrogen, a -C₁ to C₃ alkyl group, an allyl group, -Tf, a C₁ to C₃ alkyl benzyl group, or 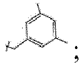 R₆ is 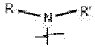 wherein R and R' are each independently a benzyl group or a C₁ to C₃ alkyl group; and R₁₃ is -Boc, -CONHBn, -m-Ns; 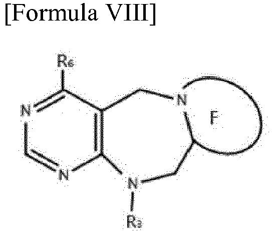
wherein F is -X-(CH₂)n-(O-CH₂)m- and forms a 3- to 6-membered heterocyclic ring together with adjacent N and C atoms wherein X is 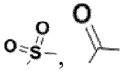 or -CH₂-, and n and m are respectively an integer from 0 to 2; R₃ is hydrogen, a -C₁ to C₃ alkyl group, an allyl group,-Tf, a C₁ to C₃ alkyl benzyl group, or 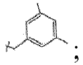 and R₆ is 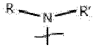 wherein R and R' are each independently a benzyl group or a C₁ to C₃ alkyl group; 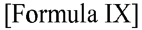 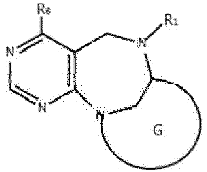
wherein G forms a 6- to 9-membered heterocyclic ring together with adjacent N and C atoms wherein the heterocyclic ring is an unsaturated heterocyclic ring which contains 2 to 3 nitrogen atoms; R₁ is -Boc or a benzyl group; and R₆ is 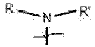 wherein R and R' are each independently a benzyl group or a C₁ to C₃ alkyl group;
or a pharmaceutically acceptable salt thereof.

Further, the present invention comprises a compound represented by any one of formulas I-1 to IX-1: 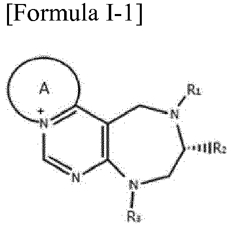
wherein A forms a heterobicyclic ring together with adjacent N and C atoms wherein the heterobicyclic ring is an 8- to 9-membered bicyclic ring which may contain 1 to 3 nitrogen atoms; R₁ is -Boc or a benzyl group; R₂ is -CH₂-O-TIPS; and
R₃ is hydrogen, a C₁ to C₃ alkyl group, an allyl group, -Tf, a C₁ to C₃ alkyl benzyl, or 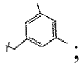 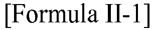 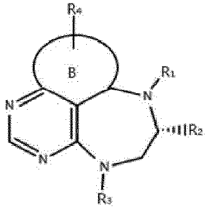
wherein B forms a 6- to 10-membered ring together with adjacent 2 to 3 C atoms wherein the ring may contain 1 to 3 nitrogen atoms; R₁ is -Boc or a benzyl group; R₂ is -CH₂-O-TIPS; and R₃ is hydrogen, a C₁ to C₃ alkyl group, an allyl group, -Tf, a C₁ to C₃ alkyl benzyl group, or 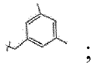 and R₄ is C₁ to C₃ alkyl group or -CH₂-PMB; 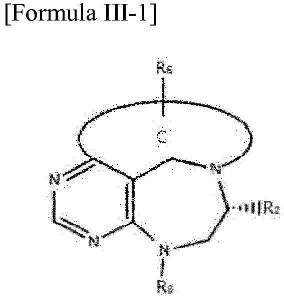
wherein a C forms a 10- to 14-membered ring together with adjacent N and C atoms wherein the ring may contain 1 to 3 nitrogen atoms; R₂ is -CH₂-O-TIPS; R₃ is hydrogen, a C₁ to C₃ alkyl group, an allyl group, -Tf, a C₁ to C₃ alkyl benzyl group, or 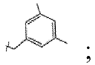 and R₅ is C₁ to C₃ alkyl group or -CH₂-PMB; 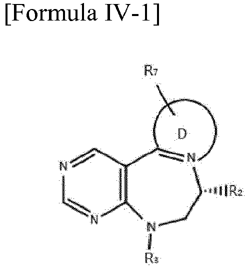
wherein D forms a 4- to 6-membered cyclic imide together with adjacent N and C atoms wherein the cyclic imide is a ring which may contain 1 to 3 nitrogen atoms; R₂ is - CH₂-O-TIPS; R₃ is hydrogen, a -C₁ to C₃ alkyl group, an allyl group, -Tf, a C₁ to C₃ alkyl benzyl group, or 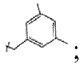 R₆ is 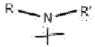 wherein R and R' are each independently a benzyl group or a C₁ to C₃ alkyl group; R₇ is a benzyl group, a phenyl group, or 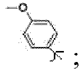 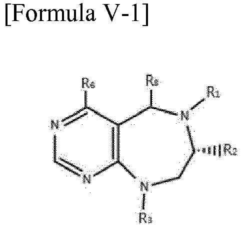
wherein R₁ is -Boc or a benzyl group; R₂ is -CH₂-O-TIPS; and R₃ is hydrogen, a -C₁ to C₃ alkyl group, an allyl group, -Tf, a C₁ to C₃ alkyl benzyl group, or 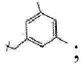 R₆ is 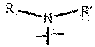 wherein R and R' are each independently a benzyl group or a C₁ to C₃ alkyl group; and R₈ is an acetylene group; 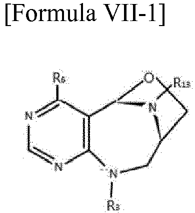
wherein R₃ is hydrogen, a -C₁ to C₃ alkyl group, an allyl group, -Tf, a C₁ to C₃ alkyl benzyl group, or 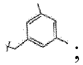 R₆ is 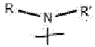 wherein R and R' are each independently a benzyl group or a C₁ to C₃ alkyl group; and R₁₃ is -Boc, -CONHBn, -m-Ns; 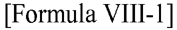 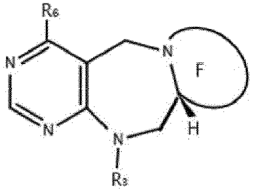
wherein F is -X-(CH₂)n-(O-CH₂)m- and forms a 3- to 6-membered heterocyclic ring together with adjacent N and C atoms wherein X is 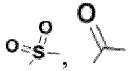 or -CH₂-, and n and m are respectively an integer from 0 to 2; R₃ is hydrogen, a -C₁ to C₃ alkyl group, an allyl group,-Tf, a C₁ to C₃ alkyl benzyl group, or 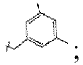 and R₆ is 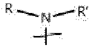 wherein R and R' are each independently a benzyl group or a C₁ to C₃ alkyl group; 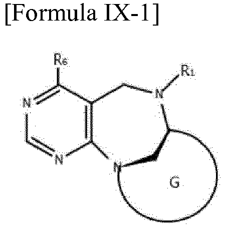
wherein G forms a 6- to 9-membered heterocyclic ring together with adjacent N and C atoms wherein the heterocyclic ring is an unsaturated heterocyclic ring which contains 2 to 3 nitrogen atoms; R₁ is -Boc or a benzyl group; and R₆ is 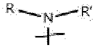 wherein R and R' are each independently a benzyl group or a C₁ to C₃ alkyl group;
or a pharmaceutically acceptable salt thereof.

Specifically, the compound of the present invention comprises a compound represented by any one of the formulas: 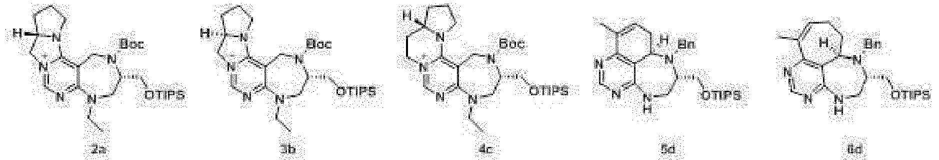 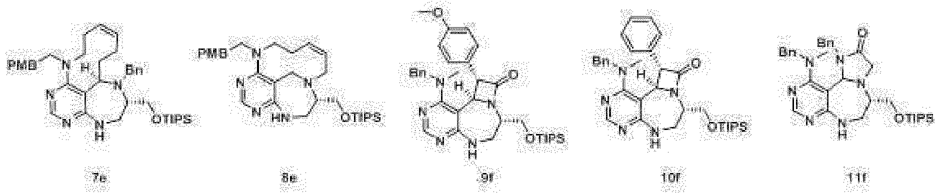 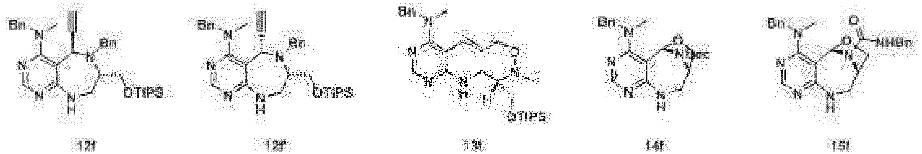 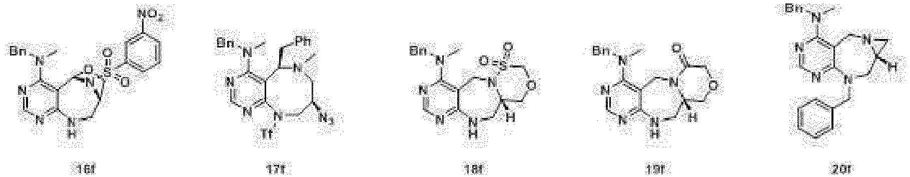 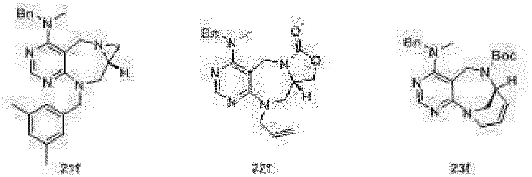 or a pharmaceutically acceptable salt thereof.

In the formulas of the present invention, A to G comprise at least one atom selected from C, N, S and O, and comprise all substituents to form a structure of a compound of formulas I to IX. Specifically, they comprise all substituents to form a structure of compounds 2a, 3b, 4c, 5d to 6d, 7e to 8e, and/or 9f to 23f.

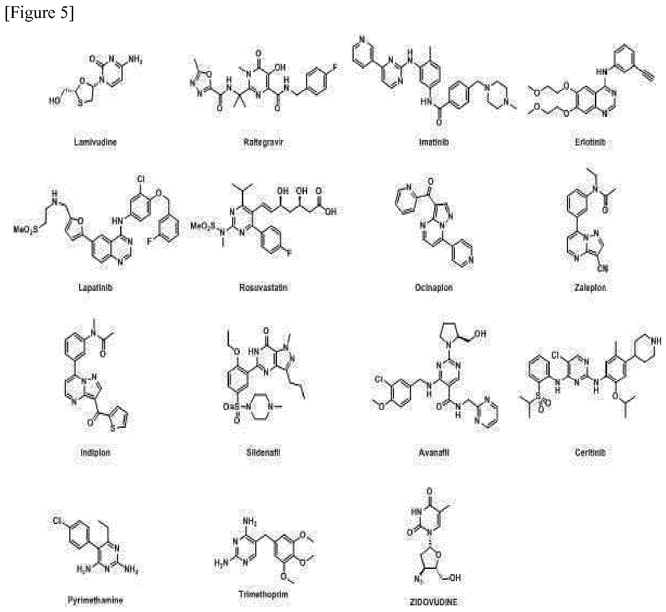

In the present invention, the pyrimidine moiety is commonly present in various bioactive small molecules (Figs. 5 and 67), and it plays a critical role as a nucleoside analogue in various kinase inhibitors or adenosine receptor modulators due to its hydrogen bonding ability (Fig. 1a). Therefore, many synthetic efforts towards pyrimidine-containing species have been focused on aromatic monocyclic or bicyclic skeletons, which limits the structural diversity of the pyrimidine-containing core skeletons. In addition, the 3D structural complexity of the core skeletons (Fig. 2) became important because planar frameworks less frequently constitute FDA-approved chemical entities, especially in regard to undruggable targets.

Based on the above, to expand the molecular diversity beyond monocyclic and bicyclic pyrimidine skeletons, the present invention developed a new pDOS strategy towards the divergent synthesis of natural product-like polyheterocycles containing pyrimidodiazepine or pyrimidine. Diazepine is also often found in complex natural products that exhibit a wide range of biological activities, and is known to be a prominent privileged structure that can improve the bioavailability and bioactivity of compounds. In addition, seven-membered rings that are fused to aromatic rings generally have higher conformational flexibility and a greater number of reactive sites than five- or six-membered fused ring systems, as confirmed by the direct comparison of pyrimidine-embedded tricyclic 6/6/6 and 6/7/6 systems by overlaying the energy-minimized conformational isomers aligned along the pyrimidine substructure (Fig. 1b). Thus, pyrimidodiazepine can serve as a versatile intermediate to access highly diverse and complex polyheterocycles through the incorporation of additional ring systems, which forms the basis of a new pyrimidodiazepine-based pDOS pathway. To establish the pDOS pathway, we first designed and synthesized, as a starting material, formula 1 comprising highly functionalized pyrimidodiazepine containing five reactive sites (A-E). In the pDOS strategy of the present invention, the compound of formula 1 as a starting material can be transformed into nine distinct pyrimidodiazepine- or pyrimidine-containing pyrimidine derivatives via pairing with different functional groups at each reactive site (Fig. 1c).

The pyrimidine derivative compounds of the present invention represented by formulas I to IX exhibit much higher structural complexity and skeletal diversity than existing pyrimidine-based drugs, as demonstrated by PMI analysis of the working examples and experiments of the present invention. Using the pyrimidine structures as chemical navigators to efficiently access unexploited regions of bioactive chemical space, the pDOS synthetic pathway provides unique library of polyheterocyclic compounds with superior biological relevancy.

In the present invention, we performed ELISA-based HTS with the pDOS library and identified 21f, a small-molecule PPI inhibitor of the LRS-RagD interaction, which mediates Leu signal sensing in the mTORC1 signalling pathway. Through western blotting and biophysical study using SPR on phosphorylation of S6K1, it was confirmed that 21f could induce cellular autophagy, and reduce cellular proliferation even in the presence of Leu by disrupting the LRS-RagD interaction and subsequently inhibiting Leu-dependent mTORC1 activity. Therefore, this PPI inhibitor could be a powerful tool to specifically delineate unrevealed amino acid-dependent biological processes of mTORC1. Furthermore, the PPI inhibitor could act as a lead structure in the development of novel therapeutic agents to treat diseases linked to the oncogenic activation of mTORC1, thus highlighting the great potential of the pDOS strategy to address unmet therapeutic challenges.

A process for preparing the pyrimidine derivative compound of the present invention may comprise:
Step 1:
   for preparing an intermediate substance, tert-(S)-(1-(6-chloro-5-formylpyrimidin-4-yl)amino)-3-((triisopropylsilyl)oxy)propan-2-yl)carbamate;
Step 1a:
   for preparing a starting material represented by formula 1 by reacting the intermediate substance prepared in step 1 with cyclic amine or tetrakis palladium; and 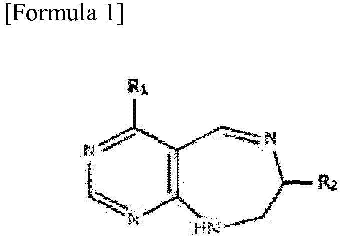 wherein R₁ is 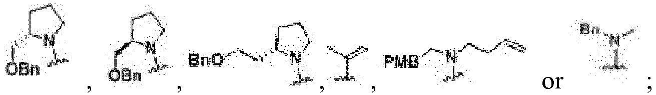 and R₂ is -CH₂-O-TIPS;
Step 2:
   for preparing a desired compound from the starting material prepared in step 1a,
   which, more specifically, further comprises at least one of the following reactions on a compound having formula 1 as a starting material prepared in step 1a: N-alkylation, debenzylation, intramolecular substitution reaction, ring-closing metathesis, nucleophilic addition reaction, allylation reaction of amine, Grubbs' catalyst condensation reaction, alcohol deprotection reaction, intramolecular nucleophilic addition reaction, aziridium intermediate formation reaction, nucleophilic ring expansion reaction, and bridge head ring formation reaction.

The derised compound in the present invention means a compound represented by formulas I to IX, more specifically, compounds 2a, 3b, 4c, 5d to 6d, 7e to 8e, and 9f to 23f.

According to one example of the present invention, step 2 in the preparation process of the present invention may comprise the steps of Schemes 1 or 2:

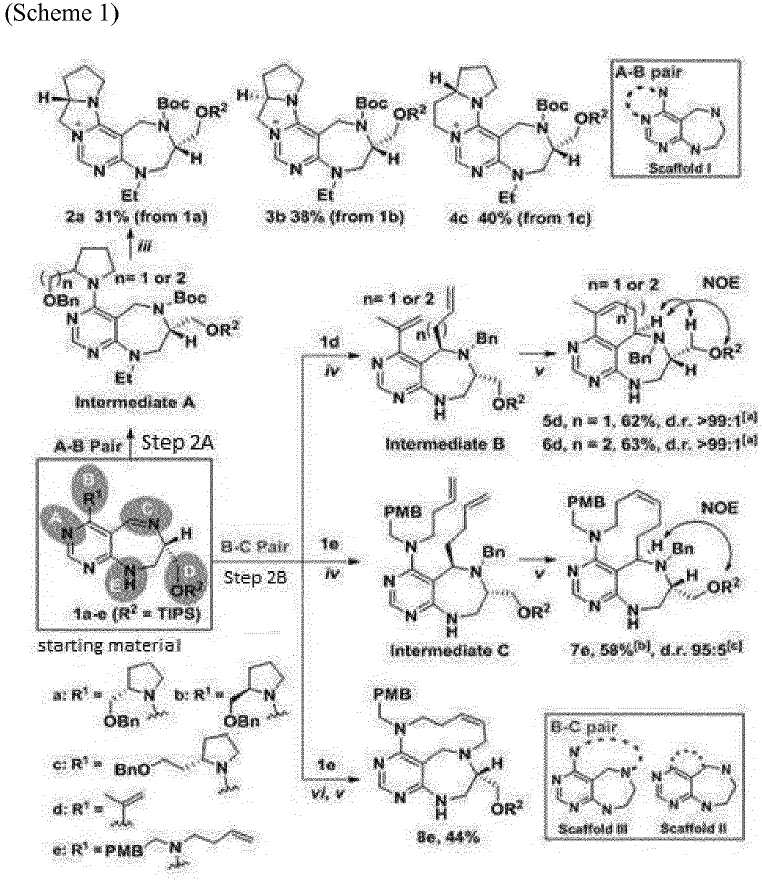

Each step of scheme 1 can be detailed as follows:

### Step 2A: Pairing at the A and B positions of the starting materials

Starting materials 1a to 1e to be discussed below can readily be synthesized through a series of transformations (Scheme 2). For the synthesis of compounds 2a, 3b, and 3c, starting materials 1a to 1c are treated with sodium borohydride (NaBH4) to afford secondary amines on R1; the resulting secondary amines are protected with -Boc and aniline is N-alkylated, which allows for the formation of intermediates A. Compound 2a, 3b, and 4c can efficiently be generated via A-B pairing through an intramolecular substitution reaction, when intermediates A are subjected to debenzylation and the resultant alcohols are subsequently activated with methanesulfonyl chloride (MsCI). Consequently, compounds 2a, 3b and 4c having stereochemically tetracycles, containing a pyrimidinium moiety, can be obtained in moderate yields.

### Step 2B: Pairing at the B and C positions of the starting materials

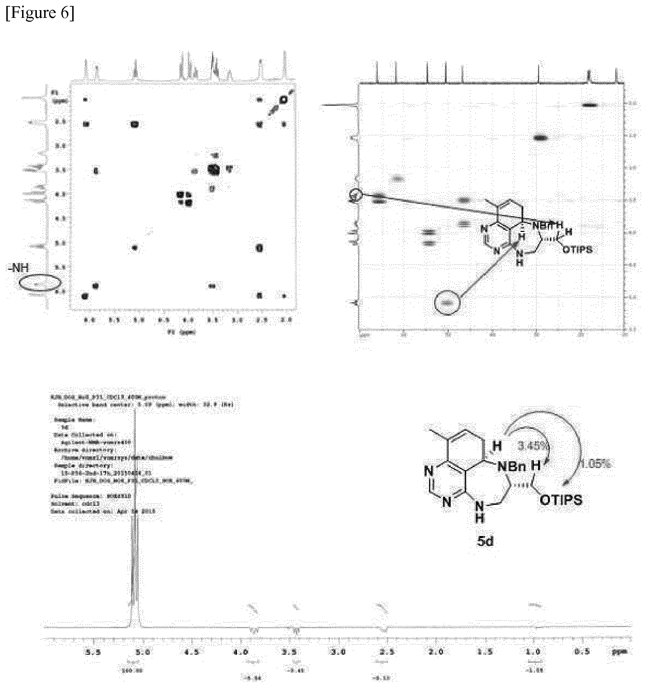

We employed ring-closing metathesis (RCM) for the synthesis of compounds 5d, 6d, 7e, and 8e via B-C pairing. For the RCM reaction, a vinyl group is important and is introduced as a substituent at the C position of the starting materials shown in the scheme above. Starting materials 1d and 1e are subjected to N-alkylation with benzyl bromide to afford iminium ions. Diastereoselective nucleophilic addition of allyl or homoallyl Grignard reagents to the iminium ions generates intermediates B and C. RCM with addition of Grubbs' second-generation catalyst provides the synthesis of compounds 5d, 6d and 7e. 6-, 7- and 10-membered rings are formed by condensation where the condensation reaction through RCM exerted good to excellent diastereoselectivity. Notably, this diastereoselectivity without chiral auxiliaries or catalysts could be rationalized by the preferential approach of Grignard reagents towards the less hindered re-face of the imine in 1d and 1e. The stereochemistry of each product was confirmed by nuclear Overhauser effect (NOE) spectroscopy (Figs. 6 to 8).

To generate compound 8e, starting material 1e was treated with NaBH₄ to start the synthesis. Allylation of the secondary amine resulting from the treatment reaction with NaBH₄ and subsequent RCM with Grubbs' second-generation catalyst yielded compound 8e. 10-membered rings are obtained by condensation in 44% overall yield.

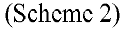

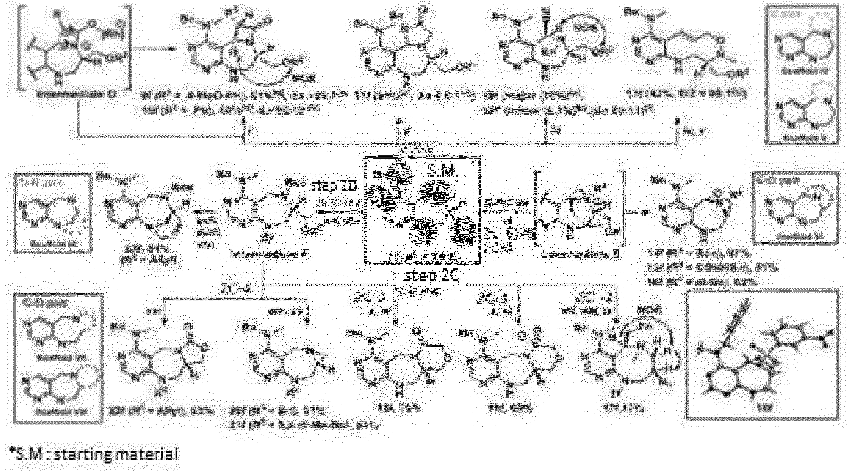

### Step 2C: Pairing at the C and D positions of the starting materials

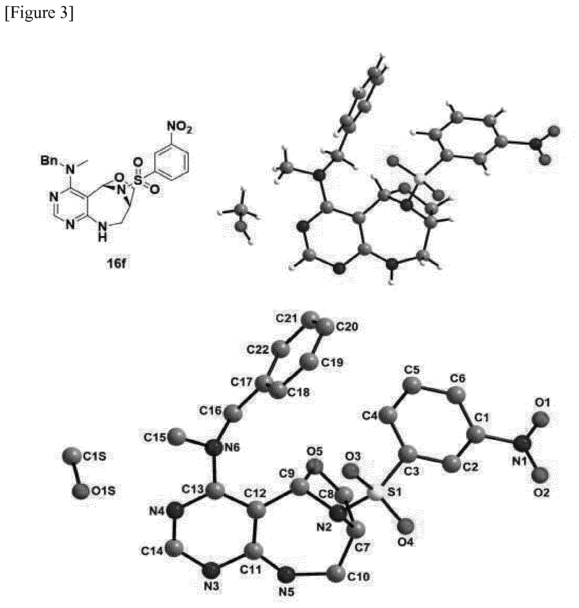

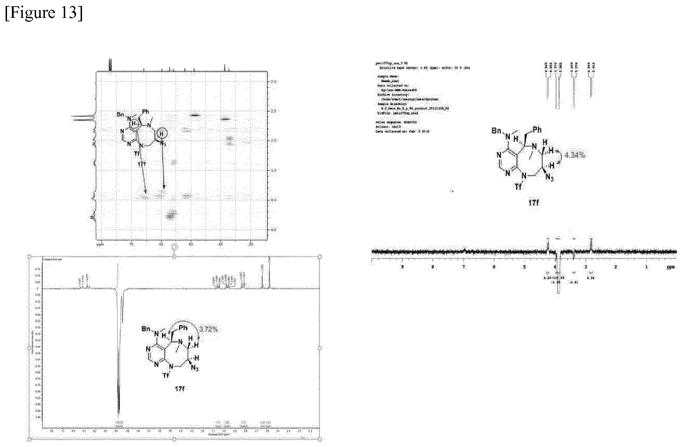

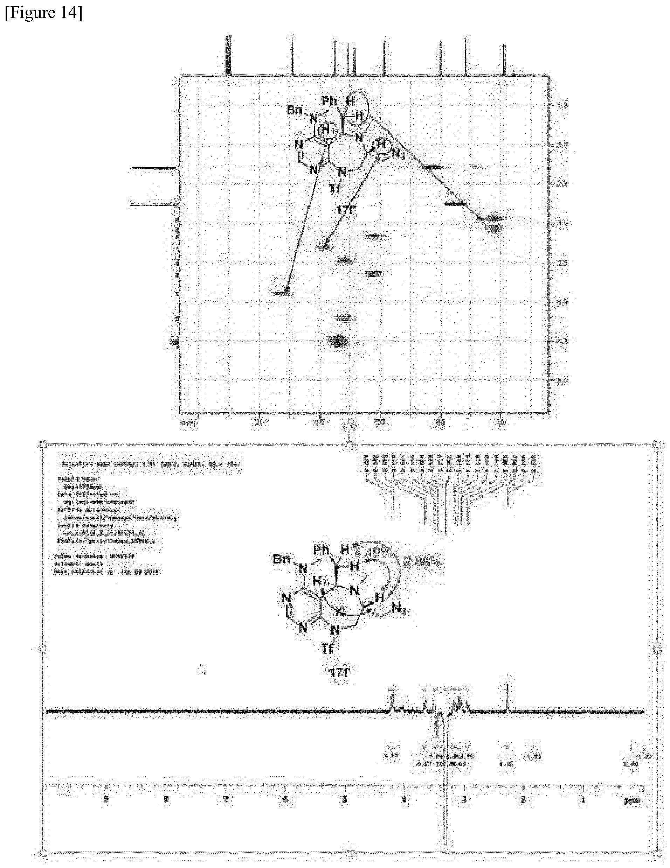

2C-1: Deprotection of the silyltri propyl (TIPS) group from starting material 1f provides an alcohol functional group, which generates the desired compounds 14f, 15f and 16f having the structure of bridged oxazolidine in good yields upon treatment with various N-modifying agents such as Boc anhydride (Boc2O), benzyl isocyanate and 3-nitrobenzene sulfonyl chloride (m-NsCI) to form iminium and subsequent intramolecular nucleophilic addition of the hydroxyl moiety. The structure of bridged oxazolidine 16f was confirmed by X-ray crystallographic analysis (Fig. 3).
2C-2: To synthesize compound 17f, starting material 1f was subjected to N-alkylation with the treatment of methyl iodide, followed by nucleophilic addition of benzylmagnesium bromide to form iminium ion and subsequent removal of the TIPS group of starting material 1f. Then, the hydroxyl moiety of intermediate substance SI-8 (Example 15) was activated with trifluoromethanesulfonic anhydride to provide an aziridium intermediate and the subsequent nucleophilic ring expansion reaction with azide anion afforded to 17f containing an eight-membered diazocane ring. In fact, benzodiazocine is known as an important pharmacophore, but the synthesis of diazocane has not been extensively studied due to the unfavourable transannular strain in medium-sized rings. The stereochemistry of 17f was confirmed by NOE spectroscopy (Figs. 13 and 14).
2C-3: For the synthesis of compounds 18f and 19f, starting material 1f was treated with NaBH4 to afford a secondary amine, which underwent sulfonamide or amide formation upon treatment with chloromethane sulfonyl chloride or chloroacetic anhydride. The subsequent removal of - TIPS group and alcohols, which resulted from intramolecular nucleophilic attack with electrophiles afforded to compounds 18f or 19f, was condensed with and introduced into six-membered or lactam rings.
2C-4: Compounds 20f and 21f contain an aziridine ring. Starting material 1f was treated with NaBH4 to result in secondary amine, which was protected with Boc. Aniline was N-alkylated to provide intermediates F (R⁵ = Benzyl or 3,5-dimethylbenzyl). The removal of -TIPS and -Boc group of intermediate F, followed by subsequent cyclization, to produce 20f or 21f in 51 and 53% yields, respectively. Finally, oxazolidinone-containing compound 22f was obtained through the TIPS deprotection of intermediate F (R⁵ = allyl) and subsequent cyclization.
Step 2D: Pairing at the D and E positions of the starting materials

The TIPS group of intermediate F was removed, followed by oxidation reaction with Dess-Martin periodinane to provide an aldehyde. The resulting unstable aldehyde from the oxidation reaction was immediately subjected to Wittig olefination, followed by RCM reaction with Grubbs' second-generation catalyst to afford compound 23f containing bridgehead [4,3,1] ring systems.

Further, the compounds of the present invention exert excellent cytotoxicity against cancer cells and thus can be used as an inhibitor against cancer cell growth, whereby being useful as a treating agent for cancer diseases. As such, the present invention provides a composition for treating cancer comprising a pyrimidine derivative compound represented by any one of formulas I to IX as an active ingredient, which is effective for cancer diseases.

The cancer diseases comprise at least one selected from the group consisting of cervical cancer, lung cancer, pancreatic cancer, non-small cell lung cancer, liver cancer, colon cancer, bone cancer, skin cancer, head or neck cancer, melanoma in skin or eye, uterine cancer, ovarian cancer, rectal cancer, gastric cancer, cancer around anal, colon cancer, breast cancer, fallopian tube carcinoma, endometrial carcinoma, vaginal carcinoma, vulva carcinoma, esophageal cancer, small intestine cancer, endocrine gland cancer, thyroid cancer, parathyroid cancer, adrenal cancer, soft tissues sarcoma, urethral cancer, penile cancer, prostate cancer, bladder cancer, cancer of renal or ureter, renal cell carcinoma, and renal pelvis carcinoma; preperably, lung cancer, liver cancer, skin cancer, or skin melanoma.

The treating agent of the present invention may be formulated as a pharmaceutically conventional formulation for an oral or parenteral administration, for example, a tablet, capsule, troche, solution, suspension, etc., by adding appropriate non-toxic carrier, excipients, adjuvants as generally used into a pyrimidine derivative compound of the present invention. Further, dosage to human of a compound according to the present invention depends on ages, weight, and sex of patients, administration route, health condition, and severity of disease. Based on an adult patient, the dosage is generally from 0.0001 to 1000 mg/kg/day, which may be divided and administered into once or several times per day over a certain interval according to the determination of a dortor or pharmacist. As such, the dosage is not construed to limit the claimed scope at any aspect.

The compound of the present invention may be administered to mammals such as rat, mouse, livestock, or human via different routes. All potential administration routes may be expected, for example, oral, rectal, intravenous, muscular, subcutaneous, intrauterine, intracerebroventricular injection.

Hereinafter, the working examples will be provided for the better understanding of the present invention. However, the examples are merely provided only for a better understanding of the present invention, but are not to be construed as the limitation of the claimed scope.

### Example

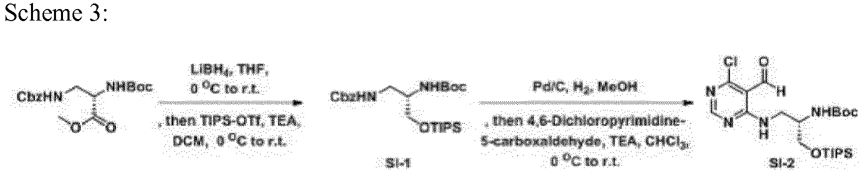

### Scheme 3-1) Preparation of SI-1

(S)-3-(((benzyloxy)carbonyl)amino)-2-((tert-butoxycarbonyl)amino)propanoate (20.00 g, 56.8 mmol) was added to THF (570.0 ml) and stirred. Lithium borohydride (2.0 M solution in THF, 56.8 ml, 113.5 mmol) was slowly added to the solution at 0 °C dropwise. Then, the resulting mixture was left to stir at r.t. After completion of the reaction as indicated by TLC, the mixture was quenched with a saturated ammonium chloride solution. The reaction solution was extracted twice with dichloromethane. The extracted organic solution was dried over anhydrous sodium sulfate and filtered. The filtrate was evaporated under reduced pressure to be condensed and dried. The compound was dissolved in dry dichloromethane (570.0 ml) under argon atmosphere. To this solution were sequentially added triethylamine (15.8 ml, 113.5 mmol) and then TIPS-OTf (22.88 ml, 85.14 mmol) at 0 °C. The resulting mixture was left to stir at r.t. After completion of the reaction as indicated by TLC, the misture was quenched with a saturated ammonium chloride solution. The solution was extracted twice with dichloromethane. The extracted organic solution was dried over anhydrous sodium sulfate and filtered. The filtrate was condensed under reduced pressure, followed by being purified using silica-gel flash column chromatography to afford the desired product SI-1 (22.37 g, 82% yield) as a light colorless oil.

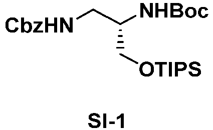

*R_{f}* = 0.4 (hexane/EtOAc 5:1); ¹H NMR (400 MHz, a CDCl₃) δ 7.35-7.29 (m, 5H), 5.32 (br s, 1H), 5.10 (s, 2H), 5.00 (br s, 1H), 3.77-3.73 (m, 3H), 3.47-3.39 (m, 2H), 1.43 (s, 9H), 1.06 (m, 21H); ¹³C NMR (100 MHz, a CDCl₃) δ 157.0, 156.1, 136.7, 128.6, 128.11, 128.07, 79.6, 66.8, 64.3, 51.8, 43.5, 28.4, 18.0, 11.9; LRMS(ESI+): Calcd for a C₂₅H₄₅N₂O₅Si⁺ [M+H]⁺ 481.31, found 481.11.(Fig. 26)

To SI-1 (22.37 g, 46.54 mmol) dissolved in methanol (470.0 ml) was added Pd/C (palladium complex compound: w/w 10%, 11.19 g) and the mixture was stirred with feeding hydrogen atmosphere (1 atm) at r.t. After completion of the reaction as indicated by TLC, the reaction mixture was filtered through Celite® while the filtrate was extracted with ethyl acetate. The filtrate was condensed under reduced pressure and was dissolved in chloroform (470.0 ml). To this solution were added triethylamine (9.74 ml, 69.81 mmol) and 4,6-dichloropyrimidine-5-carboxaldehyde (8.24 g, 46.54 mmol) at 0 °C and left to stir. After stirring over 30 min, the mixture was quenched with a saturated ammonium chloride solution. The mixture was extracted twice with chloroform, and the extracted organic solution was dried over anhydrous sodium sulfate, and filtered. The filtrate was condensed under reduced pressure and the product was purified by silica-gel flash column chromatography to afford the desired product SI-2 (15.19 g, 67% yield) as a light colorless oil.

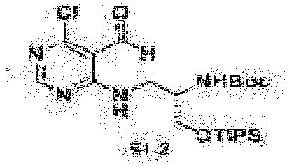

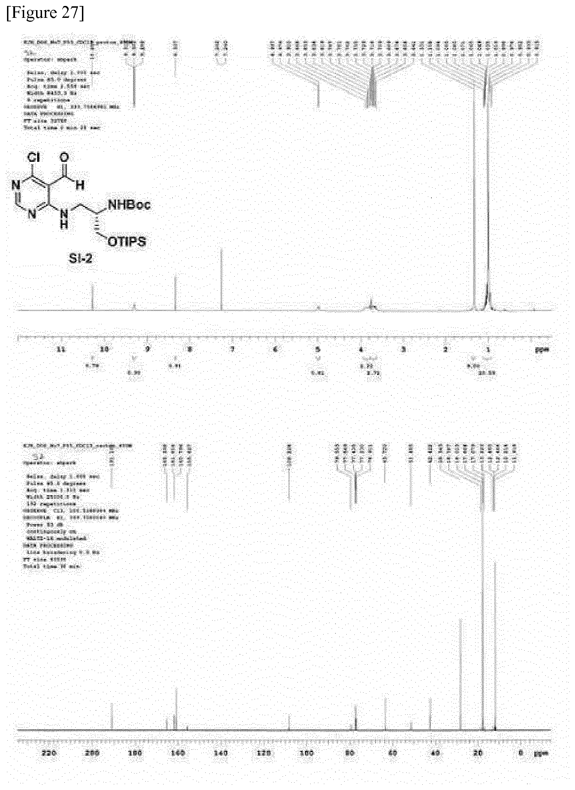

*R*_{f} = 0.5 (hexane/EtOAc 3:1); ¹H NMR (400 MHz, a CDCl₃) δ 10.28 (s, 1H), 9.30 (t, *J* = 5.7 Hz, 1H), 8.34 (s, 1H), 4.99 (d, *J* = 8.6 Hz, 1H), 3.90-3.62 (m, 5H), 1.33 (s, 9H), 1.06 (m, 21H); ¹³C NMR (100 MHz, a CDCl₃) δ 191.1, 165.3, 161.7, 160.7, 155.6, 108.2, 79.5, 63.7, 51.4, 42.4, 28.3, 17.9, 11.9; LRMS (ESI+): Calcd for a C₂₂H₄₀ClN₄O₄Si⁺ [M+H]⁺ 487.25, found 487.03. (Fig. 27).

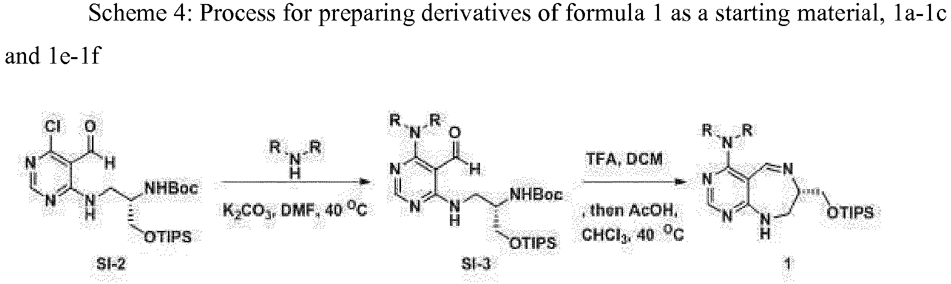

### Scheme 4-1)

SI-2 (500 mg, 1.026 mmol) and potassium carbonate (283.6 mg, 2.052 mmol) were dissolved to dimethylformamide (10.0 ml) and stirred, followed by adding cyclic amine (1.539 mmol) thereto and stirring at 40 °C. After completion of the reaction as indicated by TLC, the mixture was quenched with a saturated ammonium chloride solution. The reaction solution was extracted twice with ethyl acetate. The extracted organic solution was dried over anhydrous sodium sulfate and filtered. The filtrate was condensed under reduced pressure and the product was purified by silica-gel flash column chromatography to afford desired compound SI-3.

### Scheme 4-2)

To cyclic imine product 1, SI-3 was added into 10% trifluoroacetic acid dichloromethane solution (20.0 ml) and stirred at r.t. After completion of the reaction as indicated by TLC, any excess trifluoro acetic acid was removed by azeotropic evaporation with toluene under reduced pressure. The compound was dissolved in 1% acetic acid chloroform (100.0 ml) and stirred at 40 °C. After completion of the reaction as indicated by TLC, the reaction was quenched with a saturated sodium bicarbonate solution. The reaction solution was extracted twice with chloroform. The extracted organic solution was dried over anhydrous sodium sulfate and filtered. The filtrate was condensed under reduced pressure and the product was purified by silica-gel flash column chromatography to afford the desired product 1.

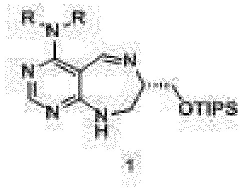

### a) Derivative 1a of starting material 1: (S)-4-((S)-2-((benzyloxy)methyl)pyrrolidin-1-yl)-7-(((triisopropylsilyl)oxy)methyl)-8,9-dihydro-7H-pyrimido[4,5-e][1,4]diazepine

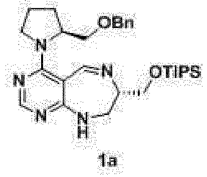

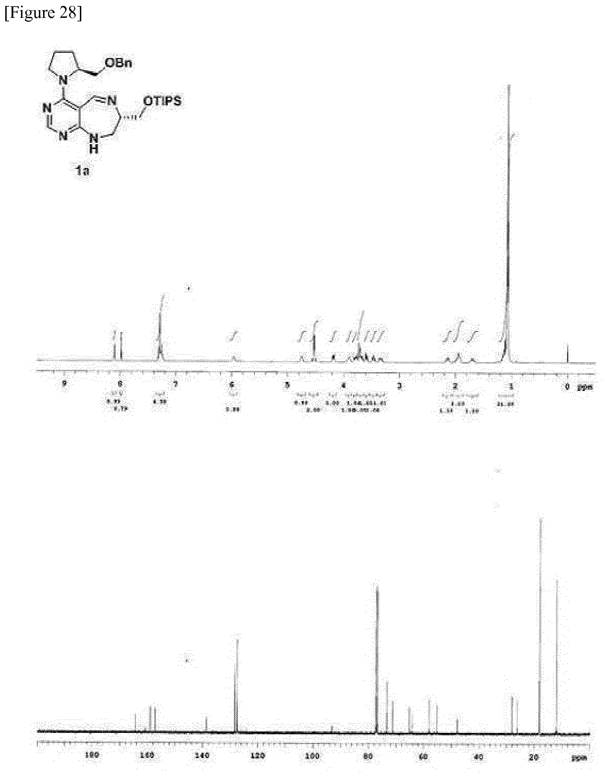

A pale yellow oil; *R*_{f} = 0.2 (DCM/MeOH = 20:1); 403.1 mg, 75% overall yield; ¹H NMR (400 MHz, a CDCl₃) δ 8.11 (s,1H), 7.98 (s, 1H), 7.29 (m, 5H), 5.97 (br s, 1H), 4.74 (m, 1H), 4.54, 4.53 (ABq, *J*_{AB} = 12.1 Hz, 2H), 4.18 (dd, *J* = 9.8, 4.7 Hz, 1H), 3.90 (m, 1H), 3.80 (dd, *J* = 12.5, 5.5 Hz, 1H), 3.73 (m, 2H), 3.67 (m, 1H), 3.60 (dd, *J* = 9.4, 5.9 Hz, 1H), 3.47 (m, 1H), 3.34 (dd, *J* = 11.3, 7.0 Hz, 1H), 2.15 (m, 1H), 1.96 (m, 2H), 1.71 (m, 1H), 1.08 (m, 21H); ¹³C NMR (100 MHz, a CDCl₃) δ 164.5, 160.9, 159.1, 157.4, 138.6, 128.4, 127.6, 127.5, 93.1, 73.3, 71.1, 65.1, 64.2, 58.0, 55.2, 47.8, 28.0, 26.1, 18.2, 12.0; HRMS(ESI+): Calcd for a C₂₉H₄₆N₅O₂Si⁺ [M+H]⁺ 524.3415, found 524.3414, Δppm -0.19. (Fig. 28)

### b) Derivative 1b of starting material 1: (S)-4-((R)-2-((benzyloxy)methyl)pyrrolidin-1-yl)-7-(((triisopropylsilyl)oxy)methyl)-8,9-dihydro-7H-pyrimido[4,5-e][1,4]diazepine

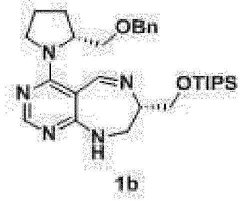

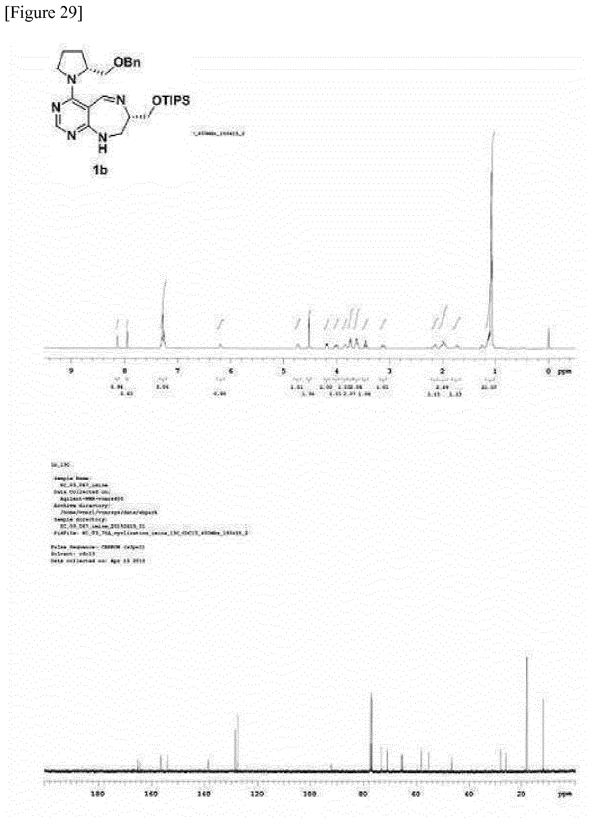

A pale yellow oil; *R*_{f} = 0.2 (DCM/MeOH = 20:1); 408.4 mg, 76% overall yield; ¹H NMR (400 MHz, a CDCl₃) δ 8.14 (s, 1H), 7.96 (s, 1H), 7.30 (m, 5H), 6.20 (br s, 1H), 4.74 (m, 1H), 4.53 (s, 2H), 4.19 (dd, *J* = 9.7, 3.1 Hz, 1H), 4.02 (m, 1H), 3.84 (m, 1H), 3.74 (m, 2H), 3.63 (m, 2H), 3.46 (t, *J* = 9.7 Hz, 1H), 3.14 (m, 1H), 2.14 (m, 1H), 1.99 (m, 2H), 1.74 (m, 1H), 1.09 (m, 21H); ¹³C NMR (100 MHz, a CDCl₃) δ 165.0, 164.2, 156.4, 154.0, 138.4, 128.2, 127.4, 127.4, 92.0, 73.1, 70.8, 65.3, 65.0, 57.9, 55.2, 46.6, 27.8, 25.8, 18.0, 11.8; HRMS(ESI+): Calcd for a C₂₉H₄₆N₅O₂Si⁺ [M+H]⁺ 524.3415, found 524.3415. (Fig. 29)

### c) Derivative 1c of starting material 1: (S)-4-((S)-2-(2-(benzyloxy)ethyl)pyrrolidin-1-yl)-7-(((triisopropylsilyl)oxy)methyl)-8,9-dihydro-7H-pyrimido[4,5-e][1,4]diazepine

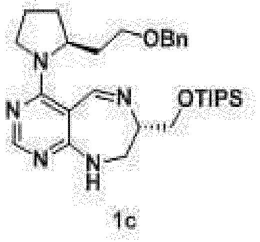

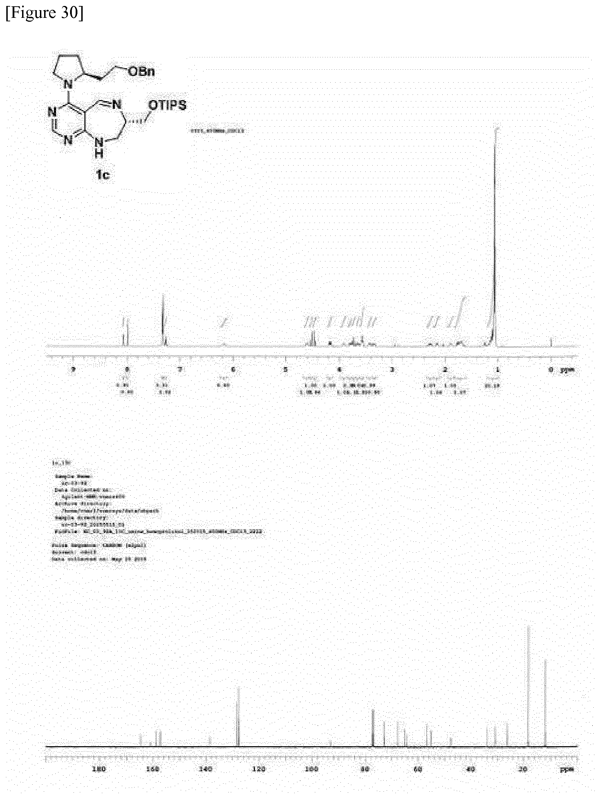

A transparent oil; *R*_{f} = 0.35 (DCM/MeOH = 20:1); 419.4 mg, 76% overall yield; 1H NMR (400 MHz, a CDCl₃) δ 8.07 (s, 1H), 7.99 (s, 1H), 7.33 (m, 4H), 7.29-7.26 (m, 1H), 6.17 (br s, 1H), 4.61 (m, 1H), 4.53, 4.26 (ABq, *J_{AB}* = 11.94 Hz, 2H), 4.18 (dd, *J* = 9.8, 4.3 Hz, 1H), 3.92 (m, 1H), 3.81-3.62 (m, 3H), 3.60-3.56 (m, 2H), 3.45 (m, 1H), 3.33 (m, 1H), 2.33-2.25 (m, 1H), 2.19-2.13 (m, 1H), 1.91-1.87 (m, 1H), 1.80-1.66 (m, 3H), 1.08 (m, 21H); ¹³C NMR (100 MHz, a CDCl₃) δ 164.8, 161.1, 159.0, 157.4, 138.6, 128.4, 127.8, 127.6, 93.2, 73.0, 67.7, 65.1, 64.3, 56.9, 55.1, 47.6, 34.1, 31.0, 26.2, 18.1, 12.0; HRMS(ESI+): Calcd for a C₃₀H₄₈N₅O₂Si⁺ [M+H]⁺ 538.3572, found 538.3571, Δppm -0.19. (Fig. 30)

### d) Derivative 1e of starting material 1: (S)-N-(but-3-en-1-yl)-N-(4-methoxyphenethyl)-7-(((triisopropylsilyl)oxy)methyl)-8,9-dihydro-7H-pyrimido[4,5-e][1,4]diazepine-4-amine

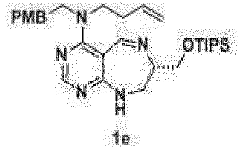

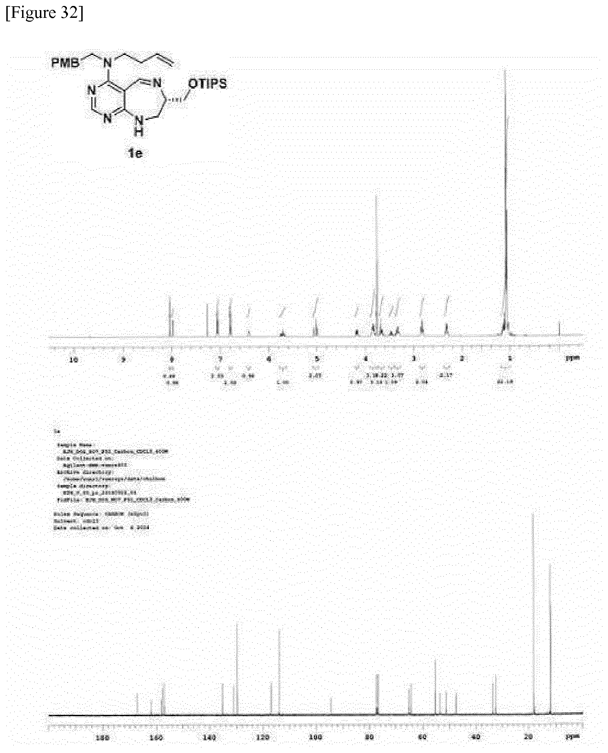

A pale yellow oil; *R*_{f} = 0.4 (DCM/MeOH = 20:1); 364.2 mg, 66% overall yield; ¹H NMR (400 MHz, a CDCl₃) δ 8.05 (s, 1H), 7.98 (d, *J* = 1.9 Hz, 1H), 7.07 (d, *J* = 8.6 Hz, 2H), 6.80 (d, *J* = 8.6 Hz, 2H), 6.42 (m, 1H), 5.72 (dddd, *J* = 16.8, 10.1, 6.6, 6.6 Hz, 1H), 5.02 (m, 2H), 4.18 (dd, *J* = 9.7, 4.3 Hz, 1H), 3.84 (m, 3H), 3.77 (s, 3H), 3.69 (m, 2H), 3.46 (m, 1H), 3.34 (m, 2H), 2.83 (t, *J* = 7.4 Hz, 2H), 2.33 (q, *J* = 7.0 Hz, 2H), 1.08 (m, 21H); ¹³C NMR (100 MHz, a CDCl₃) δ 167.2, 161.9, 158.2, 157.8, 157.1, 135.2, 131.0, 129.7, 117.0, 114.0, 94.3, 65.2, 64.5, 55.3, 53.5, 51.2, 47.6, 33.7, 32.6, 18.1, 12.0; HRMS(ESI+): Calcd for a C₃₀H₄₈N₅O₂Si⁺ [M+H]⁺ 538.3572, found 538.3572. (Fig. 32)

### e) Derivative 1f of starting material 1: (S)-N-benzyl-N-methyl-7-(((triisopropylsilyl)oxy)methyl)-8,9-dihydro-7H-pyrimido[4,5-e][1,4]diazepine-4-amine

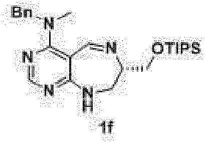

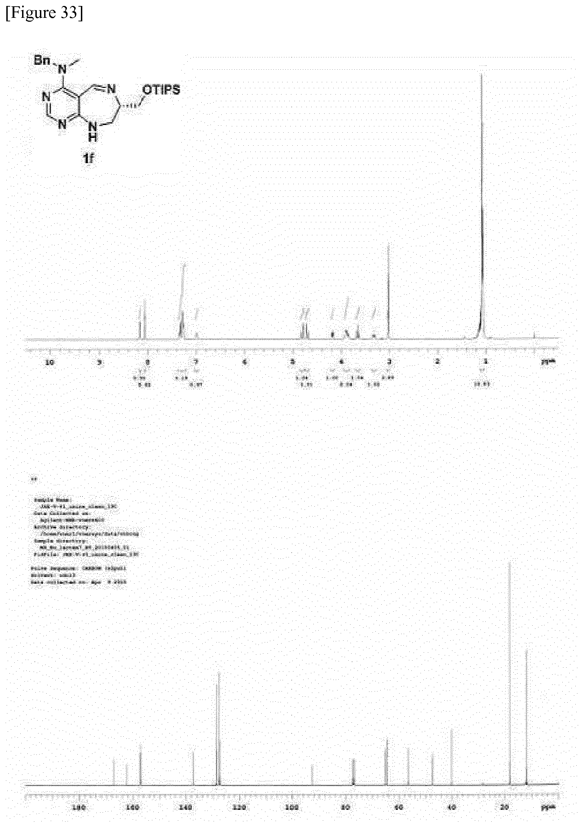

A transparent oil; *R*_{f} = 0.35 (DCM/MeOH = 20:1); 349.1 mg, 75% overall yield; ¹H NMR (400 MHz, a CDCl₃) δ 8.17 (d, *J* = 1.9 Hz, 1H), 8.06 (s, 1H), 7.35-7.25 (m, 5H), 6.99 (br s, 1H), 4.81, 4.71 (ABq, *J*_{AB} = 15.2 Hz, 2H), 4.18 (dd, *J* = 9.8, 4.3 Hz, 1H), 3.89 (m, 2H), 3.66 (t, *J* = 10.0 Hz, 1H), 3.32 (ddd, *J* = 13.0, 7.1, 2.5 Hz, 1H), 3.03 (s, 3H), 1.08 (m, 21H); ¹³C NMR (100 MHz, a CDCl₃) δ 167.4, 162.4, 157.2, 157.1, 137.3, 128.7, 127.7, 127.4, 92.7, 65.1, 64.5, 56.6, 47.3, 40.1, 18.0, 11.9; HRMS(ESI+): Calcd for a C₂₅H₄₀N₅OSi⁺ [M+H]⁺ 454.2997, found 454.2996, Δppm -0.22.. (Fig. 33)

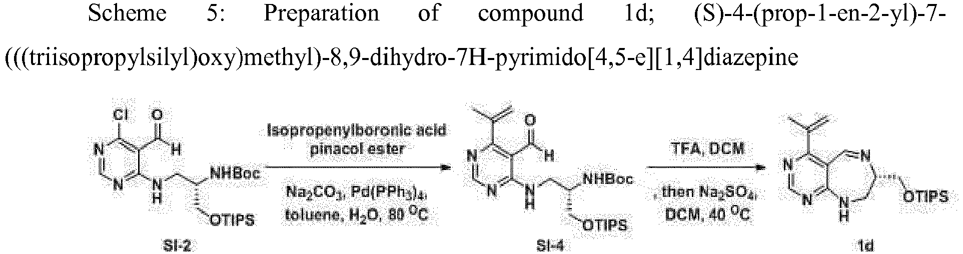

### Scheme 5-1)

SI-2 (500 mg, 1.026 mmol), isoprophenyl boronic acid pinacol ester (0.290 ml, 1.539 mmol), and sodium carbonate (380.6 mg, 3.591 mmol) were dissolved in toluene and water (w/w = 25.0 ml/25.0 ml). To the stirring solution was added tetrakis palladium (palladium tetrakis; i.e., triphenylphosphine; Pd((PPh)₃))₄), 119.0 mg, 0.103 mmol, 10 mol%) at r.t. The compound was heated and stirred at 80 °C, followed by the completion of the reaction as indicated by TLC. The reactant was dissolved with ethyl acetate, washed with brine, and then dried over anhydrous magnesium sulfate and filtered. The filtrate was condensed under reduced pressure and the reaction mixture was purified by silica-gel flash column chromatography to obtain SI-4 (379.2 mg, 75% yield).

### Scheme 5-2)

SI-4 (379.2 mg, 0.770 mmol) was added to 10% trifluoro acetic acid dichloromethane solution (100.0 ml) and stirred at r.t. After completion of the reaction as indicated by TLC, the reaction was quenched with a saturated sodium bicarbonate solution and extracted twice with dichloromethane. The extracted organic solution was dried with anhydrous sodium sulfate under reduced pressure and filtered. The compound was dissolved in dichloromethane (100.0 ml), adding sodium sulfate (2.915 g, 20.52 mmol), and then stirring at 40 °C. The completion of the reaction was indicated by TLC, followed by filterinig. The filtrate was condensed under reduced pressure and the product was purified with silica-gel flash column chromatography to obtain cyclic immine compound 1d (234.4 mg, 81% yield) a light colorless oil.

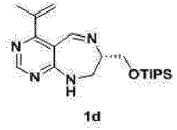

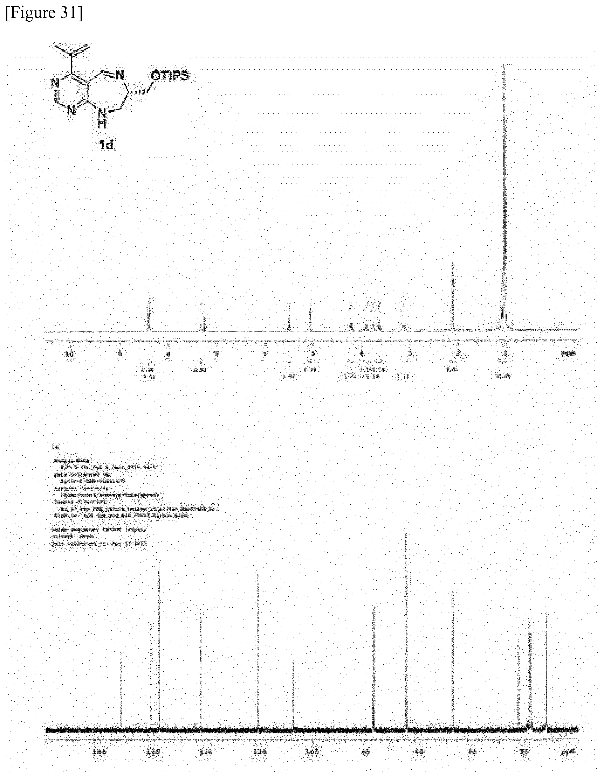

*R*_{f} = 0.2 (DCM/MeOH 20:1); ¹H NMR (400 MHz, a CDCl₃) δ 8.47 (d, *J* = 2.3 Hz, 1H), 8.43 (s, 1H), 7.37 (br d, *J* = 5.0 Hz, 1H), 5.54 (s, 1H), 5.10 (s, 1H), 4.27 (dd, *J* = 9.7, 4.7 Hz, 1H), 3.95 (dd, *J* = 13.0, 7.0 Hz, 1H), 3.81 (m, 1H), 3.69 (t, *J* = 10.1 Hz, 1H), 3.19 (m, 1H), 2.17 (s, 3H), 1.08 (m, 21H); ¹³C NMR (100 MHz, a CDCl₃) δ 172.2, 161.0, 158.0, 157.9, 142.2, 120.9, 107.3, 65.2, 64.8, 47.4, 22.3, 18.1, 11.9; HRMS(ESI+): Calcd for a C₂₀H₃₅N₄OSi⁺ [M+H]⁺ 375.2575, found 375.2574, Δppm -0.27. (Fig. 31).

### Example 1: Preparation of compound 2a

To a stirring solution where compound 1a (403.1 mg, 0.770 mmol) was added to methanol (8.0 ml), sodium borohydride (145.6 mg, 3.850 mmol) was added at 0 °C. The mixture was stirred at r.t. After completion of the reaction as indicated by TLC, the reaction was quenched with a saturated sodium bicarbonate solution and extracted twice with dichloromethane. The extracted organic solution was dried with anhydrous sodium sulfate under reduced pressure and filtered. The filtrate was condensed under reduced pressure and then dissolved in dichloromethane (8.0 ml). To the solution were added triethylamine (0.215 ml, 1.540 mmol) and di-tert-butyl dicarbonate (Boc₂O) (218.5 mg, 1.001 mmol) at 0 °C. The compound was stirred at r.t., followed by completion of the reaction as indicated by TLC. The reaction was quenched with a saturated ammonium chloride solution and the reaction solution was extracted twice with dichloromethane. The extracted organic solution was dried over anhydrous magnesium sulfate and filtered. The filtrate was then condensed under reduced pressure and the reaction mixture was purified by silica-gel flash column chromatography to obtain SI-5 (260.3 mg, 54% yield). SI-5 (260.3 mg, 0.416 mmol) was dissolved in DMF (4.0 ml), adding sodium hydride (60% dispersion in mineral oil, 33.28 mg, 0.832 mmol) under argon atmosphere at 0 °C and left to stir. After stirring over 30 min, ethyl iodide (0.067 ml, 0.832 mmol) was added and stirred at r.t. After completion of the reaction as indicated by TLC, the reaction was quenched with a saturated ammonium chloride solution. The reaction solution was extracted twice with ethyl acetate. The extracted organic solution was dried over anhydrous sodium sulfate and filtered. The filtrate was condensed under reduced pressure and the product was purified with silica-gel flash column chromatography to obtain intermediate A (258.5 mg, 95% yield). Intermediate A (258.5 mg, 0.395 mmol) was dissolved in methanol (4.0 ml), adding Pd(OH)₂/C (25.9 mg, 20 wt.%), and then stirring at r.t. while feeding hydrogen atmosphere of 1 atm. The completion of the reaction was as indicated by TLC, followed by filtering through Celite®. The filtrate was extracted with ethyl acetate. The filtrate was condensed under reduced pressure and dissolved in dichloromethane (4.0 ml). To the compound were triethylamine (0.165 ml, 1.185 mmol) and methanesulfonyl chloride (MsCl, 0.061 ml, 0.790 mmol) at 0 °C. The compound was stirred at r.t., followed by completion of the reaction as indicated by TLC. The compound after completion of the reaction was diluted with dichloromethane, washing twice with IN chloride chloride. The washed organic solution was dried over anhydrous sodium sulfate and filtered. The filtrate was condensed under reduced pressure and the product was purified with silica-gel flash column chromatography to obtain 2a (280.6 mg, 61 % yield) as a white solid.

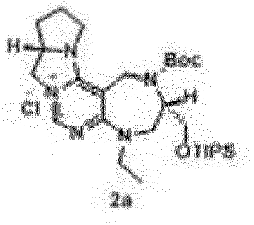

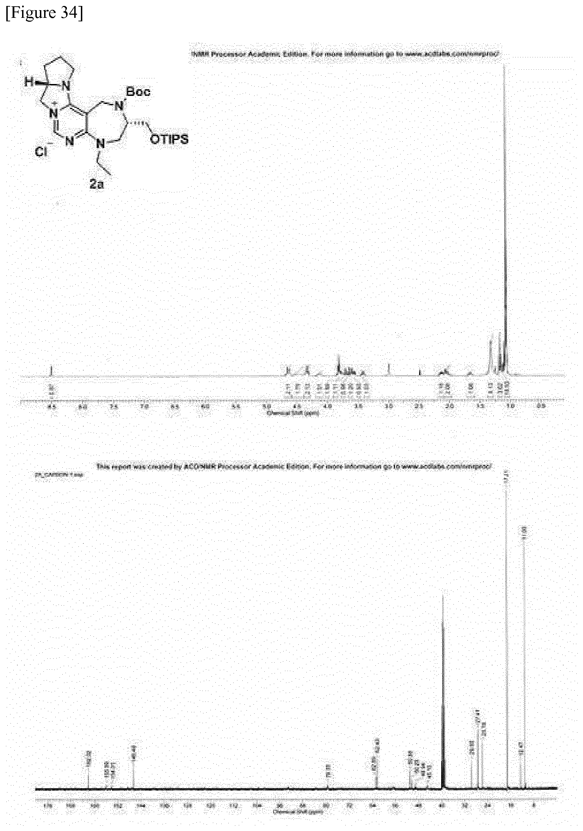

*R*_{f} = 0.1 (DCM/MeOH = 10:1); ¹H NMR (400 MHz, DMSO-*d₆*, 100 °C) δ 8.50 (s, 1H), 4.64 (m, 2H), 4.48 (m, 2H), 4.33 (m, 2H), 4.13 (m, 1H), 3.82 (m, 2H), 3.76 (m, 1H), 3.69 (m, 1H), 3.62 (dd, *J* = 15.1, 4.6 Hz, 1H), 3.57 (m, 1H), 3.42 (m, 1H), 2.14 (m, 1H), 2.04 (m, 2H), 1.66 (m, 1H), 1.32 (br s, 9H), 1.17 (t, *J* = 7.1 Hz, 3H), 1.08 (m, 21H); ¹³C NMR (100 MHz, DMSO-*d₆*, 100 °C) δ 162.0, 155.8, 154.0, 146.5, 79.3, 62.8, 62.4., 50.8, 50.2, 48.9, 45.1, 29.6, 27.4, 25.7, 17.2, 12.4, 11.0; IR (neat) vₘₐₓ: 2940, 2866, 1698, 1638, 1523, 1174 cm⁻¹; HRMS(ESI+): Calcd for a C₂₉H₅₂N₅O₃Si⁺ [M]⁺ 546.3834, found 546.3831, Δppm -0.55; mp: 136-138 °C. (Fig. 34).

### Example 2: Preparation of compound 3b

Compound 3b was prepared based on Preparing Example 5, provided that 1b was used in place of 1a.

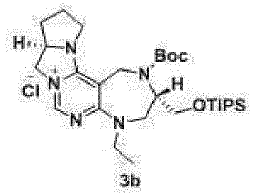

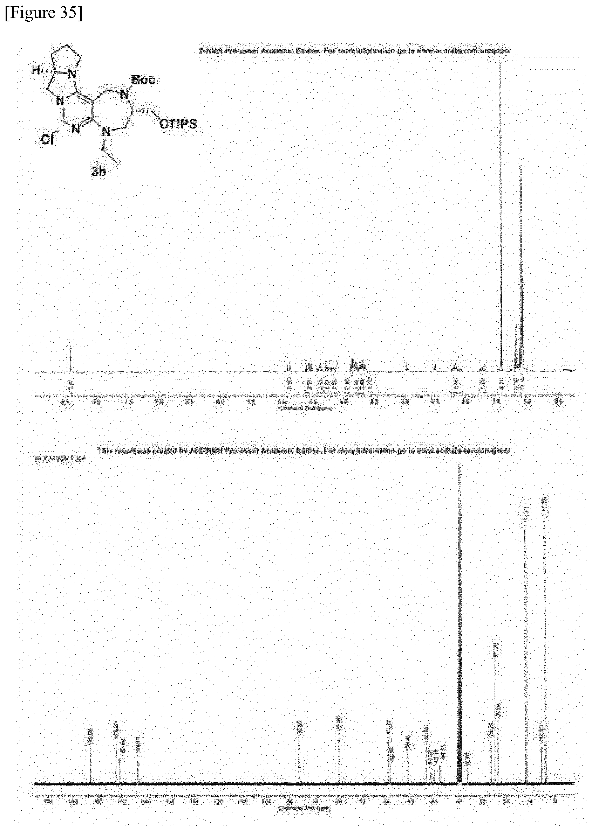

A pale yellow solid; *R*_{f} = 0.1 (DCM/MeOH = 10:1); 38% overall yield; ¹H NMR (400 MHz, DMSO-*d₆*, 100 °C) δ 8.42 (s, 1H), 4.88 (d, *J* = 16.9 Hz, 1H), 4.58 (d, *J* = 16.9 Hz, 1H), 4.54 (dd, *J* = 10.8, 9.4 Hz, 1H), 4.38 (m, 2H), 4.27 (dd, *J* = 10.9, 8.2 Hz, 1H), 4.16 (dd, *J* = 15.1, 11.9 Hz, 1H), 3.86 (m, 2H), 3.80 (m, 2H), 3.70 (m, 2H), 3.65 (m, 1H), 2.17 (m, 3H), 1.73 (m, 1H), 1.41 (s, 9H), 1.18 (t, *J* = 6.9 Hz, 3H), 1.09 (m, 21H); ¹³C NMR (100 MHz, DMSO-*d₆*, 100 °C) δ 162.3, 153.8, 152.8, 146.5, 93.0, 79.8, 63.2, 62.5, 56.9, 50.6, 49.0, 48.0, 46.1, 36.7, 29.2, 27.5, 26.6, 17.2, 12.3, 10.9; IR (neat) vₘₐₓ: 2944, 2863, 1698, 1636, 1517, 1166 cm⁻¹; HRMS(ESI+): Calcd for a C₂₉H₅₂N₅O₃Si⁺ [M]⁺ 546.3834, found 546.3833, Δppm -0.18; mp: 77-79 °C. (Fig. 35).

### Example 3: Preparation of compound 4c

Compound 4c was prepared based on Preparing Example 5, provided that 1c was used in place of 1a.

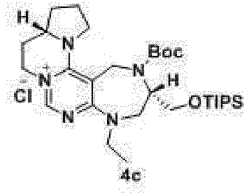

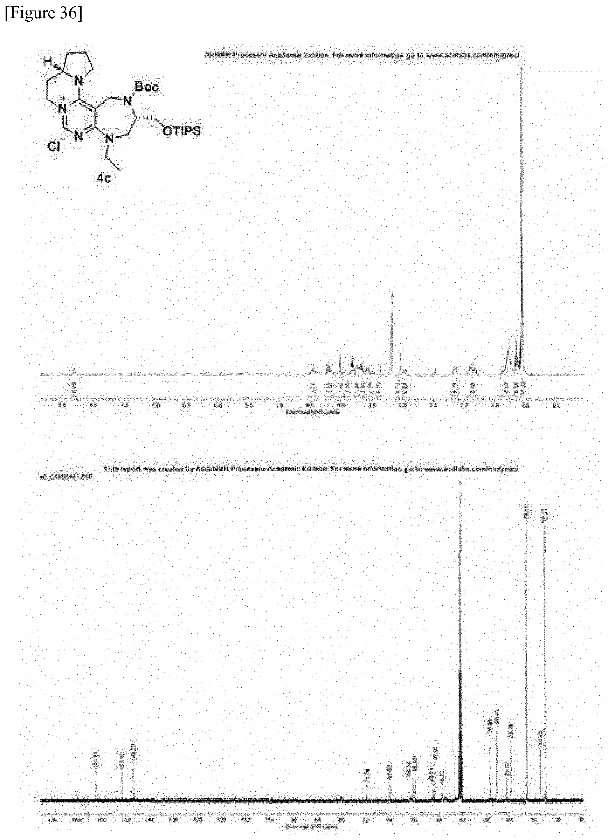

A pale yellow solid; *R*_{f} = 0.2 (DCM/MeOH = 10:1); 40 % overall yield; ¹H NMR (400 MHz, DMSO-*d₆*, 100 °C) δ 8.34 (s, 1H), 4.48 (m, 2H), 4.23 (m, 2H), 4.05 (m, 1H), 3.85 (m, 2H), 3.79 (m, 2H), 3.71 (m, 3H), 3.61 (dd, *J* = 15.0, 4.6 Hz, 1H), 3.53 (m, 1H), 3.06 (br s, 1H), 3.00 (m, 1H), 2.18 (m, 1H), 1.91 (m, 3H), 1.29 (br s, 9H), 1.18 (t, *J* = 7.0 Hz, 3H), 1.09 (m, 21H); ¹³C NMR (100 MHz, DMSO-*d₆*, 100 °C) δ 161.5, 153.1, 149.2, 149.2, 71.7, 63.9, 56.3, 55.8, 49.7, 49.0, 46.8, 30.5, 28.4, 25.0, 23.6, 18.2, 13.7, 12.0; IR (neat) vₘₐₓ: 2946, 2867, 1694, 1625, 1529, 1457, 1116 cm⁻¹; HRMS(ESI+): Calcd for a C₃₀H₅₄N₅O₃Si⁺ [M]⁺ 560.3990, found 560.3993, Δppm -0.54.; mp: 71-73 °C. (Fig. 36).

### Example 4: Preparation of compound 5d

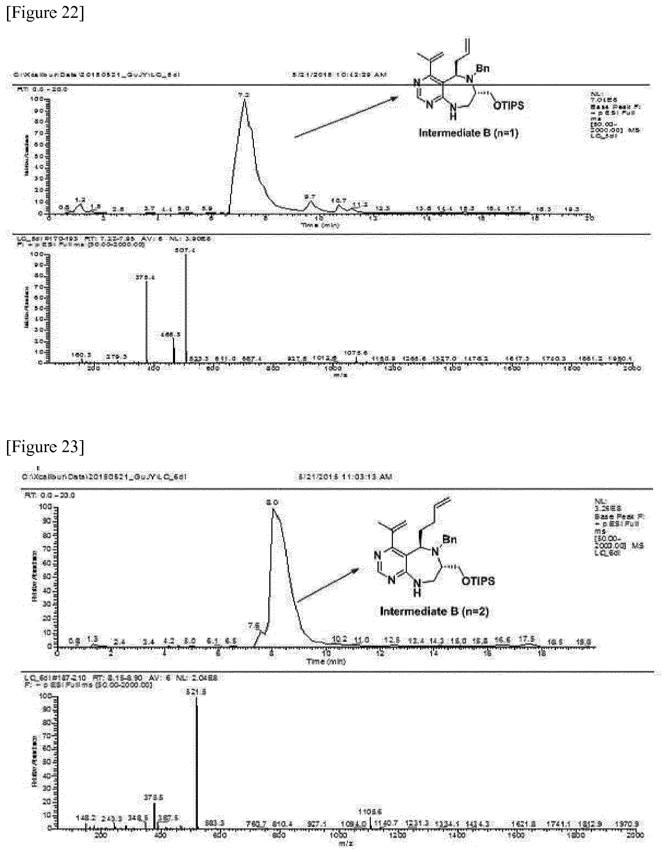

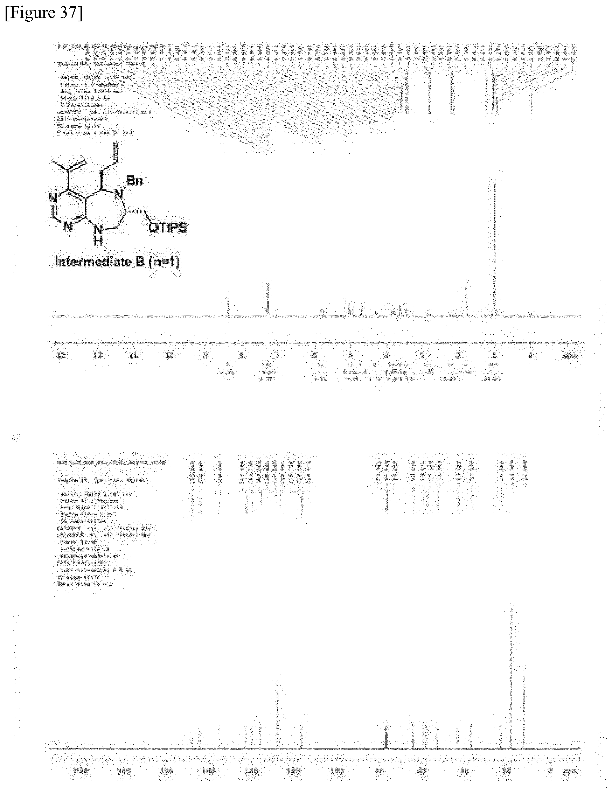

1) Formation of intermediate B: 1d (100 mg, 0.267 mmol) was dissolved in acetonitrile (13.5 ml) with stirring and to this solution was added benzyl bromide (0.048 ml, 0.401 mmol). The compound was stirred at 80 °C, followed by completion of the reaction as indicated by TLC. The compound after completion of the reaction was condensed under reduced pressure, washed with hexane to remove any excess benzyl bromide, and then condensed. The iminium ion was dissolved in cold anhydrous THF (27.0 ml), to which was added allylmagnesium bromide solution (1.0 M diethyl ether, 1.335 ml, 1.335 mmol) dropwise over 30 min at -78 °C. The compound was then stirred for 18 hours at r.t. The reaction was quenched with a saturated ammonium chloride solution and the reaction solution was extracted twice with dichloromethane. The extracted organic solution was dried over anhydrous magnesium sulfate and filtered. The filtrate was was condensed under reduced pressure and the reaction mixture was purified by silica-gel flash column chromatography to obtain intermediate B (n= 1, 115.0 mg, yield 85%, d.r. >99:1) as a pale yellow oil. 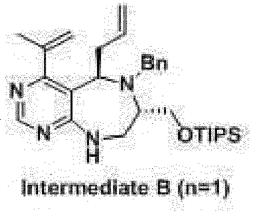
   *R*_{f} = 0.5 (hexane/EtOAc = 1:1); ¹H NMR (400 MHz, a CDCl₃) δ 8.40 (s, 1H), 7.32-7.29 (m, 3H), 7.24-7.20 (m, 2H), 5.88-5.78 (m, 2H), 5.03 (m, 2H), 4.94 (s, 1H), 4.69 (s, 1H), 4.29 (dd, *J* = 9.4, 5.9 Hz, 1H), 3.86 (d, *J* = 15.3 Hz, 1H), 3.78 (dd, *J* = 9.6, 3.7 Hz, 1H), 3.64-3.57 (m, 3H), 3.50-3.40 (m, 2H), 2.84 (m, 1H), 2.22 (m, 1H), 1.81 (s, 3H), 1.01 (s, 21H); ¹³C NMR (100 MHz, a CDCl₃) δ 168.4, 164.6, 155.6, 143.0, 140.1, 136.0, 128.4, 127.7, 126.9, 116.7, 116.3, 116.0, 64.4, 59.3, 57.9, 53.0, 43.3, 37.1, 23.0, 18.1, 11.9; HRMS(ESI+): Calcd for a C₃₀H₄₇N₄OSi⁺ [M+H]⁺ 507.3514, found 507.3516, Δppm +0.39.. (Fig. 37)
   The diastereomeric ratio was determined by LC-MS analysis of crude compound, while 1H NMR was analyzed after silica-gel column (Figs. 22 and 37).
2) Preparation of compound 5d : To intermediate B (115.0 mg, 0.227 mmol) dissolved in toluene (11.5 ml) was added 2^{nd} generation Grubbs' catalyst (38.20 mg, 0.045 mmol, 20 mol%) and left to stir at reflux. After completion of the reaction as indicated by TLC, the organic solution was condensed under reduced pressure, and the reaction mixture was purified by silica-gel flash column chromatography to obtain desired compound 5d (79.34 mg, yield 73 %) as a pale yellow oil.

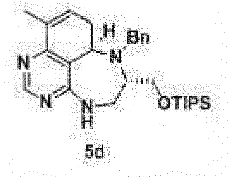

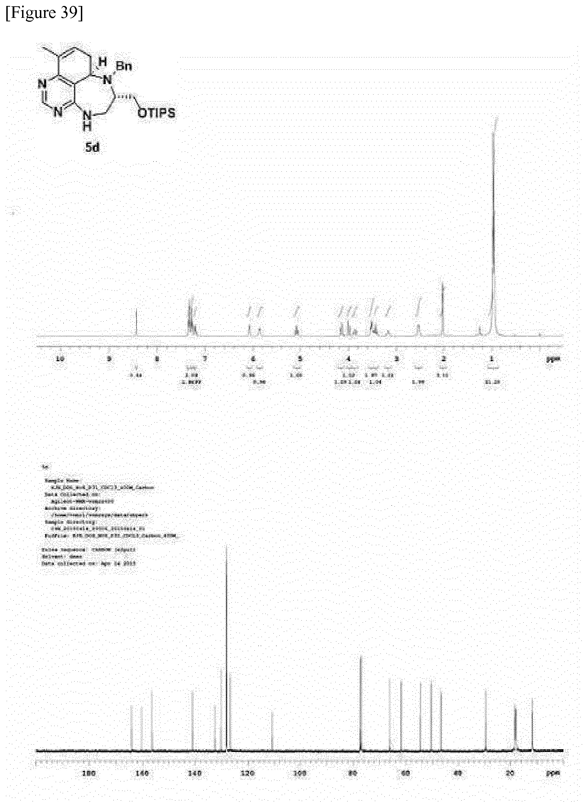

*R*_{f} = 0.4 (hexane/EtOAc = 2:1); ¹H NMR (400 MHz, a CDCl₃) δ 8.43 (s, 1H), 7.34-7.18 (m, 5H), 6.08 (br s, 1H), 5.87 (d, 6.3 Hz, 1H), 5.08 (t, *J* = 11.0 Hz, 1H), 4.16, 3.99 (ABq, *J*_{AB} = 14.9 Hz, 2H), 3.87 (t, *J* = 13.3 Hz, 1H), 3.54-3.41 (m, 3H), 3.17 (m, 1H), 2.54 (d, *J* = 10.5 Hz, 2H), 2.03 (s, 3H), 0.97 (m, 21 H); ¹³C NMR (100 MHz, a CDCl₃) δ 164.2, 160.4, 156.4, 141.1, 132.6, 130.4, 128.3, 128.1, 126.7, 110.9, 66.1, 61.8, 54.6, 50.5, 46.7, 29.5, 18.3, 17.9, 11.8; IR (neat) vₘₐₓ: 3241, 3028, 2940, 2865, 1565, 1460, 1116, 1066 cm⁻¹; HRMS(ESI+): Calcd for a C₂₈H₄₃N₄OSi⁺ [M+H]⁺ 479.3201, found 479.3206, Δppm +1.04.(Fig. 39).

Through Nuclear Overhauser Effect (NOE) spectroscopy, 1D-NOE was analyzed to confirm a chiral center (Fig. 6).

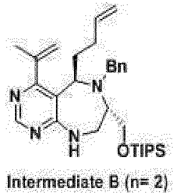

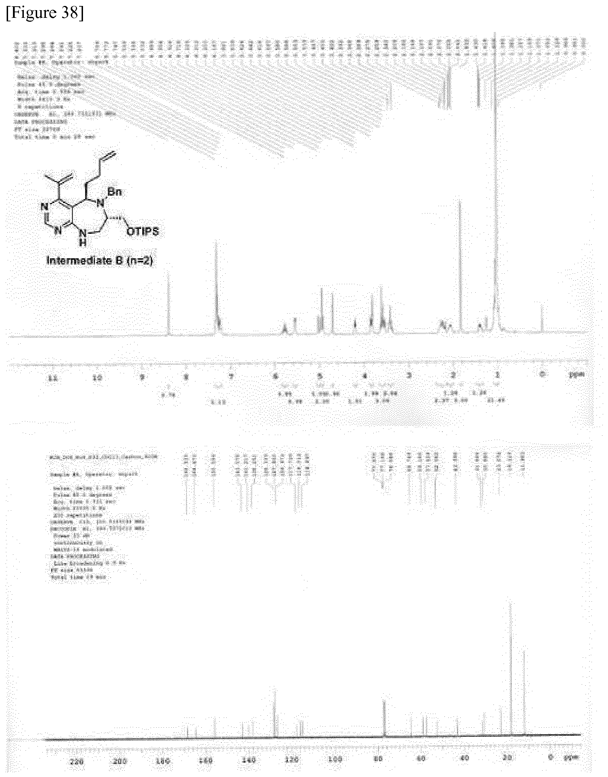

A yellow oil; *R*_{f} = 0.5 (hexane/EtOAc = 1:1); 120.8 mg, 87% yield; d.r. >99:1; ¹H NMR (400 MHz, a CDCl₃) δ 8.40 (s, 1H), 7.33-7.21 (m, 5H), 5.78 (m, 1H), 5.55 (d, *J* = 5.1 Hz, 1H), 5.01 (dd, *J* = 17.2, 1.2 Hz, 1H), 4.94 (m, 2H), 4.72 (s, 1H), 4.21 (dd, *J* = 10.2, 5.1 Hz, 1H), 3.84 (m, 2H), 3.64-3.54 (m, 3H), 3.48-3.37 (m, 2H), 2.33-2.17 (m, 2H), 2.11-2.04 (m, 1H), 1.83 (s, 3H), 1.45-1.36 (m, 1H), 1.06 (m, 21H); ¹³C NMR (100 MHz, a CDCl₃) δ 168.5, 164.7, 155.6, 143.1, 140.2, 138.2, 128.4, 127.8, 127.0, 117.7, 116.0, 114.9, 64.7, 59.2, 57.5, 52.6, 43.4, 31.7, 30.9, 23.1, 18.1, 12.0; HRMS(ESI+): Calcd for a C₃₁H₄₉N₄OSi⁺ [M+H]⁺ 521.3670, found 521.3670. (Fig. 38)

Intermediate B (n=2) was synthesized by using 1d and butyl magnesium bromide solution (0.2 M TFH). The diastereomeric ratio was determined by LC-MS analysis of crude compound, while 1H NMR was analyzed after silica-gel column (Figs. 23 and 38).

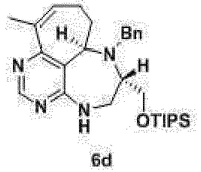

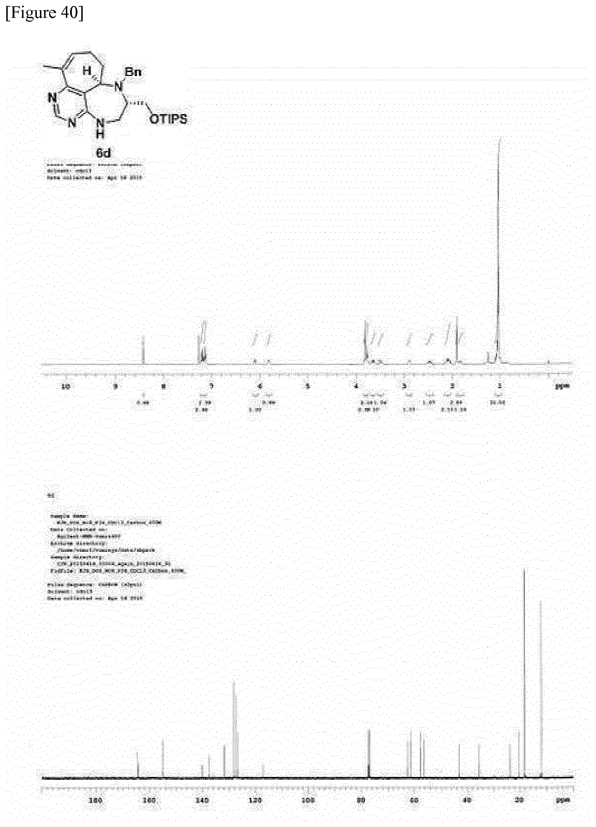

A yellow oil; *R*_{f} = 0.4 (hexane/EtOAc = 2:1); 87.95 mg, 72% yield, 63% overall yield; ¹H NMR (400 MHz, a CDCl₃) δ 8.42 (s, 1H), 7.22-7.12 (m, 5H), 6.10 (t, *J* = 6.7 Hz, 1H), 5.82 (d, *J* = 5.5 Hz, 1H), 3.84-3.76 (m, 5H), 3.65 (dd, *J* = 11.7, 7.0 Hz, 1H), 3.49 (m, 1H), 2.89 (m, 1H), 2.48 (m, 1H), 2.10 (m, 2H), 1.91 (s, 3H), 1.84 (m, 1H), 1.26 (m, 21H); ¹³C NMR (100 MHz, a CDCl₃) δ 164.5, 164.1, 154.9, 140.0, 137.4, 131.8, 128.1, 127.4, 126.7, 117.1, 62.3, 61.2, 57.6, 56.3, 43.0, 35.4, 23.6, 20.1, 17.9, 11.8; IR (neat) vₘₐₓ: 3241, 2944, 2865, 1570, 1462, 1102, 883 cm⁻¹; HRMS(ESI+): Calcd for a C₂₉H₄₅N₄OSi⁺ [M+H]⁺ 493.3357, found 493.3349, Δppm +0.41..(Fig. 40)

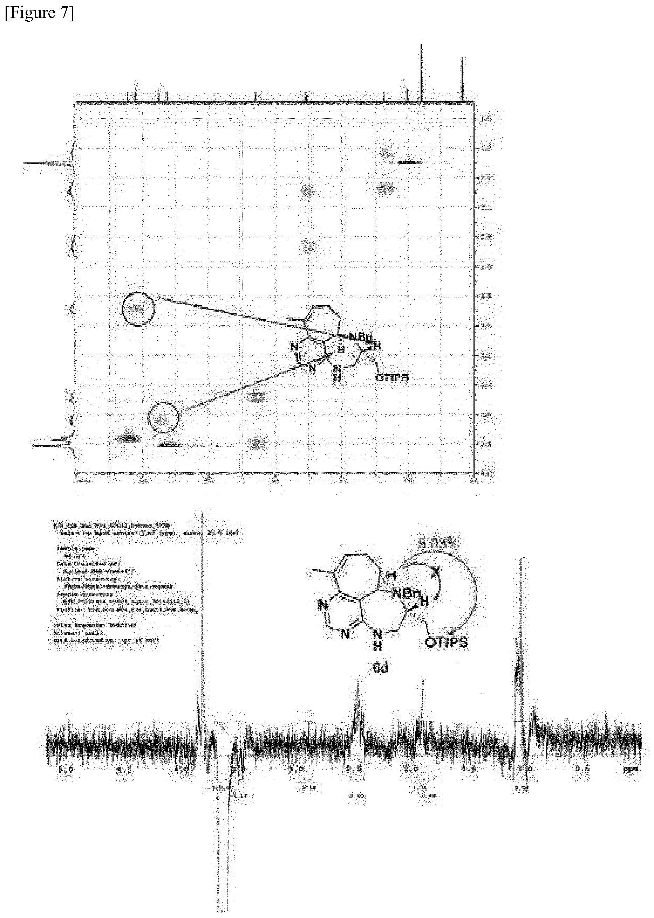

Through Nuclear Overhauser Effect (NOE) spectroscopy, 1D-NOE was analyzed to confirm a chiral center of 6d (Fig. 7).

### Example 6 : Preparation of compound 7e

1e (10 mg, 0.223 mmol) was dissolved in acetonitrile (11.0 ml) with stirring and to this solution was added benzyl bromide (0.040 ml, 0.335 mmol). The compound was stirred at 80 °C, followed by completion of the reaction as indicated by TLC. The compound after completion of the reaction was condensed under reduced pressure, washed with hexane to remove any excess benzyl bromide, and then condensed. The iminium ion was dissolved in cold anhydrous THF (22.0 ml), to which was added 3-butyl magnesium bromide solution (0.2 M THF, 5.575 ml, 1.115 mmol) dropwise over 30 min at -78 °C. The compound was then stirred for 18 hours at r.t. The reaction was quenched with a saturated ammonium chloride solution and the reaction solution was extracted twice with dichloromethane. The extracted organic solution was dried over anhydrous magnesium sulfate and filtered. The filtrate was condensed under reduced pressure and the reaction mixture was purified by silica-gel flash column chromatography to obtain intermediate C (122.0 mg, yield 80%, d.r. 95:5). The diastereomeric ratio was determined by LC-MS analysis of crude compound (Fig. 25). To intermediate C (122.0 mg, 0.178 mmol) dissolved in toluene (11.5 ml) was added 2^{nd} generation Grubbs' catalyst (30.22 mg, 0.036 mmol, 20 mol%) and left to stir at reflux. After completion of the reaction as indicated by TLC, the organic solution was condensed under reduced pressure and the reaction mixture was purified by silica-gel flash column chromatography to obtain desired compound 7e (85.24mg, yield 73 %) as a pale yellow oil.

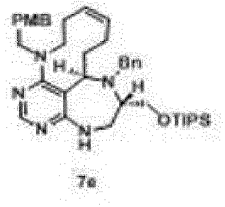

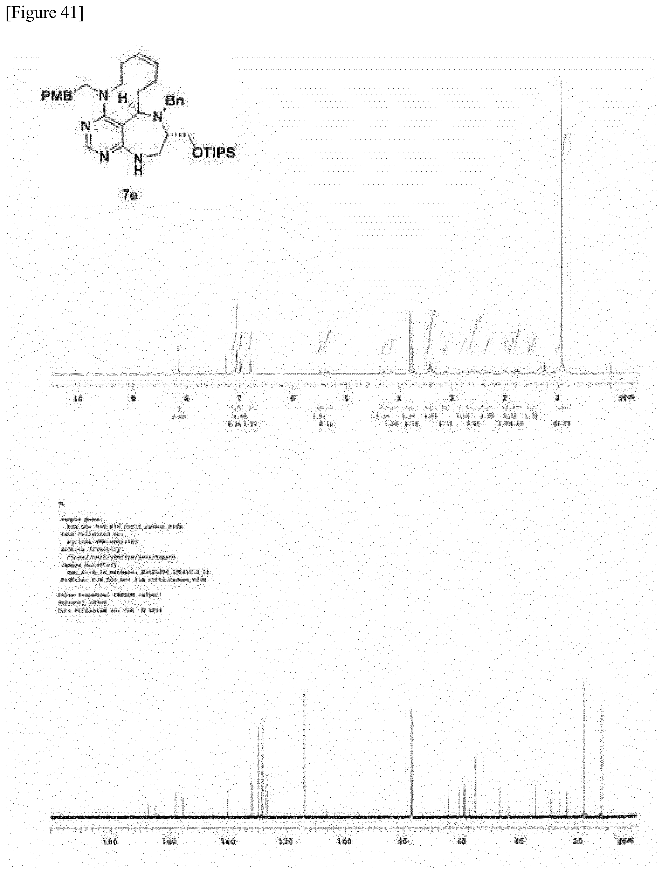

*R*_{f} = 0.2 (hexane/EtOAc = 3:1); ¹H NMR (400 MHz, a CDCl₃) δ 8.15 (s, 1H), 7.15-7.05 (m, 5H), 6.99 (d, *J* = 8.6 Hz, 2H), 6.80 (d, *J* = 8.2 Hz, 2H), 5.47 (m, 1H), 5.37 (m, 2H), 4.30 (dd, *J* = 11.9, 2.2 Hz, 1H), 4.13 (m, 1H), 3.80 (s, 3H), 3.75 (m, 2H), 3.72 (m, 1H), 3.39 (m, 4H), 3.11 (m, 1H), 2.80 (m, 1H), 2.59 (m, 3H), 2.33 (m, 1H), 2.01 (m, 1H), 1.88 (m, 1H), 1.77 (m, 2H), 1.50 (m, 1H), 0.92 (m, 21H); ¹³C NMR (100 MHz, a CDCl₃) δ 167.4, 165.0, 158.0, 155.4, 140.2, 132.1, 131.6, 129.8, 128.8, 128.3, 128.2, 126.8, 113.9, 106.1, 64.5, 60.9, 59.2, 59.1, 57.6, 55.3, 46.9, 43.8, 29.2, 26.3, 23.8, 18.0, 11.9; IR (neat) vₘₐₓ: 3245, 3000, 2942, 2865, 1572, 1512, 1247, 1113 cm⁻¹; HRMS(ESI+): Calcd for a C₃₉H₅₈N₅O₂Si⁺ [M+H]⁺ 656.4354, found 656.4345, Δppm -1.37. (Fig. 41).

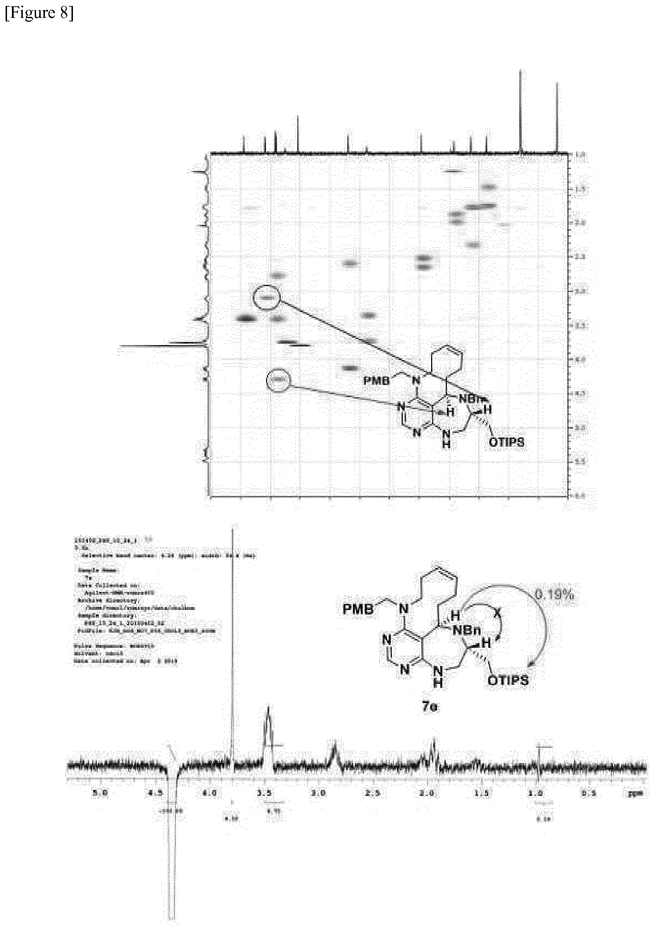

Through Nuclear Overhauser Effect (NOE) spectroscopy, 1D-NOE was analyzed to confirm a chiral center of 7e (Fig. 8).

### Example 7: Preparation of compound 8e

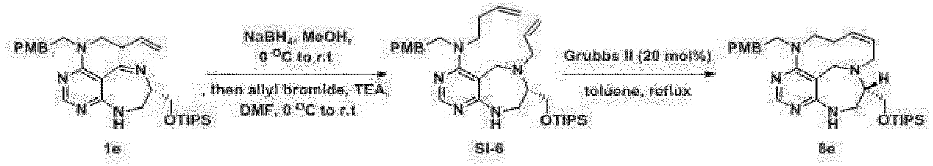

A solution of 1e (120 mg, 0.223 mmol) in methanol (MeOH, 2.2ml) was treated with sodium borohydride (NaBH₄, 42.18 mg, 1.115 mmol) at 0 °C and stirred between 0 °C and 25°C. After completion of the reaction as indicated by TLC, the mixture was quenched with addition of sodium bicarbonate (NaHCO₃) solution. The reaction solution was extracted twice with dichloromethane (DCM) and the extracted organic solution was dried over anhydrous sodium sulfate (Na₂SO₄) and filtered. The filtrate was then evaporated under reduced pressure, condensed, and dried. The compound was dissolved in DMF (2.2 ml), followed sequentially adding triethylamine (TEA, 0.055 ml, 0.396 mmol) and allyl bromide (0.025 ml, 0.290 mmol) at 0 °C and stirring between 0 °C and 25°C. After completion of the reaction as indicated by TLC, the mixture was quenched with addition of ammonium chloride (NH₄Cl) solution. The reaction solution was extracted twice with ethyl acetate (EA) and the extracted organic solution was dried over anhydrous sodium sulfate (Na₂SO₄) and filtered. The filtrate was then condensed under reduced pressure and the product was purified with silica-gel flash column chromatography to obtain compound SI-6 (81.47 mg, 63% yield).

A solution of SI-6 (81.47 mg, 0.140 mmol) in toluene (28.0 ml) was treated with second generation Grubbs' catalyst (23.77 mg, 0.028 mmol, 20 mol%) and left at reflux. After completion of the reaction as indicated by TLC, the residual solvent was removed under reduced pressure and the product was purified with silica-gel flash column chromatography to obtain compound 8e (54.08 mg, 70% yield, 44% overall yield) as a yellow oil.

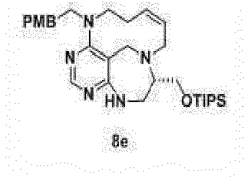

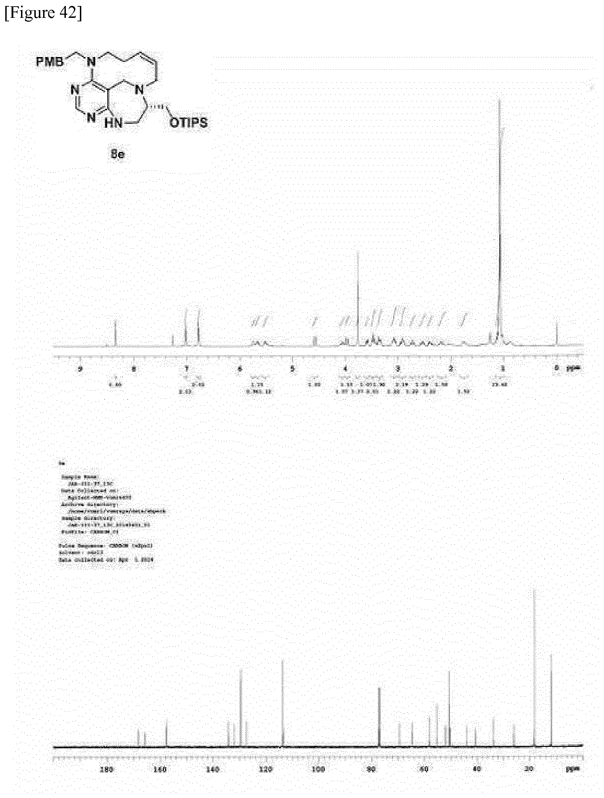

*R*_{f} = 0.3 (DCM/MeOH = 20:1); ¹H NMR (400 MHz, a CDCl₃) δ 8.35 (s, 1H), 7.02 (d, *J* = 8.6 Hz, 2H), 6.79 (d, *J* = 8.2 Hz, 2H), 5.74 (br d, *J* = 5.1 Hz, 1H), 5.67 (td, *J* = 11.2, 4.5 Hz, 1H), 5.51 (td, *J* = 10.9, 4.5 Hz, 1H), 4.58 (d, *J* = 16.0 Hz, 1H), 4.06 (ddd, *J* = 14.6, 10.9, 3.1 Hz, 1H), 3.97 (d, *J* = 16.0 Hz, 1H), 3.76 (s, 3H), 3.59 (dd, *J* = 10.0, 4.1 Hz, 1H), 3.46 (m, 2H), 3.37 (m, 2H), 3.08 (m, 2H), 2.92 (m, 2H), 2.73 (m, 1H), 2.54 (m, 1H), 2.39 (ddd, *J* = 13.4, 10.7, 5.7, 1H), 2.18 (m, 1H), 1.76 (br d, *J* = 12.1 Hz, 1H), 1.07 (m, 21H); ¹³C NMR (100 MHz, a CDCl₃) δ 168.2, 165.8, 157.9, 157.7, 134.3, 132.0, 129.5, 127.4, 113.7, 113.3, 69.4, 64.7, 58.1, 55.3, 52.0, 50.5, 43.9, 40.5, 33.8, 25.9, 18.0, 11.9; IR (neat) vₘₐₓ: 3239, 3006, 2945, 2866, 1574, 1512, 1265 cm⁻¹; HRMS(ESI+): Calcd for a C₃₁H₅₀N₅O₂Si⁺ [M+H]⁺ 552.3728, found 552.3722, Δppm -1.09. (Fig. 42).

### Example 8: Preparation of compounds 9f and 10f

In microwave reaction vessel, 1f (90mg, 0.198 mmol), alkyne (0.5 mmol), 4-picoline N-oxide (54.57mg, 0.5 mmol), and Rh(PPh₃)₃Cl (18.50 mg, 0.020 mmol, 10 mol%) were dissolved in ACN (3.0 ml) and subjected to the reaction for 35 minutes at 100 watt and 90 °C. DCM was added to the mixture and washed with brine. The extracted organic solution was dried over anhydrous sodium sulfate (Na₂SO₄) and filtered. The filtrate was then evaporated under reduced pressure, condensed, and dried. The product was purified with silica-gel flash column chromatography to obtain compound 9f or 10f.

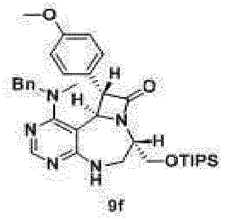

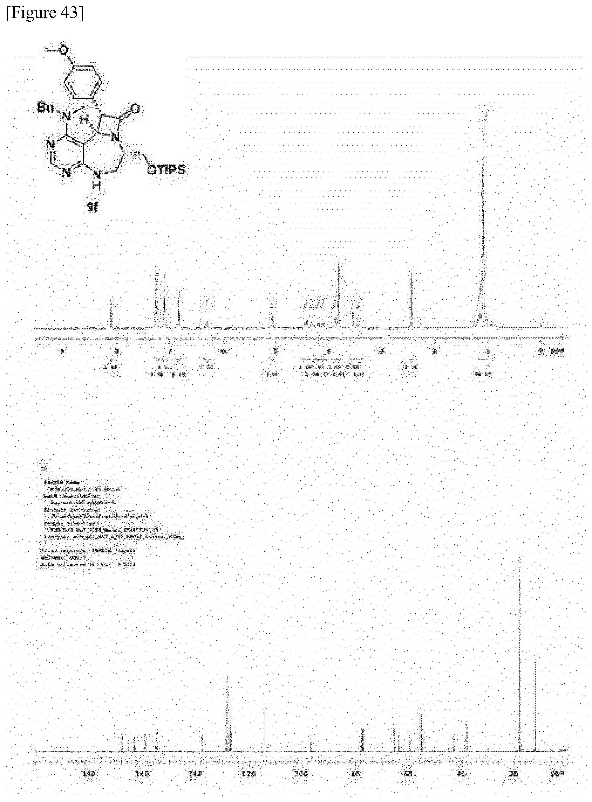

A light yellow oil; *R*_{f} = 0.3 (DCM/MeOH = 20:1); 72.69 mg, 61% yield; d.r. >99:1; ¹H NMR (400 MHz, a CDCl₃) δ 8.10 (s, 1H), 7.25 (m, 3H), 7.11 (d, *J* = 8.2 Hz, 4H), 6.83 (d, *J* = 8.2 Hz, 2H), 6.30 (dd, *J* = 6.5, 4.5 Hz, 1H), 5.06 (s, 1H), 4.43, 4.31 (ABq, *J*_{AB} = 14.9 Hz, 2H), 4.21 (dd, *J* =10.2, 3.5 Hz, 1H), 4.12 (m, 1H), 3.87 (m, 2H). 3.81 (s, 3H), 3.56 (s, 1H), 3.43 (ddd, *J* = 14.2, 7.3, 4.3 Hz, 1H), 2.44 (s, 3H), 1.09 (m, 21H); ¹³C NMR (100 MHz, a CDCl₃) δ 168.0, 165.3, 163.0, 159.2, 155.0, 137.5, 129.0, 128.5, 128.4, 127.3, 127.1, 114.1, 96.9, 65.2, 63.5, 59.5, 55.3, 55.2, 54.2, 42.9, 38.1, 18.0, 11.8; IR (neat) vₘₐₓ: 3250, 3056, 2942, 2865, 1751, 1567, 1119 cm⁻¹; HRMS(ESI+): Calcd for a C₃₄H₄₈N₅O₃Si⁺ [M+H]⁺ 602.3521, found 602.3528, Δppm +1.16. (Fig. 43).

The diastereomeric ratio was determined by LC-MS analysis of crude compound (Fig. 20).

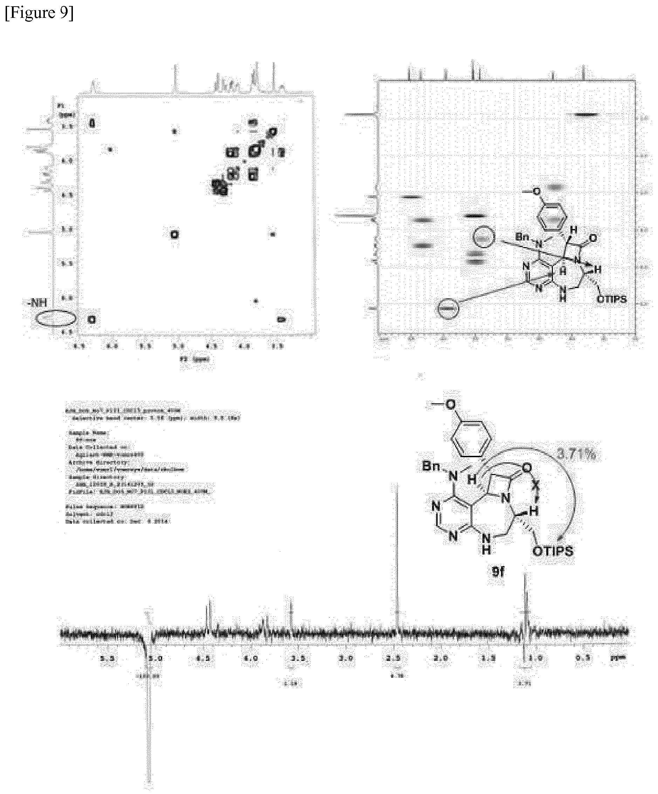

Through Nuclear Overhauser Effect (NOE) spectroscopy, 1D-NOE was analyzed to confirm a chiral center of 9f (Fig. 9).

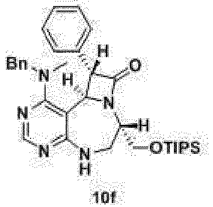

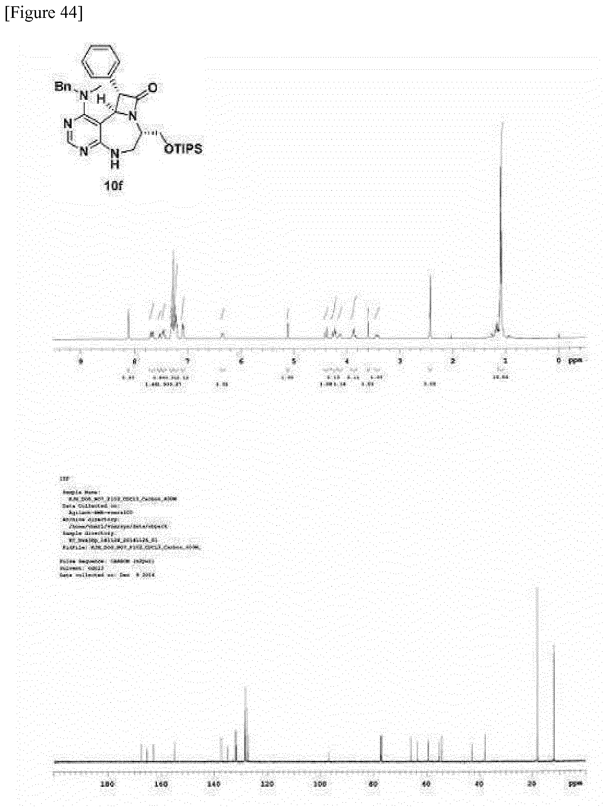

A light yellow oil; *R*_{f} = 0.3 (DCM/MeOH = 20:1); 54.35 mg, 48% yield; d.r. 90:10; ¹H NMR (400 MHz, a CDCl₃) δ 8.12 (s, 1H), 7.70-7.65 (m, 1H), 7.53 (m, 1H), 7.46 (m, 1H), 7.30 (m, 2H), 7.23 (m, 3H), 7.09 (m, 2H), 6.35 (dd, *J* = 6.7, 4.7 Hz, 1H), 5.11 (d, *J* = 1.2 Hz, 1H), 4.39 (m, 1H), 4.23 (m, 2H), 4.13 (m, 1H), 3.87 (m, 2H), 3.60 (d, *J* = 1.5 Hz, 1H), 3.43 (ddd, *J* = 14.4, 7.3, 4.5 Hz, 1H), 2.42 (s, 3H), 1.10 (m, 21H); ¹³C NMR (100 MHz, a CDCl₃) δ 167.6, 165.4, 163.0, 155.0, 137.4, 135.0, 132.1, 132.0, 128.7, 128.4, 127.8, 127.2, 96.9, 65.9, 63.5, 59.4, 55.2, 54.3, 42.8, 37.9, 18.0, 11.8; IR (neat) vₘₐₓ: 3239, 3059, 3030, 2942, 2866, 1755, 1567, 1108 cm⁻¹; HRMS(ESI+): Calcd for a C₃₃H₄₆N₅O₂Si⁺ [M+H]⁺ 572.3415, found 572.3413, Δppm -0.35; mp: 86-88 °C. (Fig. 44).

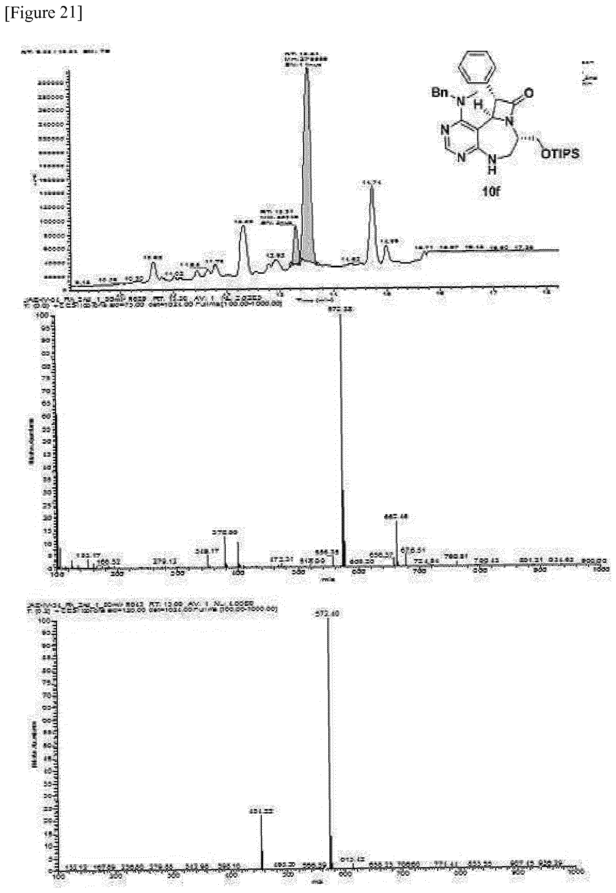

The diastereomeric ratio was determined by LC-MS analysis of crude compound (Fig. 21).

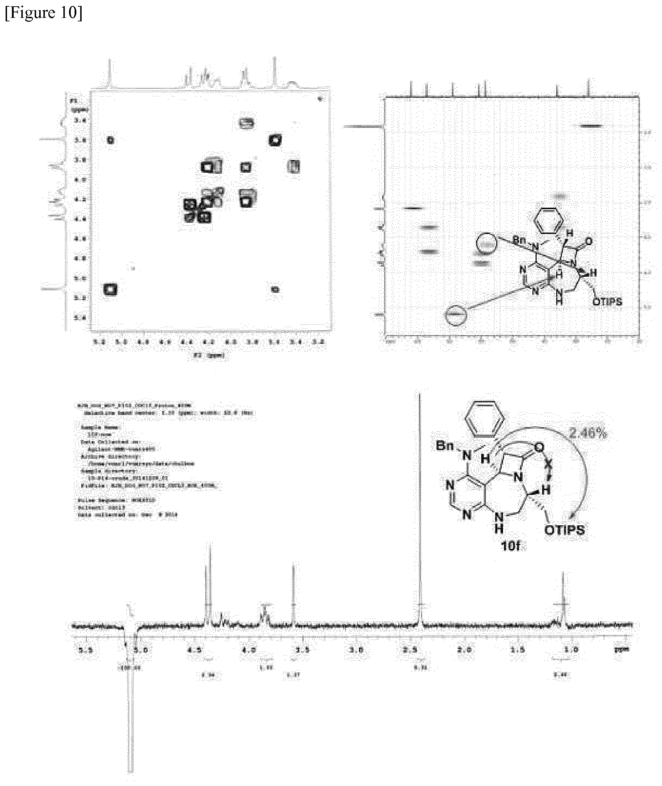

Through Nuclear Overhauser Effect (NOE) spectroscopy, 1D-NOE was analyzed to confirm a chiral center of 10f (Fig. 10).

### Example 9: Preparation of compound 11f

In microwave reaction vessel, 1f (90mg, 0.198 mmol), N-benzyl-2-chloroacetamide (54.54 mg, 0.297 mmol), and NaBr (30.56 mg, 0.297 mmol) were dissolved in DMF (3.0 ml) and subjected to the reaction over 30 minutes at 150 watt and 110 °C. The mixture was cooled down to r.t., followed by adding 1,8-Diazabicyclo[5.4.0]undec-7-ene (DBU, 0.059 ml, 0.396 mmol) and stirring at r.t. After completion of the reaction as indicated by TLC, the mixture was quenched with addition of sodium bicarbonate (NaHCO3) solution. The reaction solution was extracted twice with ethyl acetate (EA) and he extracted organic solution was dried over anhydrous sodium sulfate (Na2SO4) and filtered. The filtrate was then evaporated under reduced pressure, condensed, and dried. The product was purified by HPLC to obtain 11f (72.57 mg, 61%, d.r. 6:1 (diastereomeric ratio)) as a pale yellow oil.

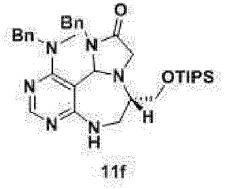

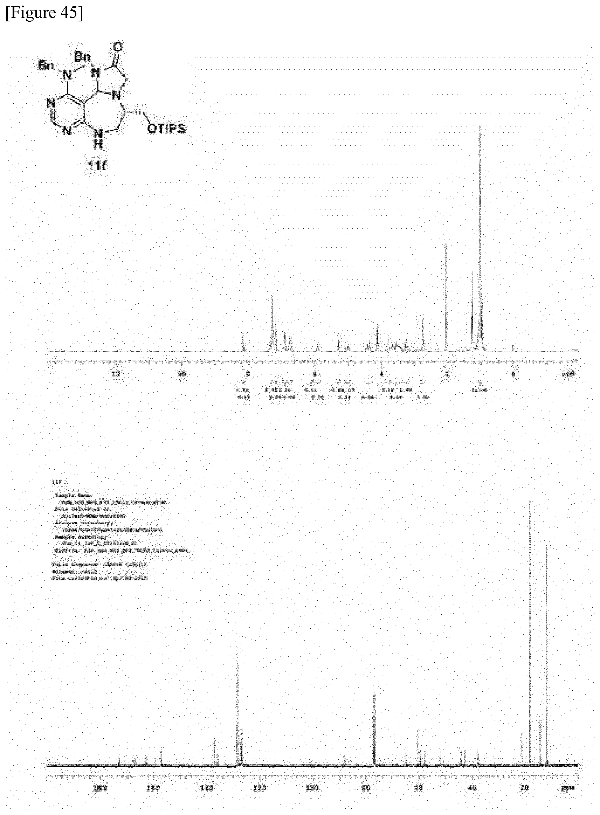

*R*_{f} = 0.3 (hexane/EtOAc = 1:1); ¹H NMR (400 MHz, a CDCl₃) δ 8.17 (s, 0.83H, *major diastereomer*), 8.12 (s, 0.13H, *minor diastereomer*), 7.28 (br s, 3H), 7.19 (br s, 3H), 6.89 (br s, 2H), 6.73 (br s, 2H), 6.11 (br s, 0.12H, *minor diastereomer*)*,* 5.90 (s, 0.78H, *major diastereomer*), 5.28 (s, 0.84H, *major diastereomer*), 5.08 (s, 0.13H, *minor diastereomer*)*,* 4.98 (m, 1H), 4.44, 4.34 (ABq, *J*_{AB} = 16.2 Hz, 2H), 3.78 (m, 2H), 3.66-3.37 (m, 4H), 3.24 (m, 2H), 2.73 (m, 3H), 1.05-0.95 (m, 21H); ¹³C NMR (100 MHz, a CDCl₃) δ 173.3, 167.1, 162.9, 157.3, 137.4, 136.0, 128.6, 128.5, 127.8, 127.1, 126.9, 126.7, 88.1, 77.3, 65.0, 59.6, 57.8, 52.0, 44.1, 42.9, 37.9, 18.03, 18.02, 11.9; IR (neat) vₘₐₓ: 3290, 3030, 2943, 2865, 1694, 1629, 1571, 1118 cm⁻¹; HRMS(ESI+): Calcd for a C₃₄H₄₉N₆O₂Si⁺ [M+H]⁺ 601.3681, found 601.3693, Δppm +2.00. (Fig. 45).

The diastereomeric ratio was determined by 1H HMR analysis after silica-gel column (Fig. 45).

### Example 10: Preparation of compounds 12f and 12f'

1f (180.0 mg, 0.397 mmol) was dissolved in acetonitrile solution (ACN, 20.0 ml), followed by treating with benzyl bromide (0.071 ml, 0.596 mmol) and stirring at 80 °C. After completion of the reaction as indicated by TLC, the residual solution was removed under reduced pressure, and, sequentially, washed with hexane to remove any excess benzyl bromide. The compound was dissolved in anhydrous THF (40.0ml) under argon atmosphere, followed by adding ethynylmagnesium bromide solution (0.5 M solution in THF, 3.970 ml, 1.985 mmol) dropwise with a syringe over 30 minutes at -78 °C and stirring over 18 hours between -78 °C and 25°C. After 18 hours, the mixture was quenched with addition of ammonium chloride (NH₄Cl) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain compounds 12f (major product) and 12f (minor product).

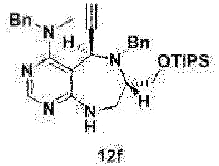

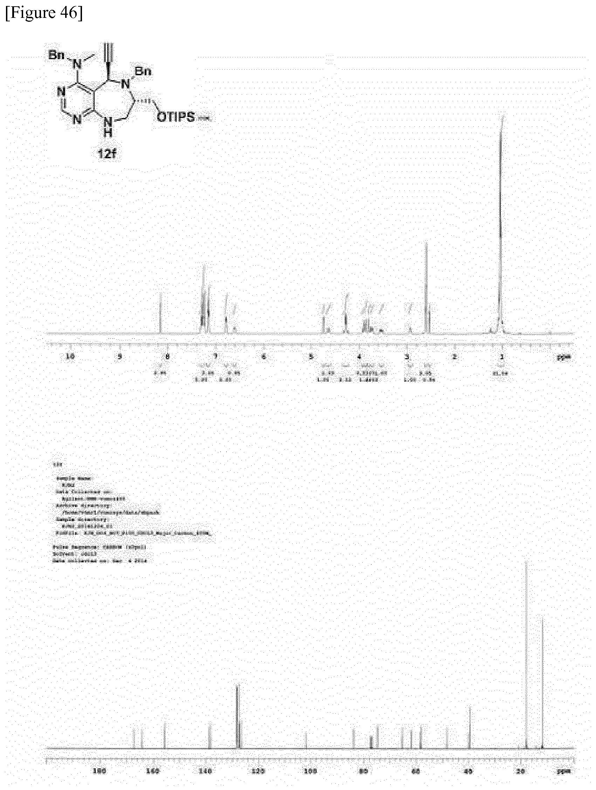

Major product (12f) as a light yellow oil; *R*_{f} = 0.4 (hexane/EtOAc = 3:1); 158.4 mg, 70% yield; ¹H NMR (400 MHz, a CDCl₃) δ 8.16 (s, 1H), 7.33-7.24 (m, 5H), 7.17-7.14 (m, 3H), 6.79-6.78 (m, 2H), 6.27 (m, 1H), 4.74 (d, *J* = 2.3 Hz, 1H), 4.64 (d, *J* = 14.9 Hz, 1H), 4.31, 4.26 (ABq, *J*_{AB} = 15.1 Hz, 2H), 3.92-3.85 (m, 2H), 3.78 (d, *J* = 14.5 Hz, 1H), 3.73 (dd, *J* = 10.0, 3.3 Hz, 1H), 3.54 (ddd, *J* = 14.4, 7.6, 1.4 Hz 1H), 2.93 (m, 1H), 2.60 (s, 3H), 2.53 (d, *J* = 2.3 Hz, 1H), 1.10-1.00 (m, 21H); ¹³C NMR (100 MHz, a CDCl₃) δ 167.4, 164.2, 155.8, 138.9, 138.3, 128.5, 128.4, 128.3, 127.6, 127.4, 126.7, 102.0, 83.9, 74.9, 65.3, 62.0, 58.5, 58.2, 48.5, 40.2, 39.6, 18.1, 12.0; IR (neat) vₘₐₓ: 3241, 2943, 2865, 1573, 1404, 1101, 669 cm⁻¹; HRMS(ESI+) Calcd for a C₃₄H₄₈N₅OSi⁺ [M+H]⁺ 570.3623, found 570.3614, Δppm - 1.58. (Fig. 46).

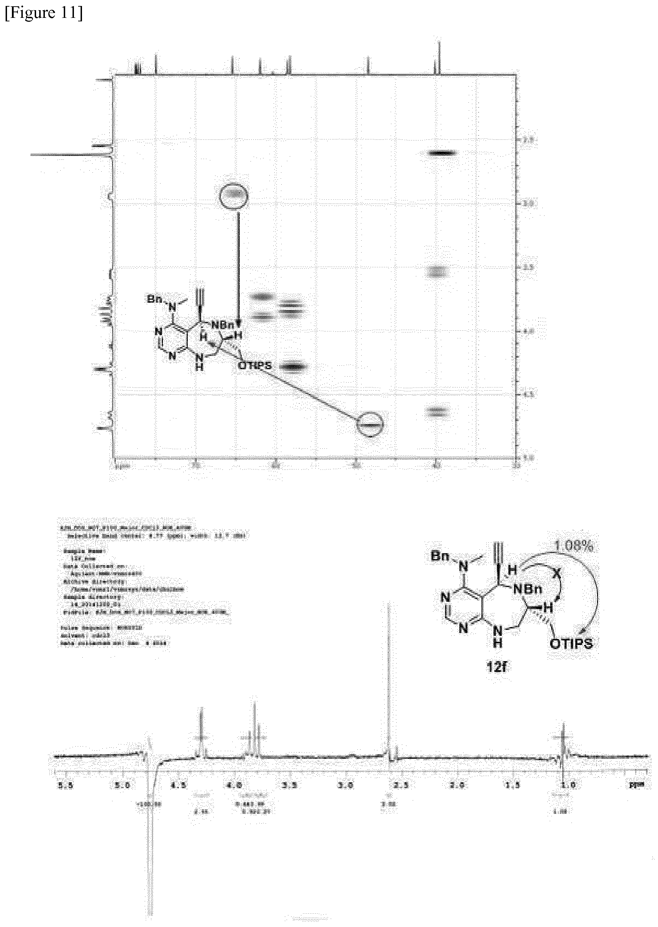

Through Nuclear Overhauser Effect (NOE) spectroscopy, 1D-NOE was analyzed to confirm a chiral center of 12f (Fig. 11).

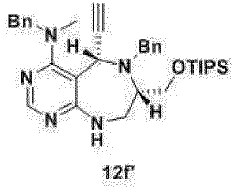

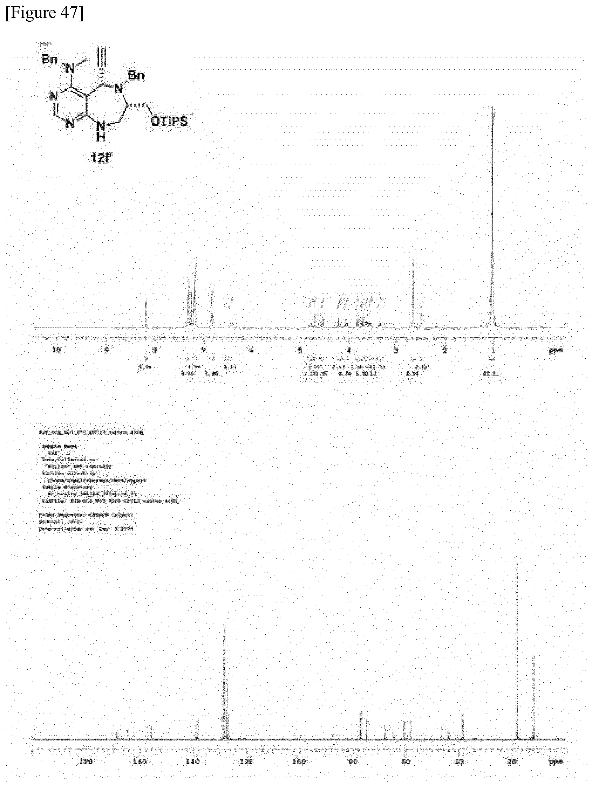

Minor product(12f') as a light yellow oil; ; *R*_{f} = 0.25 (hexane/EtOAc = 3:1); 18.78 mg, 8.3% yield; ¹H NMR (400 MHz, a CDCl₃) δ 8.21 (s, 1H), 7.33-7.31 (m, 3H), 7.21-7.17 (m, 5H), 6.84 (m, 2H), 6.34 (m, 1H), 4.79 (m, 1H), 4.71 (d, *J* = 2.3 Hz, 1H), 4.54, 4.20 (ABq, *J*_{AB} = 15.5 Hz, 2H), 4.07 (t, *J* = 10.2 Hz, 1 H), 3.84, 3.71 (ABq, *J*_{AB} = 13.7 Hz, 2H), 3.64 (dd, *J* = 9.8, 5.1 Hz, 1H), 3.57 (ddd, *J* = 15.0, 7.7, 4.7 Hz 1H), 3.37 (m, 1H), 2.68 (s, 3H), 2.50 (d, *J* = 2.7 Hz, 1H), 1.04 (br s, 21H); ¹³C NMR (100 MHz, a CDCl₃) δ 168.7, 164.4, 156.1, 139.0, 138.2, 129.0, 128.5, 128.4, 127.6, 127.3, 126.8, 99.9, 87.5, 74.8, 68.3, 64.7, 60.7, 58.5, 46.7, 44.0, 38.9, 18.1, 12.0; IR (neat) vₘₐₓ: 3239, 2940, 2865, 1571, 1404, 1102, 698 cm⁻¹; HRMS(ESI+): Calcd for a C₃₄H₄₈N₅OSi⁺ [M+H]⁺ 570.3623, found 570.3625, Δppm +0.35. (Fig. 47).

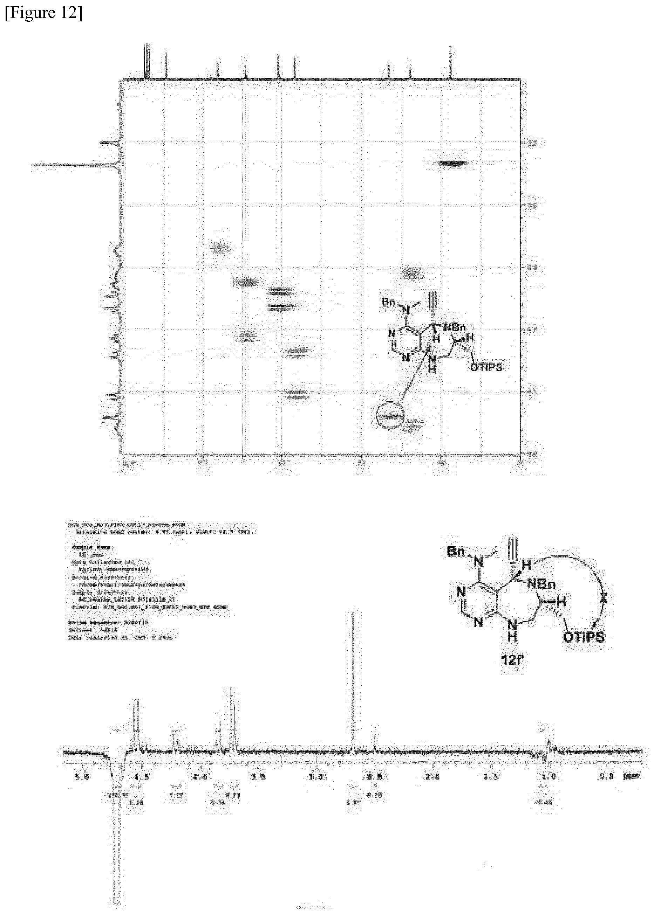

Through Nuclear Overhauser Effect (NOE) spectroscopy, 1D-NOE was analyzed to confirm a chiral center of 12f (Fig. 12).

### Example 11: Preparation of compounds 13f and 13f'

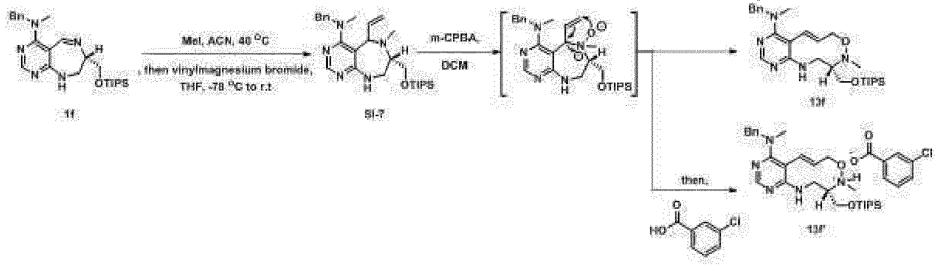

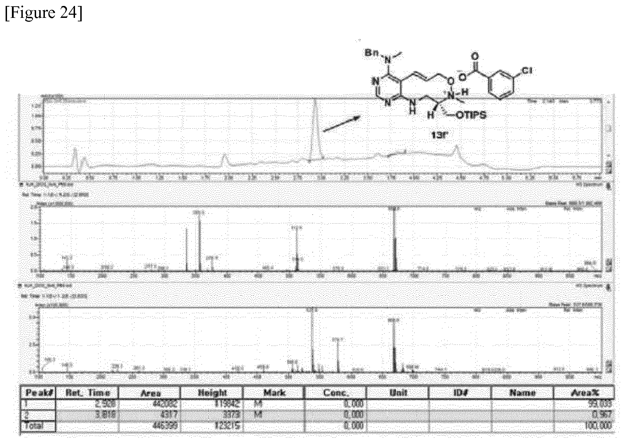

1) Preparation of SI-7: 1f (90 mg, 0.198 mmol) was dissolved in acetonitrile (ACN, 10.0 ml) solution, followed by treating with iodomethane (0.018 ml, 0.297 mmol) and stirring at 40 °C. After completion of the reaction as indicated by TLC, any excess iodomethane was removed under reduced pressure. The compound was dissolved in anhydrous THF (20.0ml) under argon atmosphere, followed by adding benzylmagnesium bromide solution (2.0 m solution in THF, 0.99 ml, 1.98 mmol) dropwise with a syring over 30 minutes at - 78 °C and stirring over 18 hours between -78 °C and 25°C. After 18 hours, the mixture was quenched with addition of ammonium chloride (NH₄Cl) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain compound SI-7 (70.68 mg, 72% yield, d.r. 9:1 (diastereomeric ratio)) as a paleyellow oil.
2) Preparation of 13f: SI-7 (70.68 mg, 0.143 mmol) was dissolved in DCM (14.3 ml), treated with 3-chloroperbenzoic acid (m-CPBA, 32.08 mg, 0.186 mmol) and left at reflux. After 1 hour, the residual solution was removed under reduced pressure and the product was then purified with silica-gel flash column chromatography to obtain compound 13f (42.45 mg, 58% yield, 42% overall yield) as a pale transparent oil.
3) Preparation of 13f: SI-7 (70.68 mg, 0.143 mmol) was dissolved in DCM (14.3 ml), treated with 3-chloroperbenzoic acid (*m*-CPBA, 32.08 mg, 0.186 mmol) and left at reflux. After 1 hour, the above was treated with 3-chlorobenzoic acid (22.39mg, 0.143 mmol) and left to stir at r.t. After completion of the reaction as indicated by TLC, the residual solution was removed under reduced pressure and the product was purified with silica-gel flash column chromatography to obtain compound **13f** (70.72mg, 74% yield, 53% overall yield) as a pale yellow oil. Eiastereomeric ratio (E:Z=99:1) was confirmed by LS-MS analysis of crude mixture (Fig. 24)

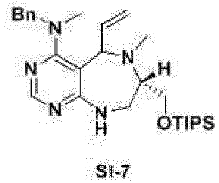

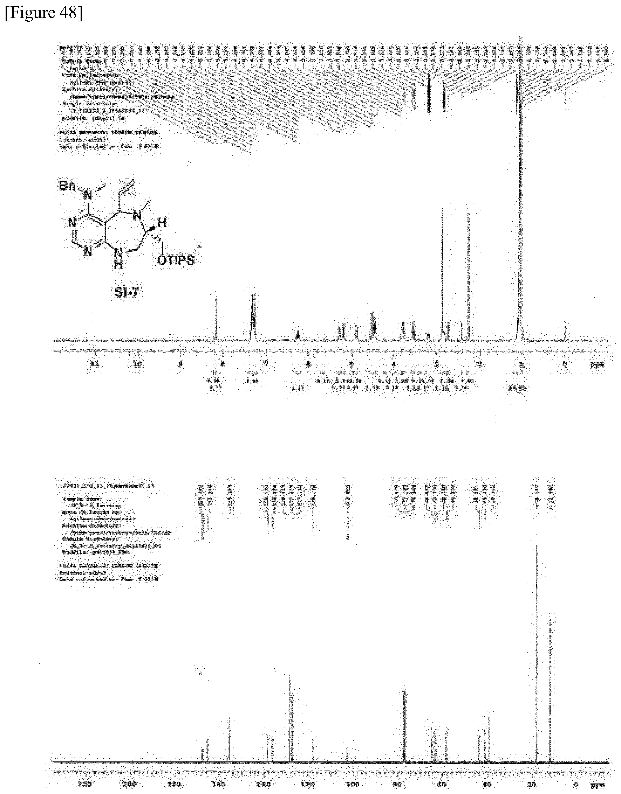

*R*_{f} = 0.27 (DCM/MeOH = 20:1); ¹H NMR (400 MHz, a CDCl₃) δ 8.22 (s, 0.08 H, *minor diastereomer*), 8.17 (s, 0.71H, *major diastereomer*)*,* 7.36-7.24 (m, 6H, *major diastereomer*+*minor diastereomer*), 6.25 (m, 1H), 5.64 (br s, 0.12H, *minor diastereomer*)*,* 5.29 (br s, 0.97H, *major diastereomer*), 5.20 (d, *J* = 10.2 Hz, 1H), 4.88 (d, *J* = 17.2 Hz, 1H), 4.56-4.45 (m, 3H), 5.20 (d, *J* = 10.2 Hz, 1H), 3.84-3.77 (m, 2H), 3.55 (t, *J* = 9.4 Hz, 1.11H, *major diastereomer*), 3.43 (t, *J* = 9.8 Hz, 0.15 H, *minor diastereomer*), 3.19 (m, 1H), 2.86-2.81 (m, 4H), 2.74 (s, 0.38H, *minor diastereomer*), 2.42 (s, 0.38H, *minor diastereomer*), *2.27* (s, 3H, *major diastereomer*), 1.10-1.02 (m, 25H, *major diastereomer*+*minor diastereomer*); ¹³C NMR (100 MHz, a CDCl₃) δ 167.6, 165.5, 155.4, 138.7, 136.5, 128.6, 127.3, 127.1, 118.2, 102.9, 64.9, 63.6, 62.8, 58.3, 44.2, 41.4, 39.4, 18.1, 12.0; HRMS(ESI+): Calcd for a C₂₈H₄₆N₅OSi^{÷} [M+H]⁺ 496.3466, found 496.3465, Δppm -0.20. (Fig. 48)

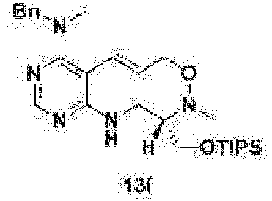

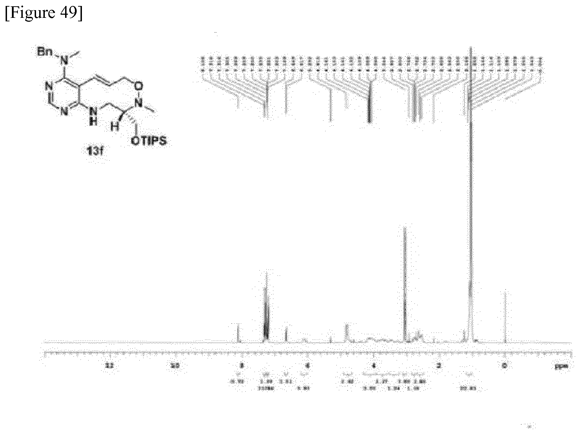

*R_{f}* = 0.3 (hexane/EtOAc = 3:1); ¹H NMR (500 MHz, a CDCl₃) δ 8.11 (s, 1H), 7.32 (m, 2H), 7.24 (m, 1H), 7.20 (m, 2H), 6.63 (d, *J=* 16.1 Hz, 1H), 6.09 (m, 1H), 4.82 (br s, 3H), 4.09 (m, 3H), 3.71 (m, 2H), 3.45-3.27 (m, 1H), 3.04 (s, 3H), 2.77 (m , 1H), 2.63-2.55 (m, 3H), 1.08 (m, 21H);); IR (neat) vₘₐₓ: 3395, 3254, 3060, 3028, 2926, 1946, 1713, 1567, 1493, 1452, 1266, 1105 cm⁻¹; HRMS(ESI+): Calcd for a C₂₈H₄₆N₅O₂Si⁺ [M+H]⁺ 512.3415, found 512.3417, Δ ppm +0.39. (Fig. 49)

The ¹H NMR spectrum of compound 13f was too broad to analyze. As shown below, the structure of 13f was fully confirmed only when it is in a salt form after treatment of 3-chlorobenzoic acid. The NMR peak of 13f was converted to be sharp to readily analyze.

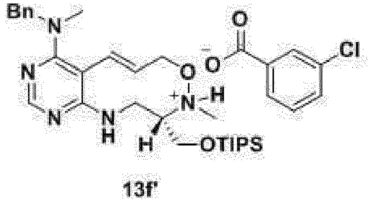

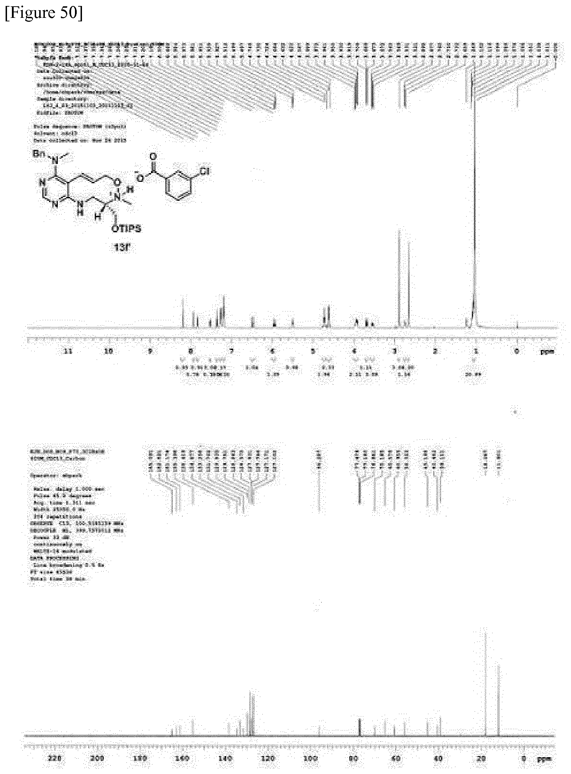

*R_{f}* = 0.3 (hexane/EtOAc 1:1); ¹H NMR (500 MHz, a CDCl₃) δ 8.19 (s, 1H), 7.95 (s, 1H, 3-chlorobenzoic acid-H), 7.85 (d, *J* = 7.8 Hz, 1H, 3-chlorobenzoic acid-H), 7.54 (d, *J* = 8.3 Hz, 1H, 3-chlorobenzoic acid-H), 7.37 (t, *J* = 7.8 Hz, 1H, 3-chlorobenzoic acid-H), 7.29-7.26 (m, 2H), 7.22-7.19 (m, 3H), 6.49 (d, *J* = 16.6 Hz, 1H), 5.96 (dt, *J* = 16.6, 5.9 Hz, 1H), 5.51 (t, *J* = 6.4 Hz, 1H), 4.74 (m, 2H), 4.66, 4.62 (ABq, *J*_{AB} = 15.0 Hz, 2H), 3.96 (m, 2H), 3.70 (dd, *J=* 9.8, 8.3 Hz, 1H), 3.55 (dt, *J* = 14.3, 5.6 Hz, 1H), 2.90 (s, 3H), 2.76 (m, 1H), 2.67 (s, 3H), 1.06 (m, 21H), ¹³C NMR (100 MHz, a CDCl₃) δ 165.1, 162.8, 161.2, 155.4, 138.4, 134.7, 133.3, 131.7, 129.84, 129.75, 128.9, 128.6, 127.8, 127.79, 127.2, 127.1, 96.3, 70.2, 65.6, 61.0, 56.0, 45.2, 40.5, 39.1, 18.1, 11.9; IR (neat) vₘₐₓ: 3396, 3062, 3026, 2945, 2863, 1724, 1572, 1253, 1117 cm⁻¹; HRMS(ESI+): Calcd for a C₃₅H₅₁CN₅O₄Si⁺ [M+H]⁺ 668.3393, found 668.3387, Δppm -0.90. (Fig. 50)

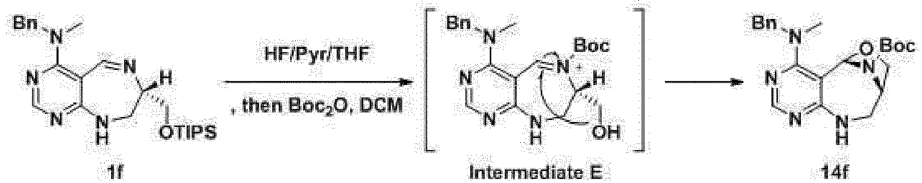

If (90.00 mg, 0.198 mmol) was dissolved in HF/pyridine/THF (5/5/90) solution (2 ml) and stirred at r.t. The reaction was completed as indicated by TLC, treated with ethoxytrimethylsilane (2 ml) and stirred over 1 hour to remove any residual excess HF. After 1 hour, the residual solution was removed under reduced pressure. The compound was dissolved in DCM (4ml), treating with Boc₂O (di-tert-butyl dicarbonate, 56.09 mg, 0.257 mmol), and the mixture was stirred at r.t. After completion of the reaction as indicated by TLC, the residual solution was removed under reduced pressure and the obtained solid was recrystallized with hexane to obtain compound 14f (68.47 mg, 87% overall yield) as a white solid.

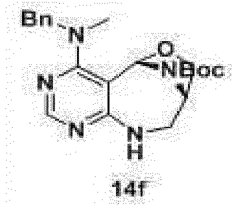

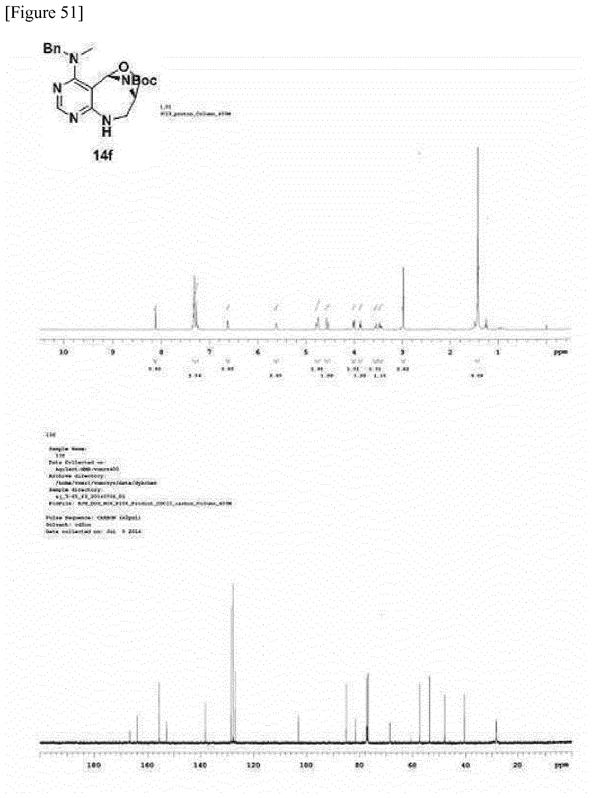

*R*_{f} = 0.3 (EA); ¹H NMR (400 MHz, a CDCl₃) δ 8.13 (s, 1H), 7.35-7.24 (m, 5H), 6.62 (s, 1H), 5.63 (br s, 1H), 4.76 (m, 2H), 4.55 (d, *J* = 15.3 Hz, 1H), 4.01 (d, *J* = 7.0 Hz, 1H), 3.87 (t, *J=* 6.7 Hz, 1 H), 3.55 (br d, *J* = 13.6 Hz, 1H), 3.48-3.43 (m, 1H), 2.98 (s, 3H), 1.43 (s, 9H); ¹³C NMR (100 MHz, a CDCl₃) δ 166.7, 164.0, 155.8, 153.0, 138.3, 128.6, 127.9, 127.2, 103.0, 85.2, 81.6, 68.5, 57.5, 53.7, 48.0, 40.7, 28.5, 28.4, 28.3; IR (neat) vₘₐₓ: 3248, 2975, 2931, 1700, 1571, 1403, 1162, 1048, 699 cm⁻¹; HRMS(ESI+): Calcd for a C₂₁H₂₈N₅O₃⁺ [M+H]⁺ 398.2187, found 398.2174, Δppm -3.26; mp: 50-52 °C. (Fig. 51)

### Example 13: Preparation of compound 15f

The compound was prepared based on Example 12, provided that benzyl isocyanate was used in place of Boc₂O.

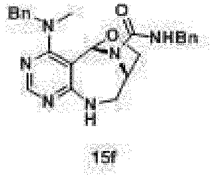

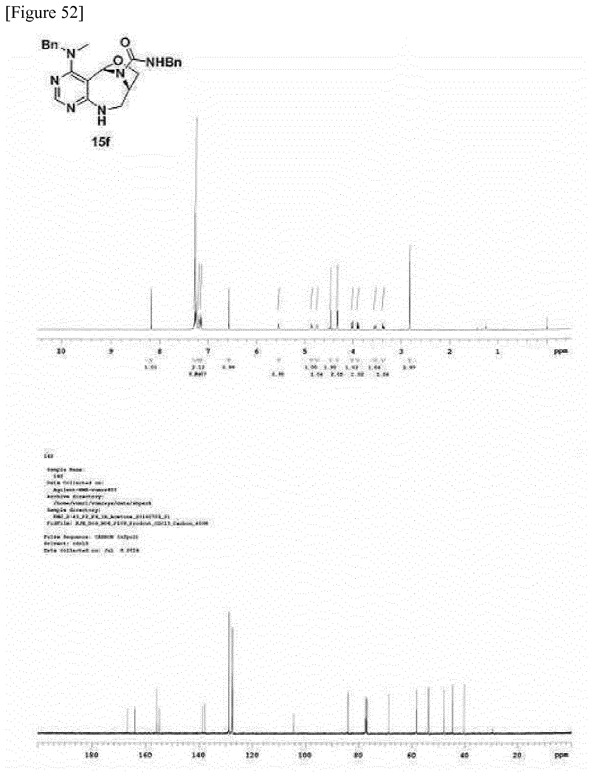

A white solid; *Rf=* 0.4 (EA); 77.57 mg, 91% yield; ¹H NMR (500 MHz, a CDCl₃) δ 8.18 (s, 1H), 7.29-7.28 (m, 2H), 7.25-7.24 (m, 4H), 7.19-7.18 (m, 2H), 7.14 (d, *J=* 6.4 Hz, 2H), 6.57 (s, 1H), 5.55 (br s, 1H), 4.86 (t, *J* = 5.4 Hz, 1 H), 4.74 (m, 1H), 4.46 (br d, *J* = 1.5 Hz, 2H), 4.32 (d, *J* = 5.4 Hz, 2H), 4.02 (dd, *J* = 7.3, 1.0 Hz, 1H), 3.91 (m, 1H), 3.55 (m, 1H), 3.38 (dt, *J=* 13.2, 4.2 Hz, 1H), 2.83 (s, 3H); ¹³C NMR (100 MHz, a CDCl₃) δ 166.8, 164.1, 156.0, 154.9, 138.8, 137.8, 128.7, 128.6, 127.7, 127.5, 127.4, 127.3, 104.3, 84.0, 68.7, 58.2, 53.7, 48.0, 44.7, 40.3; IR (neat) vₘₐₓ: 3258, 2971, 1633, 1542, 1405, 1049, 698 cm⁻¹; HRMS(ESI+): Calcd for a C₂₄H₂₇N₆O₂⁺ [M+H]⁺ 431.2190, found 431.2184, Δppm -1.39; mp: 67-69 °C. (Fig. 52)

### Example 14: Preparation of compound 16f

The compound was prepared based on Example 12, provided that 3-nitrobenzene sulfonyl chloride (m-NsCI) was used in place of Boc₂O. The product was purified with silica-gel flash column chromatography to obtain compound 16f.

The structure 16f was confirmed by X-ray crystallographic analysis (Figs. 3 and 64, a CCDC number 1500586).

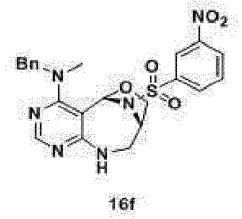

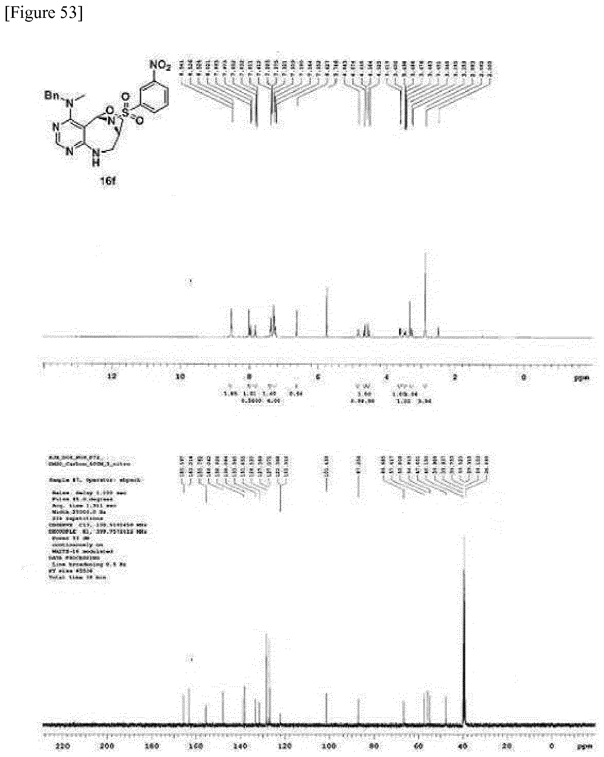

A white solid; *R_{f}* = 0.3 (EA); 59.2 mg, 62% yield; ¹H NMR (400 MHz, DMSO-*d₆*) δ 8.54-8.53 (m, 2H), 8.02 (s, 1H), 7.98 (d, *J* = 7.8 Hz, 1H), 7.83 (t, *J* = 8.2 Hz, 1H), 7.39 (m, 2H), 7.26 (m, 4H), 6.63 (s, 1H), 4.84 (br s, 1H), 4.65, 4.54 (ABq, *J*_{AB} = 15.6 Hz, 2H), 3.61 (d, *J* = 7.4 Hz, 1H), 3.51-3.45 (m, 1H), 3.28 (d, *J* = 14.1 Hz, 1H), 2.88 (m, 4H); ¹³C NMR (400 MHz, DMSO-*d₆*) δ 165.6, 163.2, 155.8, 148.0, 138.9, 138.1, 133.3, 131.7, 128.5, 127.4, 127.1, 122.4, 122.3, 101.4, 87.3, 66.7, 57.4, 55.8, 54.9, 47.8, 40.0; IR (neat) vₘₐₓ: 2920, 1573, 1535, 1354, 1176, 734 cm⁻¹; HRMS(ESI+): Calcd for a C₂₂H₂₃N₆O₅S⁺ [M+H]⁺ 483.1445, found 483.1450, Δppm +1.03; mp: 101-103 °C. (Fig. 53)

### Example 15: Preparation of compounds 17f and 17f'

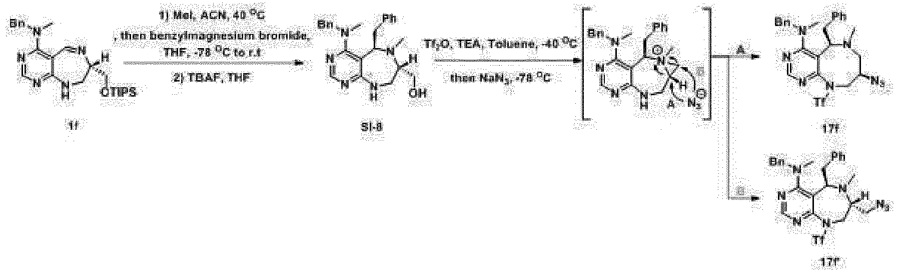

### 1) Preparation of compound SI-8

If (180 mg, 0.397 mmol) was dissolved in acetonitrile solution (ACN, 20.0 ml), treating with iodomethane (0.036 ml, 0.594 mmol) and stirring at 40 °C. After completion of the reaction as indicated by TLC, any excess iodomethane was removed under reduced pressure. The compound was dissolved in anhydrous THF (40.0ml) under argon atmosphere, followed by adding benzylmagnesium bromide solution (2.0 m solution in THF, 0.99 ml, 1.98 mmol) dropwise with a syring over 30 minutes at -78 °C and stirring over 18 hours between -78 °C and 25°C. After 18 hours, the mixture was quenched with addition of ammonium chloride (NH₄Cl) solution. The reaction solution was extracted twice with dichloromethane (DCM) and the extracted organic solution was dried over anhydrous sodium sulfate (Na₂SO₄) and filtered. The filtrate was then condensed under reduced pressure and the product was first purified by a short silica gel flash column chromatography. The compound was dissolved in THF solution (4 ml), treating with TBAF solution (Tetrabutylammonium fluoride, 1.0 M solution in THF, 0.516 ml, 0.516 mmol) and stirring at r.t. After completion of the reaction as indicated by TLC, the residual solution was removed under reduced pressure and the product was purified with silica-gel flash column chromatography to obtain compound SI-8 (121.8 mg, 76% yield) as white powder.

### 2) Preparation of 17f from SI-8

SI-8 (121.8 mg, 0.302 mmol) was dissolved, under argon atmosphere, in anhydrous toluene (3.0ml), sequentially adding triethylamine (Et3N, 0.126 ml, 0.906 mmol) and trifluoromethanesulfonic anhydride (Tf₂O) (0.112 ml, 0.664 mmol) at -40 °C and stirring at - 40 °C over 1 hour. After 1 hour, NaN₃ (58.9 mg, 0.906 mmol) was added at -78 °C and stirred between -78 °C and 25°C over 18 hours. After 18 hours, the mixture was quenched with addition of sodium bicarbonate (NaHCO3) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain, respectively, 17f (path A, 8-membered ring)) and 17f (path B,7-membered ring)).

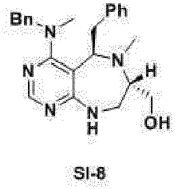

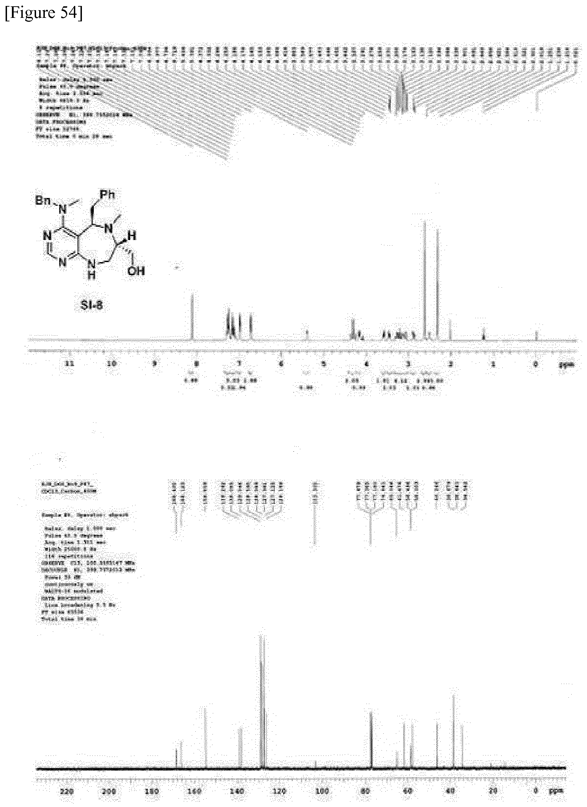

*R_{f}* = 0.4 (DCM/MeOH = 10:1); ¹H NMR (400 MHz, a CDCl₃) δ 8.13 (s, 1H), 7.32-7.25 (m, 3H), 7.20-7.13 (m, 3H), 6.99 (d, *J* = 7.2 Hz, 2H), 6.73 (d, *J* = 6.7 Hz, 2H), 5.40 (d, *J* = 5.9 Hz, 1H), 4.35, 4.28 (ABq, *J*_{AB} = 16.0, 15.6 Hz, 2H), 4.19 (dd, *J=* 9.6, 4.5 Hz, 1H), 3.60 (dd, *J* = 10.6, 5.1 Hz, 1H), 3.46 (dd, *J* = 10.8, 5.7 Hz, 1H), 3.31-3.04 (m, 4H), 2.88 (dd, *J* = 12.9, 4.3 Hz, 1H), 2.62 (s, 3H), 2.51 (br s, 1H), 2.32 (s, 3H); ¹³C NMR (100 MHz, a CDCl₃) δ 168.4, 166.1, 154.9, 139.3, 138.1, 129.0, 128.6, 128.1, 127.4, 127.1, 126.2, 103.3, 65.0, 61.7, 58.5, 58.0, 46.2, 38.7, 38.6, 34.5; HRMS (ESI+): Calcd for a C₂₄H₃₀N₅O⁺ [M+H]⁺ 404.2445, found 404.2446, Δppm +0.25; mp: 70-72 °C. (Fig. 54).

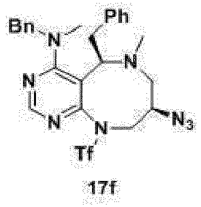

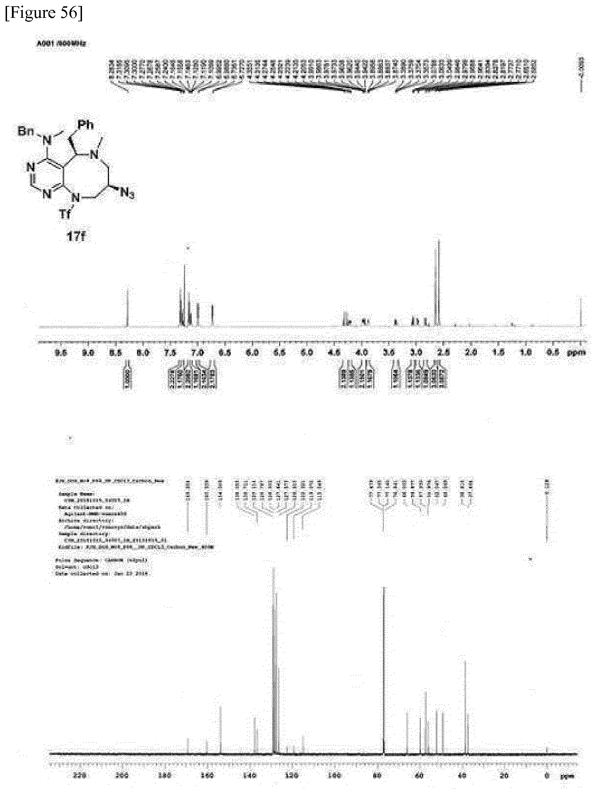

(Path A, 8-membered ring). A white solid; *Rf=* 0.6 (hexane/EtOAc = 3:1); 37.2 mg, 22% yield, 17% overall yield; ¹H NMR (800 MHz, a CDCl₃) δ 8.29 (s, 1H), 7.32 (m, 2H), 7.28 (m, 1H), 7.17 (m, 2H), 7.13 (m, 1H), 7.00 (d, *J* = 7.3 Hz, 2H), 6.74 (d, *J* = 7.3 Hz, 2H), 4.33, 4.27 (ABq, *J*_{AB} = 15.7 Hz, 2H), 4.23 (dd, *J* = 14.9, 6.6 Hz, 1H), 3.99 (dd, *J* = 10.3, 3.9 Hz, 1H), 3.96 (dd, *J* = 15.7, 1.5 Hz, 1H), 3.89 (m, 1H), 3.38 (dd, *J* = 14.9, 10.5 Hz, 1H), 3.07 (m, 1H), 2.98 (dd, *J* = 12.5, 3.7 Hz, 1H), 2.84 (br dd, *J* = 16.0, 1.6 Hz, 1H), 2.66 (s, 3H), 2.59 (s, 3H); ¹³C NMR (100 MHz, a CDCl₃) δ 169.2, 160.3, 154.0, 138.1, 136.7, 129.1, 128.8, 128.3, 127.64, 127.57, 126.5, 120.7 (q, ¹*J*_{C,F} = 324.4 Hz), 115.0, 66.0, 59.9, 57.4, 56.0, 52.1, 49.1, 38.8, 37.5; IR (neat) vₘₐₓ: 3028, 2929, 2868, 2108, 1566, 1393 cm⁻¹; HRMS(ESI+) Calcd for a C₂₅H₂₈F₃N₈O₂S⁺ [M+H]⁺ 561.2003, found 561.2009, Δppm +1.07; mp: 103-105 °C. (Fig. 56).

Through Nuclear Overhauser Effect (NOE) spectroscopy, 1D-NOE was analyzed to confirm a chiral center of 17f (Fig. 13).

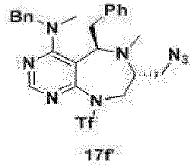

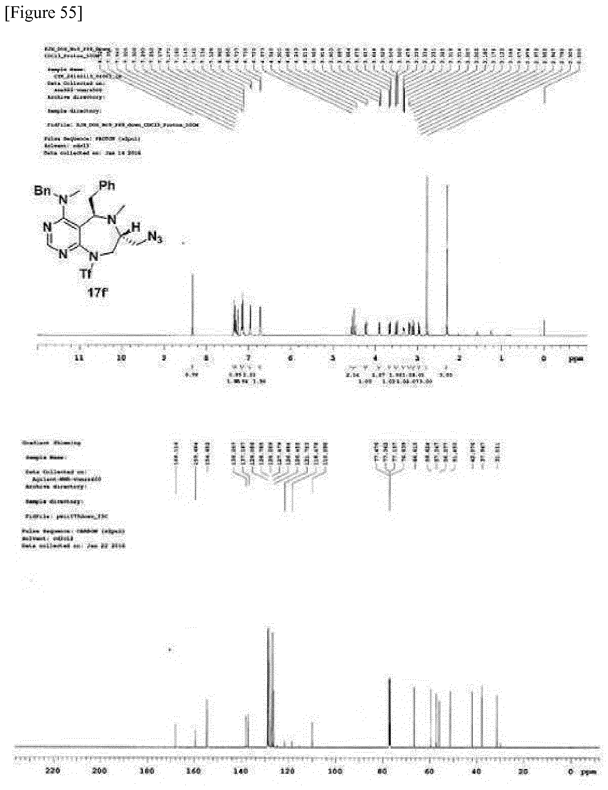

(Path B, 7-membered ring). A white solid; *R*_{f} = 0.5 (hexane/EtOAc = 3:1); 55.9 mg, 33% yield, 25% overall yield; ¹H NMR (500 MHz, a CDCl₃) δ 8.32 (s, 1H), 7.34 (m, 2H), 7.29 (m, 1H), 7.14 (m, 3H), 6.96 (d, *J* = 7.3 Hz, 2H), 6.73 (br d, *J* = 7.3 Hz, 2H), 4.56, 4.49 (ABq, *J*_{AB} = 16.5 Hz, 2H), 4.23 (d, *J* = 15.2 Hz, 1 H), 3.91 (dd, *J* = 11.2, 2.9 Hz, 1H), 3.67 (dd, *J* = 13.7, 4.4 Hz, 1H), 3.50 (dd, *J* = 14.9, 10.5 Hz, 1H), 3.32 (m, 1H), 3.19 (dd, *J* = 13.7, 2.4 Hz, 1H), 3.10 (t, *J* = 11.8 Hz, 1H), 2.96 (dd, *J* = 13.0, 3.2 Hz, 1H), 2.78 (s, 3H), 2.31 (s, 3H); ¹³C NMR (100 MHz, a CDCl₃) δ 168.1, 159.7, 154.7, 138.3, 137.2, 129.1, 128.8, 128.3, 127.5, 127.0, 126.5, 120.1 (q, ¹*J*_{C,F} = 322.6 Hz), 110.1, 66.6, 59.6, 57.4, 56.3, 51.5, 42.1, 38.0, 31.5; IR (neat) vmax: 3030, 2930, 2869, 2105, 1578, 1529, 1391, 1212, 1043 cm⁻¹; HRMS(ESI+) Calcd for a C₂₅H₂₈F₃N₈O₂S⁺ [M+H]⁺ 561.2003, found 561.2011, Δppm +1.43; mp: 140-142 °C. (Fig. 55).

Through Nuclear Overhauser Effect (NOE) spectroscopy, 1D-NOE was analyzed to confirm a chiral center of 17f (Fig. 14).

### Example 16: Preparation of compound 18f

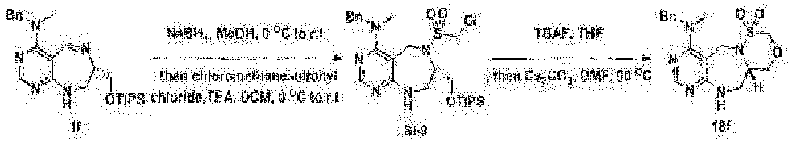

### 1) Preparation of compound SI-9

A solution of If (90mg, 0.198 mmol) in methanol (MeOH, 2ml) was treated with sodium borohydride (NaBH4, 36.88 mg, 0.975 mmol) at 0 °C and stirred between 0 °C and 25°C. After completion of the reaction as indicated by TLC, the mixture was quenched with addition of sodium bicarbonate (NaHCO3) solution. The reaction solution was extracted twice with dichloromethane (DCM) and the extracted organic solution was dried over anhydrous sodium sulfate (Na2SO4) and filtered. The filtrate was then evaporated under reduced pressure, condensed, and dried. The compound was dissolved in dichloromethane (DCM, 2 ml), followed by adding, at 0 °C, triethylamine (TEA, 0.055 ml, 0.396 mmol) and then chloromethane sulfonyl chloride (0.023 ml, 0.257 mmol), and stirring between 0 °C and 25 °C. After completion of the reaction as indicated by TLC, the mixture was quenched with addition of ammonium chloride (NH₄Cl) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain compound SI-9 (97.89 mg, 87% yield).

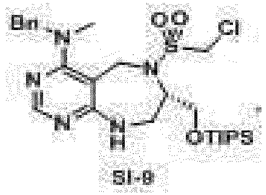

### 2) Preparation of compound 18f

A solution of SI-9 (97.89 mg, 0.172 mmol) in THF was treated with TBAF solution (Tetrabutylammonium fluoride, 1.0 M solution in THF, 0.224 ml, 0.224 mmol) and stirred at r.t. After completion of the reaction as indicated by TLC, the residual solution was removed under reduced pressure. The compound was disslved in DMF (2ml), treating with Cs₂CO₃ (168.1 mg, 0.516 mmol) and stirring at 90 °C. After completion of the reaction as indicated by TLC, the reaction solution was extracted twive with ethyl acetate (EA) and the extracted organic solution was dried over anhydrous sodium sulfate (Na2SO4) and filtered. The filtrate was then condensed under reduced pressure and the product was purified with silica-gel flash column chromatography to compound 18f (51.02 mg, 79% yield, 69% overall yield) as white powder.

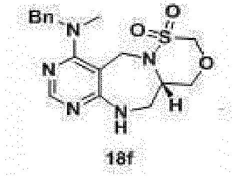

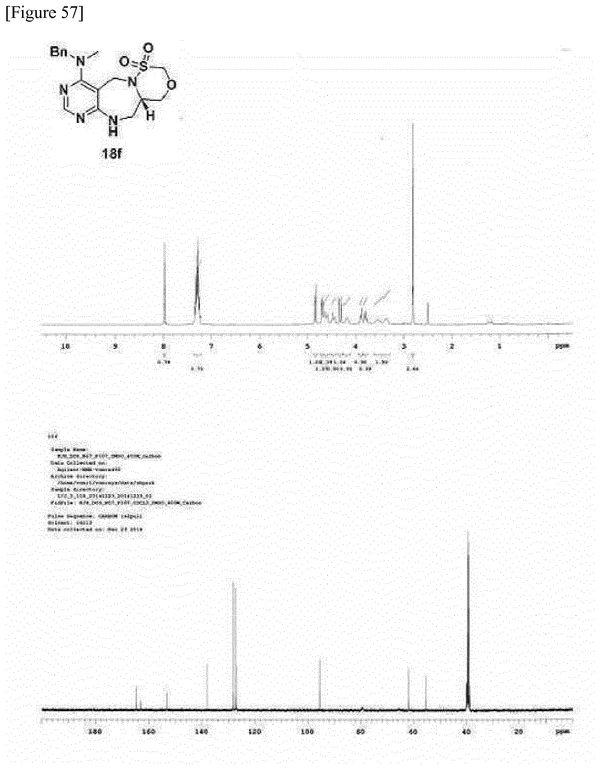

*R*_{f} = 0.4 (DCM/MeOH = 20:1); ¹H NMR (400 MHz, DMSO-*d₆*) δ 7.98 (s, 1H), 7.34-7.23 (m, 6H), 4.84 (d, *J=* 11.7 Hz, 1H), 4.71-4.59 (m, 3H), 4.47 (d, *J=* 15.3 Hz, 1H), 4.33 (d, *J* = 15.7 Hz, 1H), 4.19 (br s, 1H), 3.89 (d, *J* = 10.4 Hz, 1H), 3.80 (t, *J* = 11.2 Hz, 1H), 3.54 (br s, 1H), 3.38 (br s, 1H), 2.81 (s, 3H); ¹³C NMR (100 MHz, DMSO-*d₆*) δ 165.0, 163.2, 153.5, 138.1, 128.4, 127.5, 127.0, 95.6, 62.0, 55.4, 40.2, 39.4; IR (neat) vₘₐₓ: 3230, 2926, 1574, 1409, 1155, 736 cm⁻¹; HRMS(ESI+): Calcd for a C₁₇H₂₂N₅O₃S⁺ [M+H]⁺ 376.1438, found 376.1437, Δppm -0.27; mp: 196-198 °C. (Fig. 57).

### Example 17: Preparation of compound 19f

Compound 19f was prepared based on Example 18, provided that chloroacetic anhydride was used in place of chloromethane sulfonyl chloride.

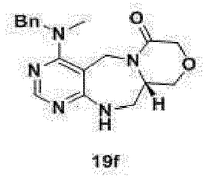

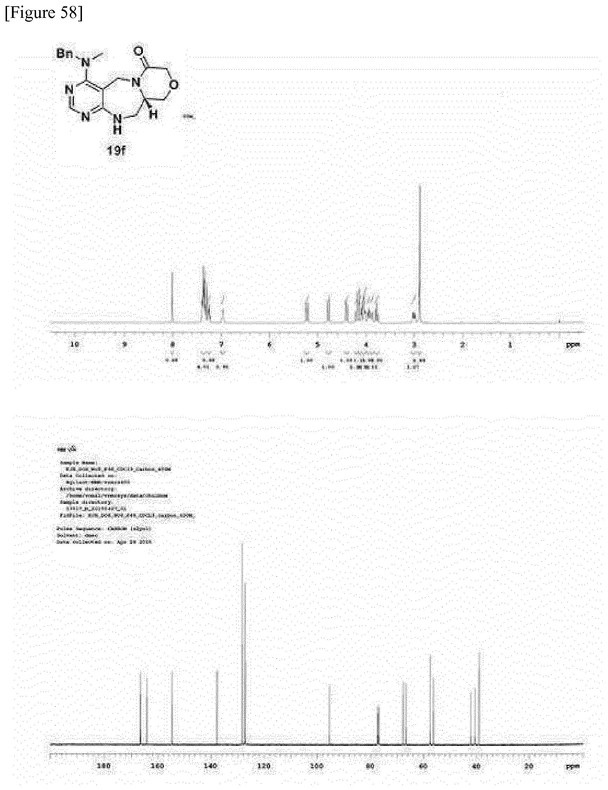

A transparent oil; *R*_{f} = 0.5 (DCM/MeOH = 20:1); 50.40 mg, 75% overall yield; ¹H NMR (400 MHz, a CDCl₃) δ 8.02 (s, 1H), 7.39-7.33 (m, 4H), 7.27-7.23 (m, 1H), 6.97 (m, 1H), 5.23 (d, *J* = 15.7 Hz, 1H), 4.78, 4.41 (ABq, *J*_{AB} = 15.7 Hz, 2H), 4.21, 4.12 (ABq, *J*_{AB} = 16.4 Hz, 2H), 4.09-4.01 (m, 2H), 3.97-3.87 (m, 2H), 3.78 (t, *J* = 11.0 Hz, 1H), 3.00 (dd, *J* = 15.3, 7.4 Hz, 1H), 2.89 (s, 3H); ¹³C NMR (100 MHz, a CDCl₃) δ 166.7, 166.5, 164.3, 154.7, 137.8, 128.5, 127.5, 127.1, 95.4, 67.7, 66.5, 57.5, 56.2, 42.2, 40.7, 39.1; IR (neat) vₘₐₓ: 3237, 2908, 1653, 1573, 1408, 1119, 737 cm⁻¹; HRMS(ESI+): Calcd for a C₁₈H₂₂N₅O₂⁺ [M+H]⁺ 340.1768, found 340.1765, Δppm -0.88. (Fig. 58).

### Example 18: Preparation of compound 20f

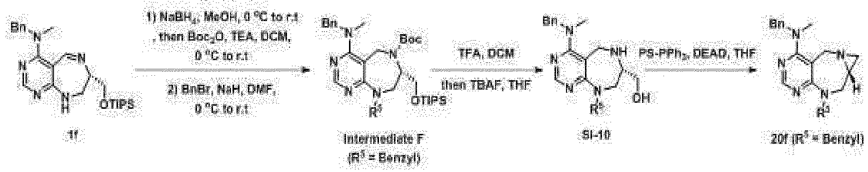

### 1) Preparation of intermediate F

A solution of If (135.0 mg, 0.298 mmol) in methanol (MeOH) was treated with sodium borohydride (NaBH4, 56.37 mg, 1.490 mmol) at 0 °C and stirred between 0 °C and 25°C. After completion of the reaction as indicated by TLC, the mixture was quenched with addition of sodium bicarbonate (NaHCO3) solution. The reaction solution was extracted twice with dichloromethane (DCM) and the extracted organic solution was dried over anhydrous sodium sulfate (Na2SO4) and filtered. The filtrate was then evaporated under reduced pressure, condensed, and dried. The compound was dissolved in dichloromethane (DCM, 3 ml), followed by sequentially adding, at 0 °C, triethylamine (TEA, 0.083 ml, 0.596 mmol) and Boc₂O (Di-tert-butyl dicarbonate,84.55 mg, 0.387 mmol), and stirring between 0 °C and 25 °C. After completion of the reaction as indicated by TLC, the mixture was quenched with addition of ammonium chloride (NH₄Cl) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain Boc-protected compound (162.3 mg, 98% yield). Boc-protected compound (162.3 mg, 0.292 mmol) was dissolved, under argon atmosphere, in anhydrous DMF solution (3.0 ml), treated with sodium hydride (NaH, 60% dispersion in mineral oil, 23.36 mg, 0.584 mmol) at 0 °C, and stirred at 0 °C over 30 minutes. After 30 minutes, the mixture was slowly treated with benzyl bromide (0.052 ml, 0.438 mmol) at 0 °C and stirred between 0 °C and 25°C. After completion of the reaction as indicated by TLC, the mixture was quenched with addition of ammonium chloride (NH₄Cl) solution. The reaction solution was extracted twice with ethyl acetate (EA) and the extracted organic solution was dried over anhydrous sodium sulfate (Na2SO4) and filtered. The filtrate was then condensed under reduced pressure and the product was purified with silica-gel flash column chromatography to obtain intermediate F (R⁵ = benzyl, 164.1 mg, 87% yield).

### 2) Preparation of compound SI-10

Intermediate F (R⁵ = benzyl,164.1 mg, 0.254 mmol) was dissolved in 10% trifluoroacetic acid (TFA) DCM (12.5 ml) solution and then stirred at r.t. After completion of the reaction as indicated by TLC, any excess TFA was removed by azeotropic evaporation with toluene under reduced pressure. The compound was dissolved in THF (2.5 ml), treating with Tetra-n-butylammonium fluoride solution (TBAF, 1.0 m solution in THF, 0.330 ml, 0.330 mmol) and stirred at r.t. After completion of the reaction as indicated by TLC, the mixture was quenched with addition of sodium bicarbonate (NaHCO3) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain compound SI-10 (73.21 mg, 74% yield).

### 3) Preparation of compound 20f

Compound SI-10 (73.21 mg, 0.188 mmol) was dissolved, under argon atmosphere, in anhydrous DMF solution (20.0 ml), treated with solid triphenylphosphine (PPh₃) (polymer-bound triphenylphosphine (587.5 mg, 0.94 mmol)) and stirred over 30 minutes. After 30 minutes, the above was slowly treated with diehtyl azodicarboxylate (DEAD, 0.058 ml, 0.376 mmol) and stirred over 18 hours. The resultant solid after the reaction was removed by using Celite® and the filtrate was condensed under reduced pressure. The product was purified with silica-gel flash column chromatography to obtain compound 20f (R⁵ = benzyl) (56.57 mg, 81% yield, 52% overall yield) compound as a pale yellow oil.

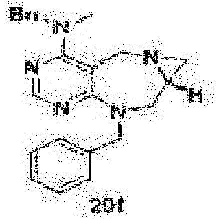

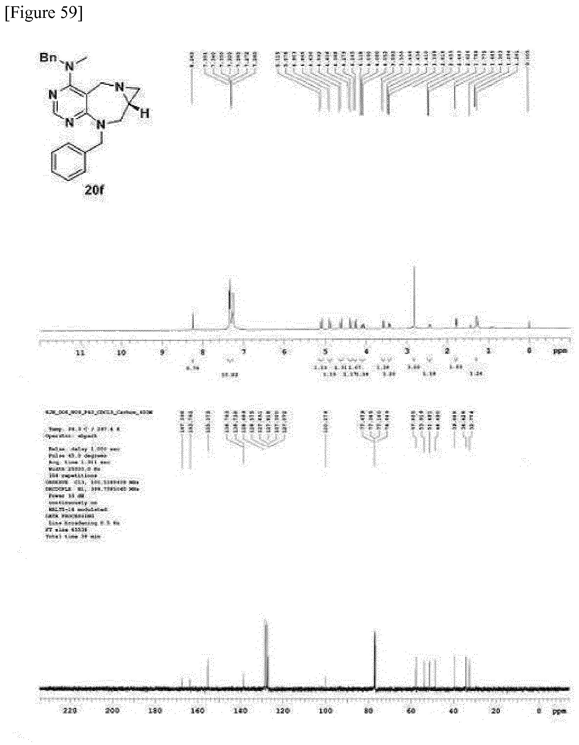

*R*_{f} = 0.4 (DCM/MeOH 20:1); ¹H NMR (400 MHz, a CDCl₃) δ 8.24 (s, 1H), 7.35-7.27 (m, 10H), 5.10, 4.88 (ABq, *J_{AB} =* 15.3 Hz, 2H), 4.61, 4.39 (ABq, *J_{AB}* = 15.3 Hz, 2H), 4.26 (d, *J* = 13.7, 1H), 4.09 (dd, *J* = 15.3, 11.3 Hz, 1H), 3.58 (d, *J* = 13.7, 1H), 3.42 (dd, *J* = 15.5, 4.1 Hz, 1H), 2.82 (s, 3H), 2.43 (m, 1H), 1.78 (d, *J=* 5.1, 1H), 1.30 (d, *J=* 3.1, 1H); ¹³C NMR (100 MHz, a CDCl₃) δ 167.4, 163.8, 155.4, 138.8, 138.7, 128.7, 128.6, 127.8, 127.6, 127.3, 127.1, 100.3, 57.8, 53.9, 51.5, 48.7, 39.7, 34.4, 32.8; IR (neat) vₘₐₓ: 3027, 2920, 2853, 1559, 1356, 735, 700 cm⁻¹; HRMS(ESI+): Calcd for a C₂₃H₂₆N₅⁺ [M+H]⁺ 372.2183, found 372.2189, Δppm +1.61. (Fig. 59).

### Example 19: Preparatio of compound 21f

Compound 21f was prepared based on Example 18, provided that 3,5-dimethylbenzyl bromide was used in place of benzyl bromide.

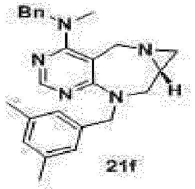

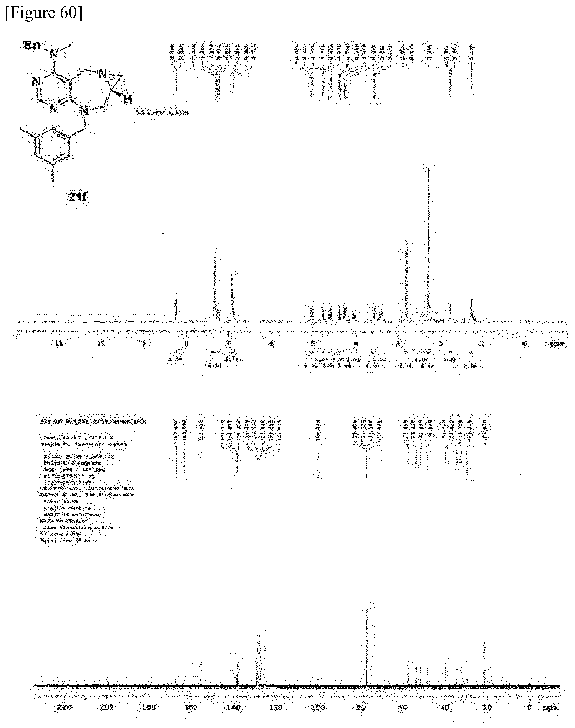

Intermediate F (R⁵ = 3,5-dimehtylbenzyl). A light yellow oil; *R_{f}* = 0.5 (DCM/MeOH = 20:1); 49.82 mg, 81% yield, 53% overall yield; ¹H NMR (500 MHz, a CDCl₃) δ 8.25 (s, 1H), 7.34-7.25 (m, 5H), 6.91 (m, 3H), 5.04, 4.78 (ABq, *J_{AB} =* 15.2 Hz, 2H), 4.61, 4.38 (ABq, *J_{AB}* = 15.2 Hz, 2H), 4.26 (d, *J* = 13.7, 1H), 4.04 (dd, *J* = 14.2, 11.7 Hz, 1H), 3.57 (d, *J* = 13.7, 1H), 3.41 (dd, *J* = 15.4, 2.2 Hz, 1H), 2.81 (s, 3H), 2.43(m, 1H), 2.29 (s, 6H), 1.77 (d, *J* = 2.9, 1H), 1.28 (s, 1H); ¹³C NMR (100 MHz, a CDCl₃) δ 167.4, 163.8, 155.4, 138.8, 138.6, 138.3, 129.0, 128.6, 127.8, 127.1, 125.4, 100.3, 57.9, 53.7, 51.5, 48.4, 39.7, 34.5, 32.7, 21.5; IR (neat) vₘₐₓ: 3026, 2922, 2863, 1564, 1355, 735, 700 cm⁻¹; HRMS(ESI+): Calcd for a C₂₅H₃₀N₅⁺ [M+H]⁺ 400.2496, found 400.2495, Δppm -0.25. (Fig. 60).

### Example 20: Preparation of compound 22f

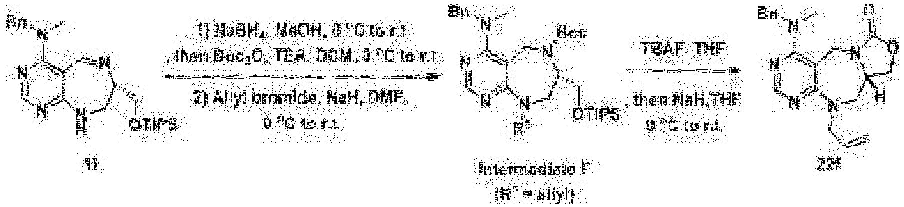

### 1) Preparation of intermediate F (R⁵=allyl)

A solultion of If (135.0 mg, 0.298 mmol) in methanol (MeOH) was treated with sodium borohydride (NaBH₄ (56.37 mg, 1.490 mmol)) at 0 °C and stirred between 0 °C and 25 °C. After completion of the reaction as indicated by TLC, the mixture was quenched with addition of sodium bicarbonate (NaHCO3) solution. The reaction solution was extracted twice with dichloromethane (DCM) and the extracted organic solution was dried over anhydrous sodium sulfate (Na2SO4) and filtered. The filtrate was then evaporated under reduced pressure, condensed, and dried. The compound was dissolved in dichloromethane (DCM 3 ml), followed by sequentially adding, at 0 °C, triethylamine (TEA, 0.083 ml, 0.596 mmol) and Boc₂O (Di-tert-butyl dicarbonate, 84.55 mg, 0.387 mmol), and stirring between 0 °C and 25°C. After completion of the reaction as indicated by TLC, the mixture was quenched with addition of ammonium chloride (NH₄Cl) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain Boc-protected compound (162.3 mg, 98% yield).

The Boc-protected compound (162.3 mg, 0.292 mmol) was dissolved, under argon atmosphere, in anhydrous DMF solution (3.0 ml), treating with sodium hydride (NaH (60% dispersion in mineral oil, 23.36 mg, 0.584 mmol)) at 0 °C, and stirring at 0 °C over 30 minutes. After 30 minutes, the mixture was slowly treated with allyl bromide (0.038 ml, 0.438 mmol) at 0 °C and stirred between 0 °C and 25 °C. After completion of the reaction as indicated by TLC, the mixture was quenched with addition of ammonium chloride (NH₄Cl) solution. The reaction solution was extracted twice with ethyl acetate (EA) and the extracted organic solution was dried over anhydrous sodium sulfate (Na2SO4) and filtered. The filtrate was then condensed under reduced pressure and the product was purified with silica-gel flash column chromatography to obtain intermediate F (R⁵ = allyl, 141.2 mg, 81% yield).

### 2) Preparation of compound 22f

A solution of intermediate F (R5 = allyl, 243.1 mg, 0.408 mmol) was treated with TBAF solution (Tetrabutylammonium fluoride, 1.0 M solution in THF, 0.530 ml, 0.530 mmol) and stirred at r.t. After completion of the reaction as indicated by TLC, the mixture was quenched with addition of sodium bicarbonate (NaHCO3) solution. The reaction solution was extracted twice with dichloromethane (DCM) and the extracted organic solution was dried over anhydrous sodium sulfate (Na2SO4) and filtered. The filtrate was then evaporated under reduced pressure, condensed, and dried. The compound was dissolved, under argon atmosphere, in anhydrous THF (2.5 ml), treating with sodium hydride (NaH (60% dispersion in mineral oil, 14.22 mg, 0.356 mmol)) at 0 °C, and stirring between 0 °C and 25°C. After completion of the reaction as indicated by TLC, the mixture was quenched with addition of ammonium chloride (NH4Cl) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain 22f (56.30 mg, 65% yield, 53% overall yield) as white powder.

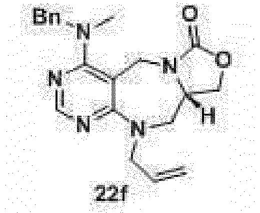

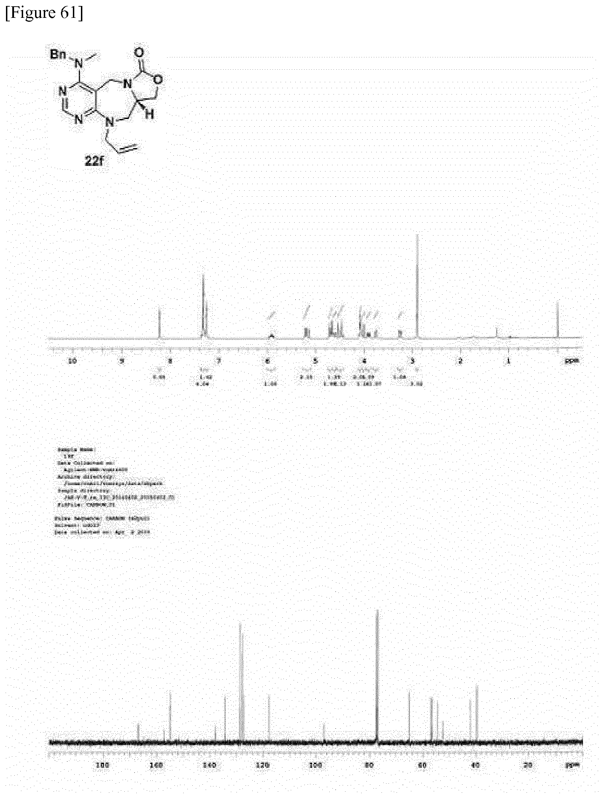

*R*_{f} = 0.2 (EA); ¹H NMR (400 MHz, a CDCl₃) δ 8.23 (s, 1H), 7.36-7.31 (m, 4H), 7.28 (m, 1H), 5.92 (m, 1H), 5.21 (d, *J* = 10.2 Hz, 1H), 5.15 (dd, *J* = 17.2, 1.2 Hz, 1H), 4.72-4.65 (m, 2H), 4.61 (dd, *J* = 15.5, 4.9 Hz, 1H), 4.55-4.44 (m, 2H), 4.11-4.07 (m, 2H), 4.02 (d, *J* = 16.0 Hz, 1H), 3.91 (dd, *J* = 15.5, 6.5 Hz, 1H), 3.76 (dd, *J* = 14.9, 2.0 Hz, 1H), 3.25 (dd, *J* = 15.1, 3.3 Hz, 1H), 2.91 (s, 3H); ¹³C NMR (100 MHz, a CDCl₃) δ 166.9, 164.9, 157.3, 155.0, 138.1, 134.2, 128.7, 127.9, 127.3, 117.8, 97.0, 65.1, 56.9, 56.7, 54.5, 52.4, 42.1, 39.7; IR (neat)vₘₐₓ: 3049, 2976, 2917, 1758, 1561, 1410, 1266, 746 cm⁻¹; HRMS(ESI+): Calcd for a C₂₀H₂₄N₅O₂⁺ [M+H]⁺ 366.1925, found 366.1924, Δppm -0.27; mp: 89-91 °C. (Fig. 61).

### Example 21: Preparation of compound 23f

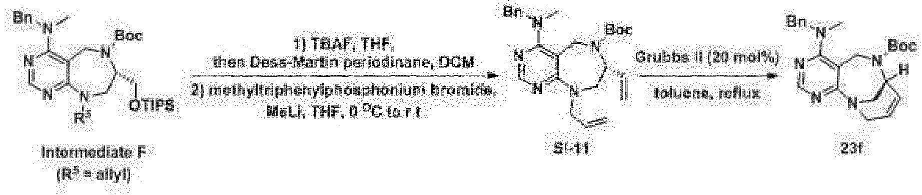

### 1) Preparation of SI-11

A solution of intermediate F (R5 = allyl, 243.1 mg, 0.408 mmol) in THF was treated with TBAF solution (Tetrabutylammonium fluoride, 1.0 M solution in THF, 0.530 ml, 0.530 mmol) and stirred at r.t. After completion of the reaction as indicated by TLC, the mixture was quenched with addition of sodium bicarbonate (NaHCO3) solution. The reaction solution was extracted twice with dichloromethane (DCM) and the extracted organic solution was dried over anhydrous sodium sulfate (Na2SO4) and filtered. The filtrate was then evaporated under reduced pressure, condensed, and dried. The compound was dissolved in dichloromethane (8.2 ml), treating with Dess-Martin periodinane (259.6 mg, 0.612 mmol) and stirred at r.t. After completion of the reaction as indicated by TLC, the mixture was quenched with addition of sodium bicarbonate (NaHCO3) solution. The reaction solution was extracted twice with dichloromethane (DCM) and the extracted organic solution was dried over anhydrous sodium sulfate (Na2SO4) and filtered. The filtrate was then condensed under reduced pressure and the product was purified with silica-gel flash column chromatography to obtain aldehyde compound (148.3 mg, 83% yield).

Next, methyltriphenylphosphonium bromide (363.3 mg, 1.017 mmol) was dissolved, under argon atmosphere, in anhydrous THF, followed by adding, at 0 °C, MeLi (1.6 M in diethyl ether, 0.530 ml, 0.848 mmol) solution, and the mixture was stirred over 30 minutes at 0 °C. After 30 minutes, the above was treated with the resultant aldehyde compound and stireed over 12 hours between 0 °C and 25°C. After 12 hours, to the mixture was added ammonium chloride solution to quench the reaction. The reaction solution was extracted twice with ethyl acetate (EA) and the extracted organic solution was dried over anhydrous sodium sulfate (Na2SO4) and filtered. The filtrate was then condensed under reduced pressure and the product was purified with silica-gel flash column chromatography to obtain compound SI-11 (130.0 mg, 88% yield).

### 2) Preparation of compound 23f

A solution of SI-11 (130.0 mg, 0.298 mmol) in toluene was treated with second generation Grubbs' catalyst (50.94 mg, 0.060 mmol, 20 mol%) and left at reflux. After 4 hours, the resultant was condensed under reduced pressure and the product was purified with silica-gel flash column chromatography to obtain compound 23f (44.83 mg, 52% recovery yield, 31% overall yield) as a yellow oil.

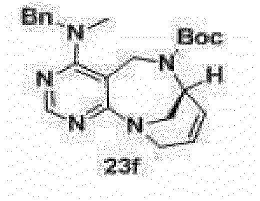

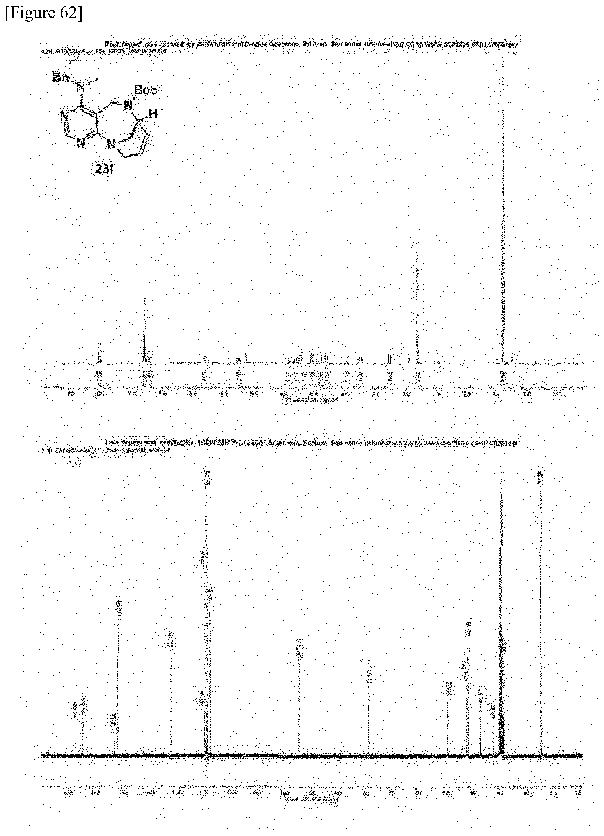

*R*_{f} = 0.2 (hexane/EA = 1:1); ¹H NMR (400 MHz, DMSO-*d₆*, 100 °C) δ 8.06 (s, 1H), 7.35-7.29 (m, 4H), 7.27-7.23 (m, 1H), 6.35 (ddd, *J =* 9.9, 5.3, 1.8 Hz, 1H), 5.78 (ddd, *J =* 10.1, 3.9, 1.8 Hz, 1H), 4.92 (d, *J* = 16.5 Hz, 1H), 4.86-4.81 (m, 1H), 4.76 (d, *J* = 15.3 Hz, 1H), 4.56 (d, *J* = 15.9 Hz, 1H), 4.42 (dd, *J* = 15.3, 1.2 Hz, 1H), 4.34 (d, *J* = 15.3 Hz, 1H), 4.00 (dd, *J* = 4.9, 1.8 Hz, 1H), 3.77 (dq, *J* = 18.3, 2.2 Hz, 1H), 3.30 (dd, *J* = 15.3, 2.4 Hz, 1H), 2.84 (s, 3H), 1.42 (s, 9H); ¹³C NMR (100 MHz, DMSO-*d₆*, 100 °C) δ 166.0, 163.6, 154.6, 153.5, 137.9, 128.0, 127.7, 127.3, 127.1, 126.3, 99.7, 79.0, 55.4, 49.9, 49.4, 45.7, 41.9, 38.7, 27.7; IR (neat) vmax: 3031, 2978, 2928, 1690, 1588, 1408, 1164 cm⁻¹; HRMS(ESI+): Calcd for a C₂₃H₃₀N₅O₂⁺ [M+H]⁺ 408.2394, found 408.2382, Δppm -2.94. (Fig. 62).

### Experiment 1 : Cell Culture

HEK293T cell was cultured in Dulbecco modified eagle medium (DMEM) with 10% (v/v) fetal bovine serum (FBS) and 1% (v/v) antibiotic-antimycotic solution. HeLa, DU145, and Ca Ski cells were cultured in RPMI 1640 medium with 10% (v/v) FBS and 1% (v/v) antibiotic-antimycotic solution. The cells were cultured in 100-mm culture dish in an incubator at 37 °C, in 5% CO₂.

### Experiment 2 : ELISA

His-tagged human LRS proteins were diluted in carbonate buffer (100 mM, pH 9.6) to the concentration of 0.5 ng µl⁻¹. The proteins were distributed to the plate (half-bottom 96 well clear plate) from CORNING 3690 and subjected to incubation overnight at 4 °C refrigerator under sealed. Protein from each well was removed and washed three times with PBS mixed with 0.05% Tween® 20 (PBST), subjected to blocking wih PBS containing 5% bovine serum albumin (BSA) for 2 hours. Each well was washed with PBST, followed by the treatment of a compound and GST-tagged human RagD protein simultaneously for 3 hours. GST protein itself was used as a negative control. Each well was treated with GST antibody diluted in PBST and incubated at room temperature for 1 h. After washing with PBST, the HRP-conjugated anti-horse IgG secondary antibody was incubated at room temperature for 1 h. TMB was added to each well for colorimetric development. Blue color was found in positive wells. To stop the chemical reaction, the treatment with 1 m H₃PO₄ was done. Finally, absorbance at 450 nm were measured.

The experiment result shows that compound 21f inhibits the LRS-RagD interaction in a dose-dependent manner (Fig. 15).

### Experiment 3: Western Blotting

Cells were lysed with Radio-ImmunoPrecipitation Assay (RIPA) buffer (50 mM Tris, pH 7.8, 150 mM NaCl, 0.5% deoxycholate, 1% IGEPAL CA-630, protease inhibitor cocktail, and phosphatase inhibitors). Protein was obtained by transferring supernatant after centrifugation at 15000 rpm for 20 min. Protein concentration was measured with Micro BCA™ protein assay kit. Overall protein sampling procedure was done at 4 °C. Prepared protein samples were analyzed with SDS-PAGE. Protein after electrophoresis was transferred into nitrocellulose membrane. Membrane was blocked with TBST mixed with 2% BSA over at least 1 h at r.t. Primary antibodies were incubated overnight at 4 °C. The treatment concentration was as follows : Anti-LC3B (ab51520); 1:1000, anti-S6KI (ab32359); 1:1000, anti-phsopho-T389 S6K1 (ab2571); 1:800, anti-p62 (CST 5114); 1:1000, anti-glyceraldehyde-3-phosphate dehydrogenase (GAPDH) (CST 2118); 1:1000, anti-phospho-S65 4E-BP1 (CST 9451); 1:1000, anti-phospho-S757 ULK1 (CST 6888); 1:800, anti-phospho-S473 Akt (CST 4058); 1: 1000, anti-phospho-T172 AMPKα (CST 2531). After washing with TBST, HRP-labeled anti-rabbit IgG secondary antibody was treated at room temperature for 1 h. After washing with TBST, developing by Amersham ECL prime solution was done. Chemiluminescent signal was imaged by ChemiDoc™ MP imaging system.

It was confirmed from the experiment that 21f inhibits an activation of mTORC1 in a dose-dependent manner as discussed below.

The effect of rapamycin on mTORC1 within cells was reviewed over 0 to 24 hours. HEK293T cells were treated with 500 nM of rapamycin. Phospho-T389 S6K1 and phospho-S65 4E-BP1 were reduced as the time passed wherein the effect lasted for at least about 12 hours. This is similar to the result with 21f. However, against phospho-S757 ULK1, it was confirmed that 21f exerted high inhibition degree, while rapamycin not so high. This shows that 21f functions in a different mechanism from rapamycin (Fig. 18).

### Experiment 4: Surface Plasmon Resonance (SPR) Assay

The dissociation rate constant (K_{D}) toward His-LRS proteins was determined by using SPR instrument (Biacore T100) at the national center for inter-university research facilities (NCIRF) in Seoul National University. The carboxyl group on the surface of CM5 chip was able to be replaced with protein by forming reactive succinimide ester with 1-ethyl-3-(3-dimethylaminopropyl)-carbodiimide (EDC) and *N*-hydroxysuccinimide (NHS). Human LRS proteins (1.5× PBS, pH 7.3) were immobilized on flow cell 2 (imed RU; 12000) by leading to amide bond formation with NHS ester. The residual NHS ester was quenched with 1 M ethanolamine-HCl (pH 8.0). Amide bond formation alone, without any proteint, was performed on flow cell 1 as a control. The immobilization process was proceeded on PBS buffer. After the immobilization of hLRS, compounds were flowed for 60 s at a flow rate of 20 µl min⁻¹ in various concentrations from 1 µM to 15 µM, Buffer was then flowed at the same flow rate for 200s for dissociation of compounds. As a running buffer, 1× PBS (pH 7.3) containing 3% DMSO and 0.005% P₂0 was used. The experiment was performed at 25 °C. Data analysis was done by using Biacore T100 Evaluation software. Final sensorgrams were obtained after the elimination of results from flow cell control and buffer-only control. The dissociation constant (K_{D}) was calculated by fitting to the 1:1 binding model.

It was confirmed from the experiment that 21f binds LRS in a dose-dependent manner (Fig. 15).

### Experiment 5: Transfection

HeLa cells were seeded in chambered coverglass from Nunc and incubated for 24 h. mCherry-GFP-LC3 plasmid was transfected to the cells using Lipofectamine™ 2000 solution. Transfection was proceeded according to manufacturer's protocol.

### Experiment 6: mCherry-GFP-LC3 puncta imaging

Delta Vision Elite imaging system was used for the imaging of mCherry-GFP-LC3 transfected HeLa cell. For live-cell imaging, chamber was maintained at 37 °C and in 5% CO₂. Cells were observed with 60× scale, using mCherry/mCherry, GFP/GFP (Excitation/Emission) filter sets. The wavelength range of the used filters is as follows : mCherry (Excitation: 575/25 nm, Emission: 625/45 nm); GFP (Excitation: 475/28 nm, Emission: 525/48 nm). Images were processed and analyzed with SoftWorks deconvolution software.

### Experiment 7: Cell proliferation assay

HEK293T cells were seeded in 96-well plate from CORNING. 24 h after seeding, the compound diluted with DMEM at 5 µM of final concentration was treated in cells while leucine-free DMEM was treated in cells as a control. Cell division assay was done with WSTs assay and BrdU colorimetric kit, following the manufacturer's protocol.

Hereinafter, the detailed results and analysis of the experiments are discussed.

### Results of Experiments

The present inventors found from the experiments that LRS can play a noncanonical role of in amino acid-dependent mTORC1 activation by sensing intracellular Leu concentration. According to this study, LRS can mediate Leu signalling to mTORC1 via direct binding to RagD-GTP protein and form an LRS-RagD protein complex in a Leu-dependent manner, which leads to the translocation of mTORC1 to the lysosome cellular membrane and subsequent activation of mTORC1 (Fig. 15a). Thus, LRS-RagD interactions could serve as a Leu-sensing mechanism in the nutrition-dependent activation of mTORC1. Further, novel small-molecule PPI inhibitors could serve as lead compounds for the development of potential therapeutic agents with new modes of action to treat human diseases linked to the oncogenic activation of mTORC1.

To identify novel small-molecule PPI modulators towards the LRS-RagD interaction, the pDOS library was subjected to ELISA-based HTS using purified LRS and GST (glutathione-S-transferase) where aziridine-containing 20f and 21f were identified as dose-dependent inhibitors of the LRS-RagD interaction (Fig. 15b). On the basis of this data, we hypothesized that 20f and 21f inhibit the LRS-mediated Leu signalling to mTORC1 via direct disruption of the LRS-RagD interaction, which resulted in decreasing phosphorylated S6K1, a dominant substrate of mTORC1 kinase. As shown in Fig. 15c, the level of phosphorylated S6K1 was decreased in the absence of Leu. Similarly, 20f and 21f suppressed the phosphorylation of S6K1 even in the presence of Leu. On the basis of the reduction of LRS-RagD interaction by ELISA (20f 15.5%; 21f 30.2%) and the phosphorylation suppression of S6K1 by western blotting (20f 16.4%; 21f 48.9%; Fig. 15d), we selected 21f as the candidate compound and subjected it to further western blot analysis and biophysical study using surface plasmon resonance (SPR; Fig. 15e) spectroscopy. As shown in Fig. 15d, compound 21f downregulated the phosphorylation of S6K1 in a dose-dependent manner. Further, compound 21f directly bound to LRS with 4.5(±0.78) of K_{D} when measured by SPR. This clearly supported that 21f directly binds to LRS and disrupts the interaction between LRS and RagD (Fig. 15).

It was examined whether compound 21f stimulates cellular autophagy through the inhibition of LRS-mediated activation of mTORC1. HeLa human cervical cancer cells were treated with rapamycin (Rap), bafilomycin (Baf), or 21f in the presence of Leu. Autophagic activity was evaluated using western blot analysis with autophagosome biomarker wherein microtubule-associated proteins 1 light chain 3 (LC3) and ubiquitin-binding protein p62 are used as the autophagosome biomarker. This is because conversion of LC3 to LC3 is associated with the formation of autophagosome and the degradation of p62 reflects autolysosome formation indicating the final progression of the autophagic processes. Baf is a known inhibitor of vacuolar-type H+-ATPase, which inhibits the autolysosomal degradation of cellular contents as well as autophagosomal-lysosomal fusion through the inhibition of acidification, thereby blocking the final-stage flux of autophagy. As shown in Fig. 16a, the treatment with Baf resulted in an increased ratio of LC3/I, and accumulation of p62 compared with the DMSO control. In contrast, the treatment with Rap or 21f as an inhibitor of mTORC1 increased the cellular level of LC3 and stimulated the degradation of p62, which was consistent with their suppressive effects on mTORC1 activity. To monitor autophagy in living cells, we performed the mCherry-green fluorescent protein (GFP)-LC3 assay. Because of the difference in acid sensitivity of mCherry (acid-stable) and GFP (acid-sensitive), the entire autophagy process from autophagosome to autolysosome formation could be differentiated by the fluorescence emission pattern (Fig. 16b). For instance, cells expression of mCherry-GFP-LC3 show yellow puncta when autophagosomes are formed. In contrast, the formation of autolysosome via autophagosomal-autolysosomal fusion results in red puncta because the acidic environment of autolysosomes quenches GFP fluorescence. As shown in Fig. 16c, yellow puncta were clearly observed upon treatment with Baf, which is consistent with the inhibitory effect of Baf on autophagosomal-autolysosomal fusion. When cells were treated with Rap or 21f, red puncta were clearly observed, which indicates that compound 21f could activate autophagy via the suppression of LRS-mediated activation of mTORC1. Finally, we evaluated whether compound 21f could inhibit Leu-mediated cell proliferation by using BrdU and WST assay, because Leu plays a critical role in promoting cellular proliferation through the activation of mTORC1. As shown in Fig. 16d (BrdU assay), compound 21f suppressed cell proliferation even in the presence of Leu, similar to in the Leu-deprived medium, which indicated that compound 21f could inhibit Leu-mediated signalling to mTORC1 by disrupting the LRS-RagD interaction (Fig. 16).

## Claims

1. A pyrimidine derivative compound represented by any one of formulas I to IX: 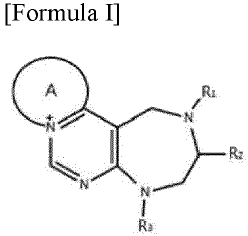 wherein:
A forms a heterobicyclic ring together with adjacent N and C atoms wherein the heterobicyclic ring is an 8- to 9-membered bicyclic ring which may contain 1 to 3 nitrogen atoms;
R₁ is -Boc or a benzyl group;
R₂ is -CH₂-O-TIPS; and
R₃ is hydrogen, a C₁ to C₃ alkyl group, an allyl group, -Tf, C₁ to C₃ alkyl benzyl, or 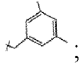 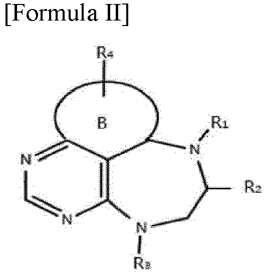
wherein
B forms a 6- to 10-membered ring together with adjacent 2 to 3 C atoms wherein the ring may contain 1 to 3 nitrogen atoms;
R₁ is -Boc or a benzyl group;
R₂ is -CH₂-O-TIPS; and
R₃ is hydrogen, a C₁ to C₃ alkyl group, an allyl group, -Tf, a C₁ to C₃ alkyl benzyl group, or 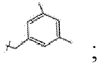 and
R₄ is a C₁ to C₃ alkyl group or -CH₂-PMB; 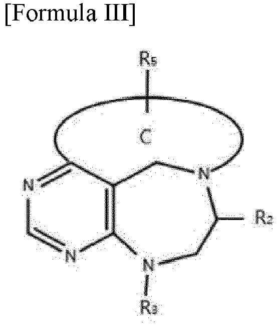
wherein
C forms a 10- to 14-membered ring together with adjacent N and C atoms wherein the ring may contain 1 to 3 nitrogen atoms;
R₂ is -CH₂-O-TIPS;
R₃ is hydrogen, a C₁ to C₃ alkyl group, an allyl group, -Tf, a C₁ to C₃ alkyl benzyl group, or 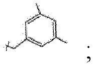 and
R₅ is a C₁ to C₃ alkyl group or -CH₂-PMB; 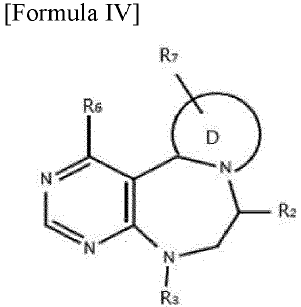
wherein
D forms a 4- to 6-membered cyclic imide together with adjacent N and C atoms wherein the cyclic imide is a ring which may contain 1 to 3 nitrogen atoms;
R₂ is -CH₂-O-TIPS;
R₃ is hydrogen, a -C₁ to C₃ alkyl group, an allyl group, -Tf, a C₁ to C₃ alkyl benzyl group, or 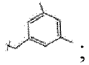
R6 is 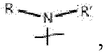 wherein R and R' are each independently a benzyl group or a C₁ to C₃ alkyl group; and R₇ is a benzyl group, a phenyl group, or 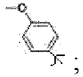 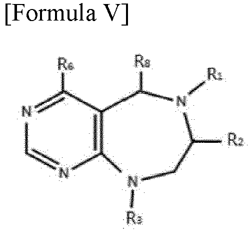
wherein
R₁ is -Boc or a benzyl group;
R₂ is -CH₂-O-TIPS; and
R₃ is hydrogen, a -C₁ to C₃ alkyl group, an allyl group, -Tf, a C₁ to C₃ alkyl benzyl group, or 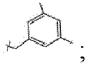
R₆ is 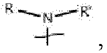 wherein R and R' are each independently a benzyl group or a C₁ to C₃ alkyl group; and
Rs is an acetylene group; 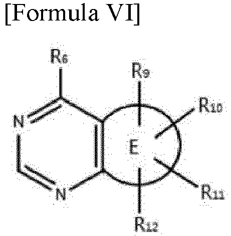
wherein
E forms an 8- to 10-membered heterocyclic ring together with adjacent two C atoms wherein the heterocyclic ring is a saturated or unsaturated ring which contains at least two of 2 to 4 nitrogen atoms and 1 to 3 oxigen atoms;
R₆ is 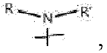 wherein R and R' are each independently a benzyl group or a C₁ to C₃ alkyl group; and
R₉ to R₁₂ are each independently hydrogen, a methyl group, an azide group, a benzyl group, -CH₂-O-TIPS or -Tf; 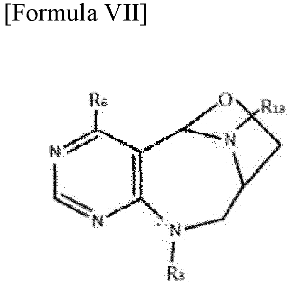
wherein
R₃ is hydrogen, a -C₁ to C₃ alkyl group, an allyl group, -Tf, a C₁ to C₃ alkyl benzyl group, or 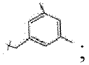
R₆ is 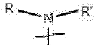 wherein R and R' are each independently a benzyl group or a C₁ to C₃ alkyl group; and
R₁₃ is -Boc, -CONHBn, -m-Ns; 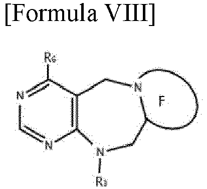
wherein
F is -X-(CH₂)n-(O-CH₂)m- and forms a 3- to 6-membered heterocyclic ring together with adjacent N and C atoms wherein X is 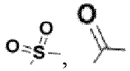 or -CH₂-, and n and m are respectively an integer from 0 to 2;
R₃ is hydrogen, a -C₁ to C₃ alkyl group, an allyl group, -Tf, a C₁ to C₃ alkyl benzyl group, or 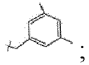 and
R₆ is 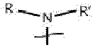 wherein R and R' are each independently a benzyl group or a C₁ to C₃ alkyl group; 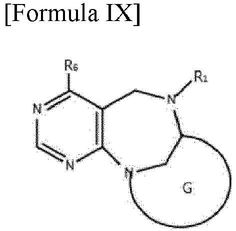
wherein
G forms a 6- to 9-membered heterocyclic ring together with adjacent N and C atoms wherein the heterocyclic ring is an unsaturated heterocyclic ring which contains 2 to 3 nitrogen atoms;
R₁ is -Boc or a benzyl group; and
R₆ is 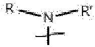 wherein R and R' are each independently a benzyl group or a C₁ to C₃ alkyl group;
or a pharmaceutically acceptable salt thereof.

2. The compound according to Claim 1, wherein the compound is represented by any one of formulas I-1 to IX-1: 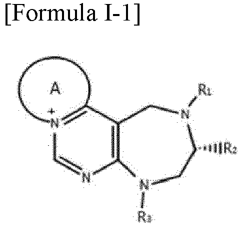 wherein
A forms a heterobicyclic ring together with adjacent N and C atoms wherein the heterobicyclic ring is an 8- to 9-membered bicyclic ring which may contain 1 to 3 nitrogen atoms;
R₁ is -Boc or a benzyl group;
R₂ is -CH₂-O-TIPS; and
R₃ is hydrogen, a C₁ to C₃ alkyl group, an allyl group, -Tf, a C₁ to C₃ alkyl benzyl, or 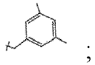 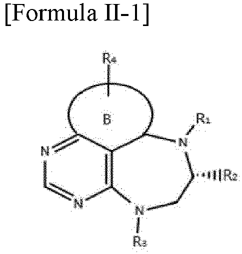
wherein
B forms a 6- to 10-membered ring together with adjacent 2 to 3 C atoms wherein the ring may contain 1 to 3 nitrogen atoms;
R₁ is -Boc or a benzyl group;
R₂ is -CH₂-O-TIPS; and
R₃ is hydrogen, a C₁ to C₃ alkyl group, an allyl group, -Tf, a C₁ to C₃ alkyl benzyl group, or 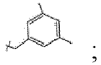 and
R₄ is a C₁ to C₃ alkyl group or -CH₂-PMB; 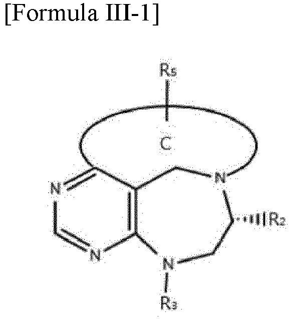
wherein
C forms a 10- to 14-membered ring together with adjacent N and C atoms wherein the ring may contain 1 to 3 nitrogen atoms;
R₂ is -CH₂-O-TIPS;
R₃ is hydrogen, a C₁ to C₃ alkyl group, an allyl group, -Tf, a C₁ to C₃ alkyl benzyl group, or 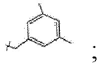 and
R₅ is a C₁ to C₃ alkyl group or -CH₂-PMB; 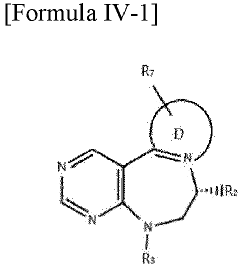
wherein
D forms a 4- to 6-membered cyclic imide together with adjacent N and C atoms wherein the cyclic imide is a ring which may contain 1 to 3 nitrogen atoms;
R₂ is -CH₂-O-TIPS;
R₃ is hydrogen, a -C₁ to C₃ alkyl group, an allyl group, -Tf, a C₁ to C₃ alkyl benzyl group, or 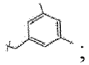
R₆ is 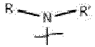 wherein R and R' are each independently a benzyl group or a C₁ to C₃ alkyl group;
R₇ is a benzyl group, a phenyl group, or 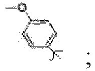 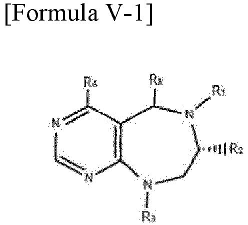
wherein
R₁ is -Boc or a benzyl group;
R₂ is -CH₂-O-TIPS; and
R₃ is hydrogen, a -C₁ to C₃ alkyl group, an allyl group, -Tf, a C₁ to C₃ alkyl benzyl group, or 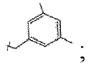
R₆ is 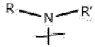 wherein R and R' are each independently a benzyl group or a C₁ to C₃ alkyl group; and
Rs is an acetylene group; 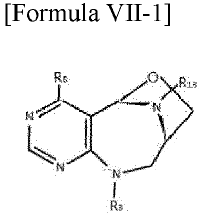
wherein
R₃ is hydrogen, a -C₁ to C₃ alkyl group, an allyl group, -Tf, a C₁ to C₃ alkyl benzyl group, or 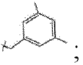
R₆ is 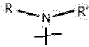 wherein R and R' are each independently a benzyl group or a C₁ to C₃ alkyl group; and
R₁₃ is -Boc, -CONHBn, -m-Ns; 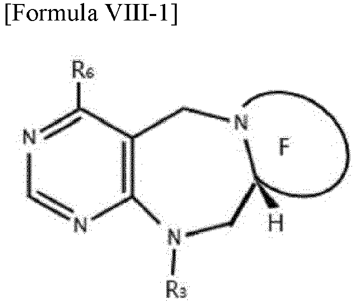
wherein
F is -X-(CH₂)n-(O-CH₂)m- and forms a 3- to 6-membered heterocyclic ring together with adjacent N and C atoms wherein X is 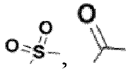 or -CH₂-, and n and m are respectively an integer from 0 to 2;
R₃ is hydrogen, a -C₁ to C₃ alkyl group, an allyl group, -Tf, a C₁ to C₃ alkyl benzyl group, or 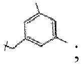 and
R₆ is 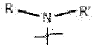 wherein R and R' are each independently a benzyl group or a C₁ to C₃ alkyl group; 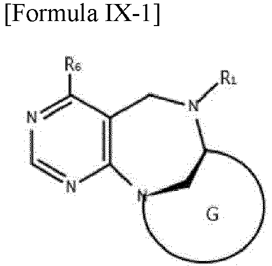
wherein
G forms a 6- to 9-membered heterocyclic ring together with adjacent N and C atoms wherein the heterocyclic ring is an unsaturated heterocyclic ring which contains 2 to 3 nitrogen atoms;
R₁ is -Boc or a benzyl group; and
R₆ is 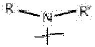 wherein R and R' are each independently a benzyl group or a C₁ to C₃ alkyl group;
or a pharmaceutically acceptable salt thereof.

3. The compound according to Claim 1, wherein the compound is represented by any one of the formulas: 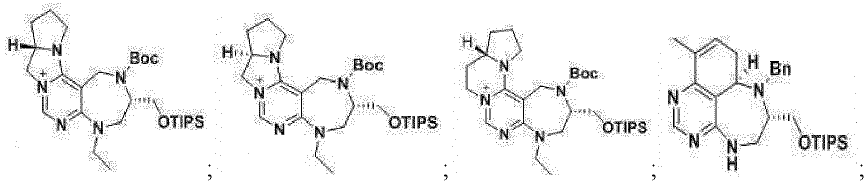 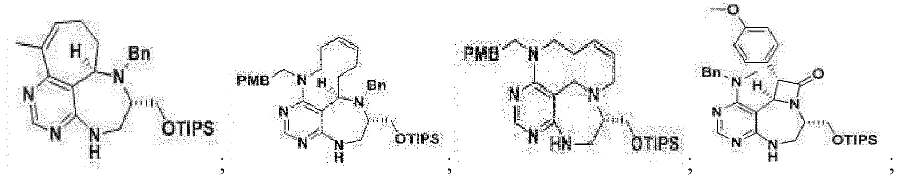 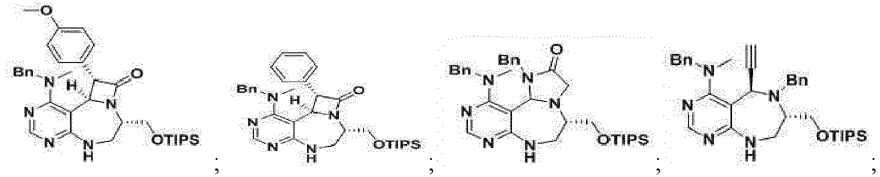 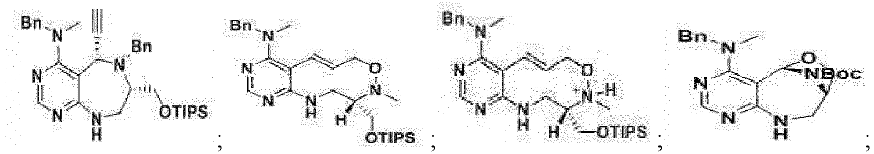 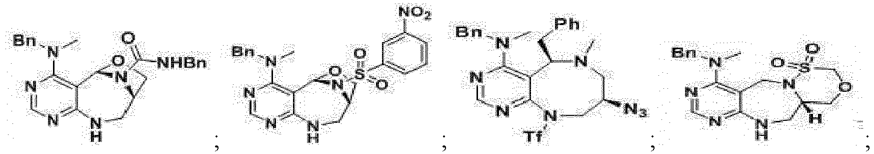 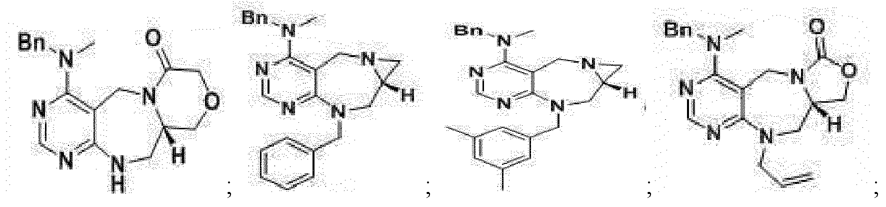 and 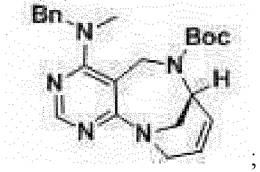 or a pharmaceutically acceptable salt thereof.

4. A process for preparing the pyrimidine derivative compound according to Claim 1 or a pharmaceutically acceptable salt thereof, **characterized in** comprising:
step 1 for preparing an intermediate substance, tert-(S)-(1-(6-chloro-5-formylpyrimidin-4-yl)amino)-3-((triisopropylsilyl)oxy)propan-2-yl)carbamate;
step 1a for preparing a starting material represented by formula 1 by reacting the intermediate substance prepared in step 1 with cyclic amine or tetrakis palladium; and 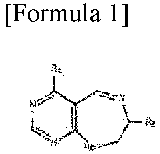 wherein
R₁ is 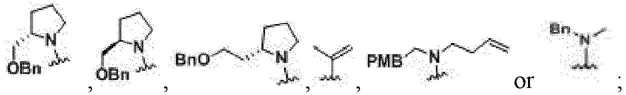
R₂ is -CH₂-O-TIPS;
step 2 for preparing a desired compound from the starting material prepared in step la.

5. The process for preparing according to Claim 4, wherein step 2 comprises at least one of the following reactions on the starting material of step la: N-alkylation, debenzylation, intramolecular substitution reaction, ring-closing metathesis, nucleophilic addition reaction, allylation reaction of amine, Grubbs' catalyst condensation reaction, alcohol deprotection reaction, intramolecular nucleophilic addition reaction, aziridium intermediate formation reaction, nucleophilic ring expansion reaction, and bridge head ring formation reaction.

6. A composition for use in treating cancer comprising the pyrimidine derivative compound according to Claim 1 or a pharmaceutically acceptable salt thereof as an active ingredient.

7. The composition for use according to Claim 6, **characterized in that** the cancer is at least one selected from the group consisting ofcervical cancer, lung cancer, pancreatic cancer, non-small cell lung cancer, liver cancer, colon cancer, bone cancer, skin cancer, head or neck cancer, melanoma in skin or eye, uterine cancer, ovarian cancer, rectal cancer, gastric cancer, cancer around anal, colon cancer, breast cancer, fallopian tube carcinoma, endometrial carcinoma, vaginal carcinoma, vulvar carcinoma, esophageal cancer, small intestine cancer, endocrine gland cancer, thyroid cancer, parathyroid cancer, adrenal cancer, soft tissues sarcoma, urethral cancer, penile cancer, prostate cancer, bladder cancer, cancer of renal or ureter, renal cell carcinoma, and renal pelvis carcinoma.

8. The composition for use according to Claim 6, **characterized in that** the cancer is cervical cancer.

## Patentansprüche

1. Pyrimidinderivatverbindung, wiedergegeben durch eine der Formeln I bis IX: 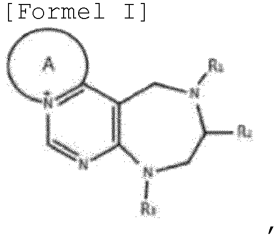 wobei:
A zusammen mit benachbarten N- und C-Atomen einen heterobicyclischen Ring bildet, wobei es sich bei dem heterobicyclischen Ring um einen 8- bis 9-gliedrigen bicyclischen Ring handelt, der 1 bis 3 Stickstoffatome enthalten kann,
R₁ für -Boc oder eine Benzylgruppe steht,
R₂ für -CH₂-O-TIPS steht und
R₃ für Wasserstoff, eine C₁- bis C₃-Alkylgruppe, eine Allylgruppe, -Tf, C₁- bis C₃-Alkylbenzyl oder 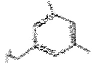 steht, 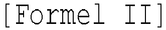 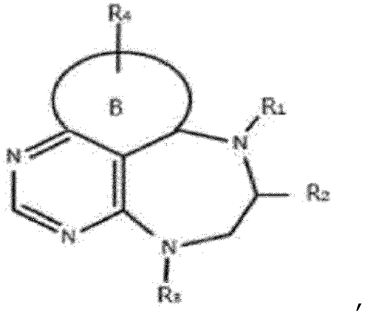
wobei
B zusammen mit 2 bis 3 benachbarten C-Atomen einen 6- bis 10-gliedrigen Ring bildet, wobei der Ring 1 bis 3 Stickstoffatome enthalten kann,
R₁ für -Boc oder eine Benzylgruppe steht,
R₂ für -CH₂-O-TIPS steht und
R₃ für Wasserstoff, eine C₁- bis C₃-Alkylgruppe, eine Allylgruppe, -Tf, eine C₁- bis C₃-Alkylbenzylgruppe oder 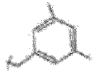 steht und
R₄ für eine C₁- bis C₃-Alkylgruppe oder -CH₂-PMB steht, 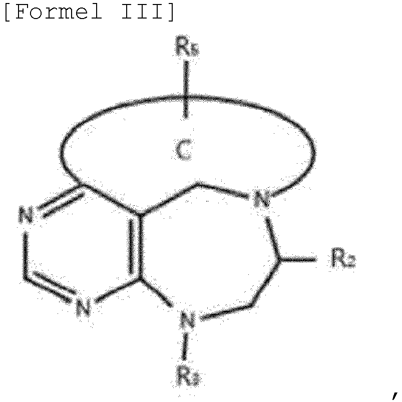
wobei
C zusammen mit benachbarten N- und C-Atomen einen 10- bis 14-gliedrigen Ring bildet, wobei der Ring 1 bis 3 Stickstoffatome enthalten kann,
R₂ für -CH₂-O-TIPS steht,
R₃ für Wasserstoff, eine C₁- bis C₃-Alkylgruppe, eine Allylgruppe, -Tf, eine C₁- bis C₃-Alkylbenzylgruppe oder 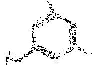 steht und
R₅ für eine C₁- bis C₃-Alkylgruppe oder -CH₂-PMB steht, 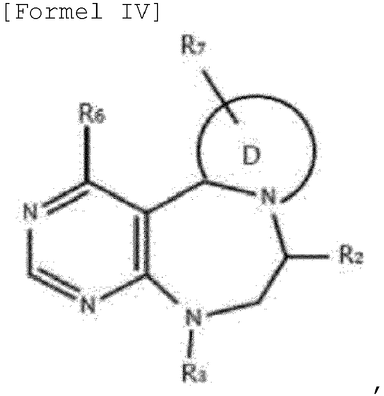
wobei
D zusammen mit benachbarten N- und C-Atomen ein 4- bis 6-gliedriges cyclisches Imid bildet, wobei es sich bei dem cyclischen Imid um einen Ring handelt, der 1 bis 3 Stickstoffatome enthalten kann,
R₂ für -CH₂-O-TIPS steht,
R₃ für Wasserstoff, eine C₁- bis C₃-Alkylgruppe, eine Allylgruppe, -Tf, eine C₁- bis C₃-Alkylbenzylgruppe oder 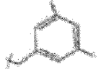 steht,
R₆ für 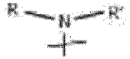 steht, wobei R und R' jeweils unabhängig für eine Benzylgruppe oder eine C₁- bis C₃-Alkylgruppe stehen und R₇ für eine Benzylgruppe, eine Phenylgruppe oder 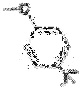 steht, 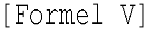 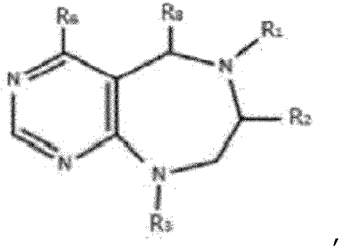
wobei
R₁ für -Boc oder eine Benzylgruppe steht,
R₂ für -CH₂-O-TIPS steht und
R₃ für Wasserstoff, eine C₁- bis C₃-Alkylgruppe, eine Allylgruppe, -Tf, eine C₁- bis C₃-Alkylbenzylgruppe oder 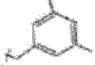 steht,
R₆ für 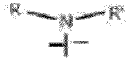 steht, wobei R und R' jeweils unabhängig für eine Benzylgruppe oder eine C₁- bis C₃-Alkylgruppe stehen, und
R₈ für eine Acetylengruppe steht, 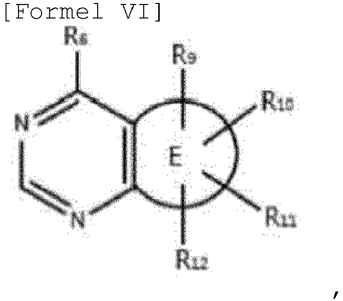
wobei
E zusammen mit zwei benachbarten C-Atomen einen 8- bis 10-gliedrigen heterocyclischen Ring bildet, wobei es sich bei dem heterocyclischen Ring um einen gesättigten oder ungesättigten Ring handelt, der mindestens zwei von 2 bis 4 Stickstoffatomen und 1 bis 3 Sauerstoffatomen enthält,
R₆ für 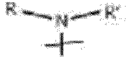 steht, wobei R und R' jeweils unabhängig für eine Benzylgruppe oder eine C₁- bis C₃-Alkylgruppe stehen und
R₉ bis R₁₂ jeweils unabhängig für Wasserstoff, eine Methylgruppe, eine Azidgruppe, eine Benzylgruppe, -CH₂-O-TIPS oder -Tf stehen, 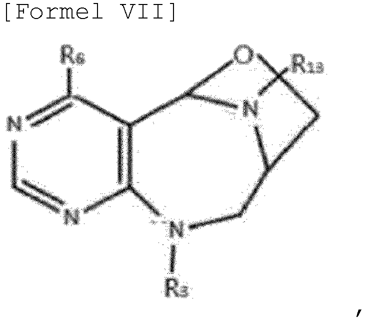
wobei
R₃ für Wasserstoff, eine C₁- bis C₃-Alkylgruppe, eine Allylgruppe, -Tf, eine C₁- bis C₃-Alkylbenzylgruppe oder 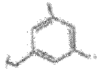 steht,
R₆ für 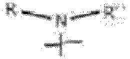 steht, wobei R und R' jeweils unabhängig für eine Benzylgruppe oder eine C₁- bis C₃-Alkylgruppe stehen und
R₁₃ für -Boc, -CONHBn oder -m-Ns steht, 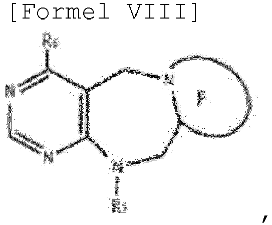
wobei
F für -X-(CH₂)ₙ-(O-CH₂)ₘ- steht und zusammen mit benachbarten N- und C-Atomen einen 3- bis 6-gliedrigen heterocyclischen Ring bildet, wobei X für 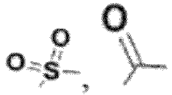 oder -CH₂- steht und n und m jeweils für eine ganze Zahl von 0 bis 2 stehen,
R₃ für Wasserstoff, eine C₁- bis C₃-Alkylgruppe, eine Allylgruppe, -Tf, eine C₁- bis C₃-Alkylbenzylgruppe oder 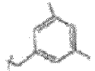 steht und
R₆ für 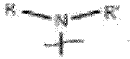 steht, wobei R und R' jeweils unabhängig für eine Benzylgruppe oder eine C₁- bis C₃-Alkylgruppe stehen, 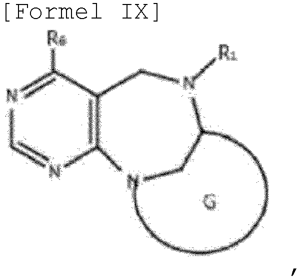
wobei
G zusammen mit benachbarten N- und C-Atomen einen 6- bis 9-gliedrigen heterocyclischen Ring bildet, wobei es sich bei dem heterocyclischen Ring um einen ungesättigten heterocyclischen Ring handelt, der 2 bis 3 Stickstoffatome enthält,
R₁ für -Boc oder eine Benzylgruppe steht und
R₆ für 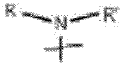 steht, wobei R und R' jeweils unabhängig für eine Benzylgruppe oder eine C₁- bis C₃-Alkylgruppe stehen,
oder ein pharmazeutisch unbedenkliches Salz davon.

2. Verbindung nach Anspruch 1, wobei die Verbindung durch eine der Formeln I-1 bis IX-1 wiedergegeben wird: 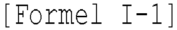 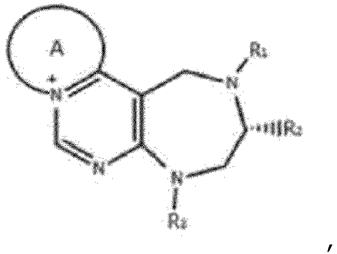 wobei:
A zusammen mit benachbarten N- und C-Atomen einen heterobicyclischen Ring bildet, wobei es sich bei dem heterobicyclischen Ring um einen 8- bis 9-gliedrigen bicyclischen Ring handelt, der 1 bis 3 Stickstoffatome enthalten kann,
R₁ für -Boc oder eine Benzylgruppe steht,
R₂ für -CH₂-O-TIPS steht und
R₃ für Wasserstoff, eine Ci- bis C₃-Alkylgruppe, eine Allylgruppe, -Tf, C₁- bis C₃-Alkylbenzyl oder 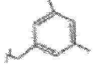 steht, 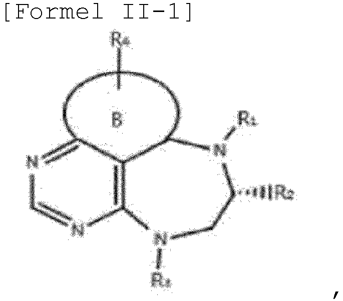
wobei
B zusammen mit 2 bis 3 benachbarten C-Atomen einen 6- bis 10-gliedrigen Ring bildet, wobei der Ring 1 bis 3 Stickstoffatome enthalten kann,
R₁ für -Boc oder eine Benzylgruppe steht,
R₂ für -CH₂-O-TIPS steht und
R₃ für Wasserstoff, eine C₁- bis C₃-Alkylgruppe, eine Allylgruppe, -Tf, eine C₁- bis C₃-Alkylbenzylgruppe oder 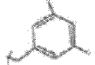 steht und
R₄ für eine C₁- bis C₃-Alkylgruppe oder -CH₂-PMB steht, 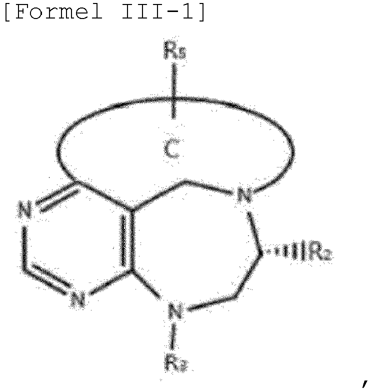
wobei
C zusammen mit benachbarten N- und C-Atomen einen 10- bis 14-gliedrigen Ring bildet, wobei der Ring 1 bis 3 Stickstoffatome enthalten kann,
R₂ für -CH₂-O-TIPS steht,
R₃ für Wasserstoff, eine C₁- bis C₃-Alkylgruppe, eine Allylgruppe, -Tf, eine C₁- bis C₃-Alkylbenzylgruppe oder 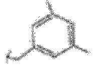 steht und
R₅ für eine C₁- bis C₃-Alkylgruppe oder -CH₂-PMB steht, 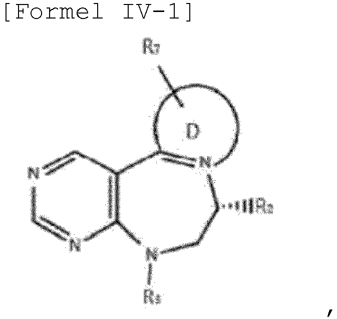
wobei
D zusammen mit benachbarten N- und C-Atomen ein 4- bis 6-gliedriges cyclisches Imid bildet, wobei es sich bei dem cyclischen Imid um einen Ring handelt, der 1 bis 3 Stickstoffatome enthalten kann,
R₂ für -CH₂-O-TIPS steht,
R₃ für Wasserstoff, eine C₁- bis C₃-Alkylgruppe, eine Allylgruppe, -Tf, eine C₁- bis C₃-Alkylbenzylgruppe oder 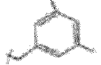 steht,
R₆ für 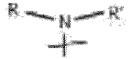 steht, wobei R und R' jeweils unabhängig für eine Benzylgruppe oder eine C₁- bis C₃-Alkylgruppe stehen und R₇ für eine Benzylgruppe, eine Phenylgruppe oder 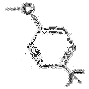 steht, 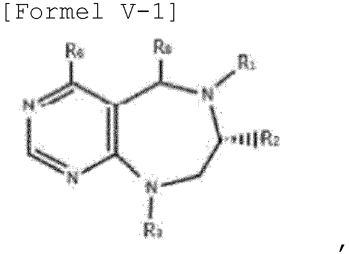
wobei
R₁ für -Boc oder eine Benzylgruppe steht,
R₂ für -CH₂-O-TIPS steht und
R₃ für Wasserstoff, eine C₁- bis C₃-Alkylgruppe, eine Allylgruppe, -Tf, eine C₁- bis C₃-Alkylbenzylgruppe oder 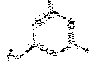 steht,
R₆ für 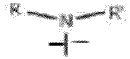 steht, wobei R und R' jeweils unabhängig für eine Benzylgruppe oder eine C₁- bis C₃-Alkylgruppe stehen, und
R₈ für eine Acetylengruppe steht, 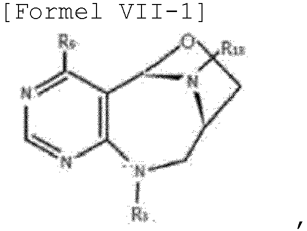
wobei
R₃ für Wasserstoff, eine C₁- bis C₃-Alkylgruppe, eine Allylgruppe, -Tf, eine C₁- bis C₃-Alkylbenzylgruppe oder 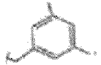 steht,
R₆ für 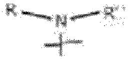 steht, wobei R und R' jeweils unabhängig für eine Benzylgruppe oder eine C₁- bis C₃-Alkylgruppe stehen und
R₁₃ für -Boc, -CONHBn oder -m-Ns steht, 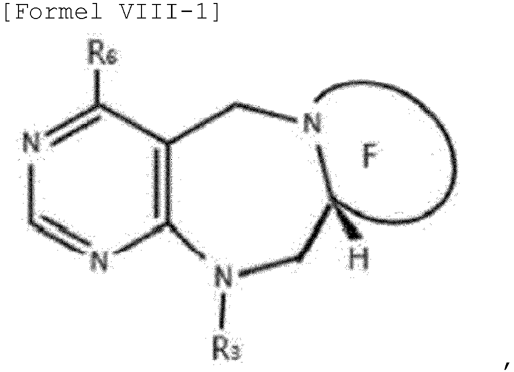
wobei
F für -X-(CH₂)ₙ-(O-CH₂)ₘ- steht und zusammen mit benachbarten N- und C-Atomen einen 3- bis 6-gliedrigen heterocyclischen Ring bildet, wobei X für 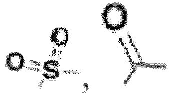 oder -CH₂- steht und n und m jeweils für eine ganze Zahl von 0 bis 2 stehen,
R₃ für Wasserstoff, eine C₁- bis C₃-Alkylgruppe, eine Allylgruppe, -Tf, eine C₁- bis C₃-Alkylbenzylgruppe oder 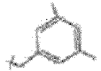 steht und
R₆ für 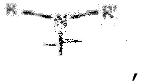
wobei R und R' jeweils unabhängig für eine Benzylgruppe oder eine C₁- bis C₃-Alkylgruppe stehen, 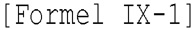 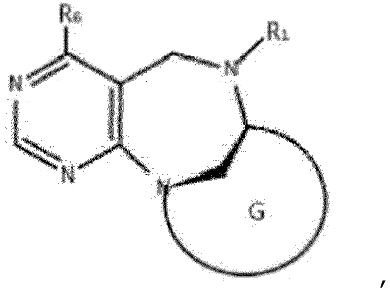
wobei
G zusammen mit benachbarten N- und C-Atomen einen 6- bis 9-gliedrigen heterocyclischen Ring bildet, wobei es sich bei dem heterocyclischen Ring um einen ungesättigten heterocyclischen Ring handelt, der 2 bis 3 Stickstoffatome enthält,
R₁ für -Boc oder eine Benzylgruppe steht und
R₆ für 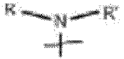 steht, wobei R und R' jeweils unabhängig für eine Benzylgruppe oder eine C₁- bis C₃-Alkylgruppe stehen,
oder ein pharmazeutisch unbedenkliches Salz davon.

3. Verbindung nach Anspruch 1, wobei die Verbindung durch eine der folgenden Formeln wiedergegeben wird: 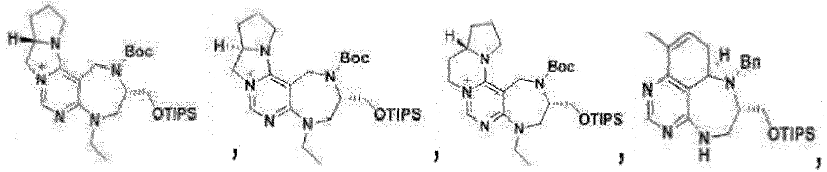 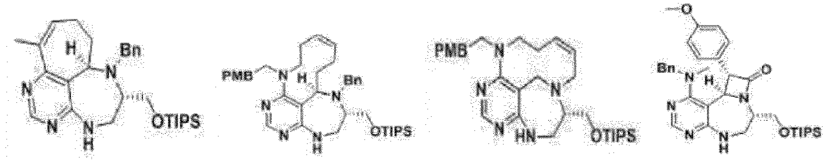 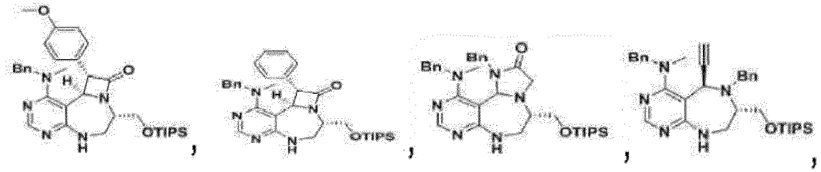 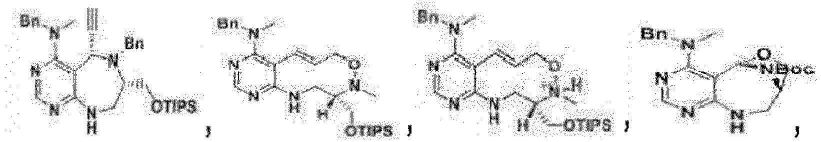 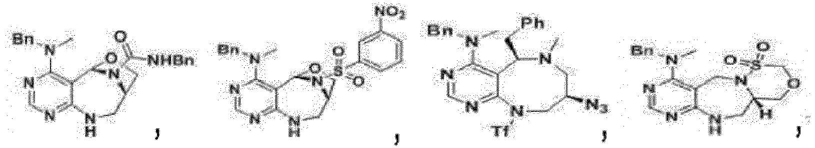 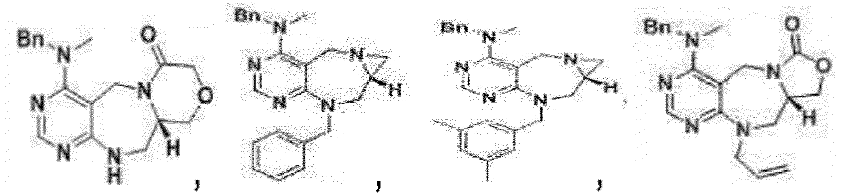 und 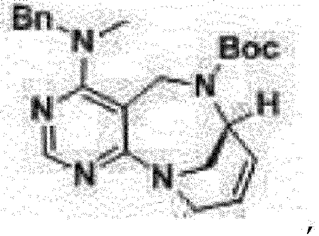 oder ein pharmazeutisch unbedenkliches Salz davon.

4. Herstellungsverfahren der Pyrimidinderivatverbindung nach Anspruch 1 oder eines pharmazeutisch unbedenklichen Salzes davon, **dadurch gekennzeichnet, dass** es Folgendes umfasst:
Schritt 1 zur Herstellung eines Zwischenprodukts tert.-(S)-(1-(6-Chlor-5-formylpyrimidin-4-yl)amino)-3-((triisopropylsilyl)oxy)propan-2-yl)carbamat,
Schritt 1a zur Herstellung eines durch Formel 1 wiedergegebenen Ausgangsmaterials durch Umsetzung des in Schritt 1 hergestellten Zwischenprodukts mit cyclischen Aminen oder Tetrakispalladium und 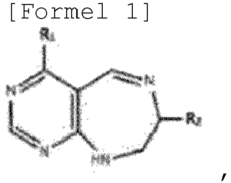
wobei R₁ für 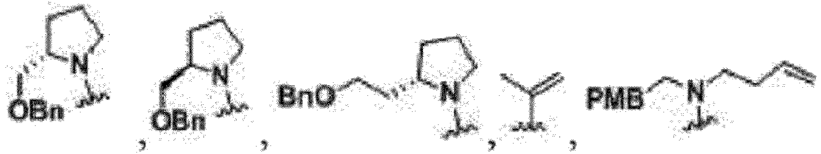 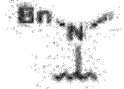 oder steht,
R₂ für -CH₂-O-TIPS steht,
Schritt 2 zur Herstellung einer gewünschten Verbindung aus dem in Schritt 1a hergestellten Ausgangsmaterial.

5. Herstellungsverfahren nach Anspruch 4, wobei Schritt 2 mindestens eine der folgenden Reaktionen mit dem Ausgangsmaterial von Schritt 1a umfasst: N-Alkylierung, Debenzylierung, intramolekulare Substitutionsreaktion, ringschließende Metathese, nukleophile Additionsreaktion, Allylierungsreaktion des Amins, Kondensationsreaktion mit Grubbs-Katalysator, Alkoholentschützungsreaktion, intramolekulare nukleophile Additionsreaktion, Reaktion mit Bildung eines Aziridiumzwischenprodukts, nukleophile Ringerweiterungsreaktion und brückenkopfringbildende Reaktion.

6. Zusammensetzung zur Verwendung bei der Behandlung von Krebs, umfassend die Pyrimidinderivatverbindung nach Anspruch 1 oder ein pharmazeutisch unbedenkliches Salz davon als Wirkstoff.

7. Zusammensetzung zur Verwendung nach Anspruch 6, **dadurch gekennzeichnet, dass** es sich bei dem Krebs um mindestens einen, ausgewählt aus der Gruppe bestehend aus Gebärmutterhalskrebs, Lungenkrebs, Bauchspeicheldrüsenkrebs, nichtkleinzelligem Lungenkrebs, Leberkrebs, Dickdarmkrebs, Knochenkrebs, Hautkrebs, Kopf- oder Halskrebs, Melanom in der Haut oder im Auge, Gebärmutterkrebs, Eierstockkrebs, Rektalkrebs, Magenkrebs, Analkrebs, Dickdarmkrebs, Brustkrebs, Eileiterkarzinom, Endometriumkarzinom, Vaginalkarzinom, Vulvakarzinom, Speiseröhrenkrebs, Dünndarmkrebs, eine Krebserkrankung endokriner Drüsen, Schilddrüsenkrebs, Nebenschilddrüsenkrebs, Nebennierenkrebs, Weichteilsarkom, Harnröhrenkrebs, Peniskrebs, Prostatakrebs, Blasenkrebs, Nieren- oder Harnleiterkrebs, Nierenzellkarzinom und Nierenbeckenkarzinom handelt.

8. Zusammensetzung zur Verwendung nach Anspruch 6, **dadurch gekennzeichnet, dass** es sich bei dem Krebs um Gebärmutterhalskrebs handelt.

## Revendications

1. Composé de type dérivé de pyrimidine représenté par l'une quelconque des formules I à IX : 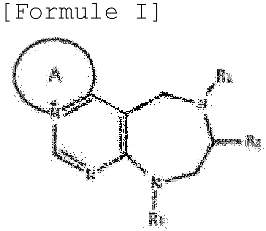
A formant un cycle hétérobicyclique conjointement avec des atomes N et C adjacents, le cycle hétérobicyclique étant un cycle bicyclique à 8 à 9 chaînons qui peut contenir 1 à 3 atomes d'azote ;
R₁ étant -Boc ou un groupe benzyle ;
R₂ étant -CH₂-O-TIPS ; et
R₃ étant hydrogène, un groupe C₁₋₃-alkyle, un groupe allyle, -Tf, C₁₋₃-alkylbenzyle, ou 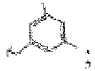 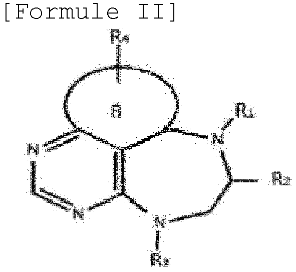
B formant un cycle à 6 à 10 chaînons conjointement avec 2 à 3 atomes C adjacents, le cycle pouvant contenir 1 à 3 atomes d'azote ;
R₁ étant -Boc ou un groupe benzyle ;
R₂ étant -CH₂-O-TIPS ; et
R₃ étant hydrogène, un groupe C₁₋₃-alkyle, un groupe allyle, -Tf, un groupe C₁₋₃-alkylbenzyle, ou 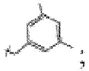 et
R₄ étant un groupe C₁₋₃-alkyle ou -CH₂-PMB ; 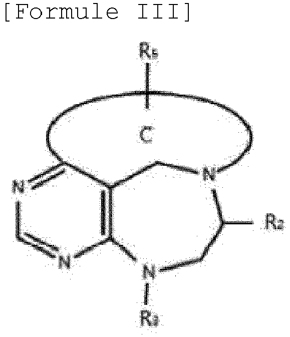
C formant un cycle à 10 à 14 chaînons conjointement avec les atomes N et C adjacents, le cycle pouvant contenir 1 à 3 atomes d'azote ;
R₂ étant -CH₂-O-TIPS ;
R₃ étant hydrogène, un groupe C₁₋₃-alkyle, un groupe allyle, -Tf, un groupe C₁₋₃-alkylbenzyle, ou 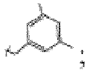 et
R₅ étant un groupe C₁₋₃-alkyle ou -CH₂-PMB ; 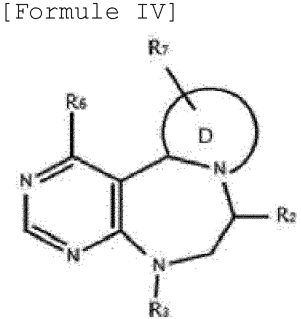
D formant un imide cyclique à 4 à 6 chaînons conjointement avec les atomes N et C adjacents, le cycle imide étant un cycle qui peut contenir 1 à 3 atomes d'azote ;
R₂ étant -CH₂-O-TIPS ;
R₃ étant hydrogène, un groupe C₁₋₃-alkyle, un groupe allyle, -Tf, un groupe C₁₋₃-alkylbenzyle, ou 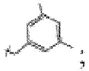
R₆ étant 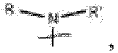 R et R' étant chacun indépendamment un groupe benzyle ou un groupe C₁₋₃-alkyle ; et R₇ étant un groupe benzyle, un groupe phényle, ou 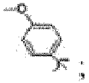 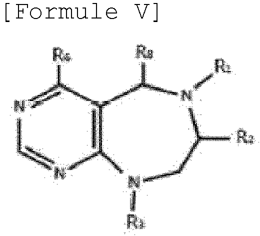
R₁ étant -Boc ou un groupe benzyle ;
R₂ étant -CH₂-O-TIPS ; et
R₃ étant hydrogène, un groupe C₁₋₃-alkyle, un groupe allyle, -Tf, un groupe C₁₋₃-alkylbenzyle, ou 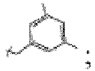
R₆ étant 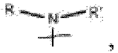 R et R' étant chacun indépendamment un groupe benzyle ou un groupe C₁₋₃-alkyle ; et
R₈ étant un groupe acétylène ; 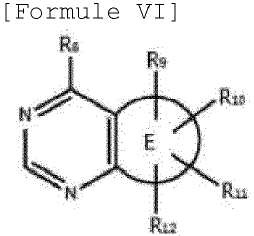
E formant un cycle hétérocyclique à 8 à 10 chaînons conjointement avec deux atomes C adjacents, le cycle hétérocyclique étant un cycle saturé ou insaturé qui contient au moins deux parmi 2 à 4 atomes d'azote et 1 à 3 atomes d'oxygène ;
R₆ étant 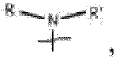 R et R' étant chacun indépendamment un groupe benzyle ou un groupe C₁₋₃-alkyle ; et
R₉ à R₁₂ étant chacun indépendamment hydrogène, un groupe méthyle, un groupe azoture, un groupe benzyle, -CH₂-O-TIPS ou -TF ; 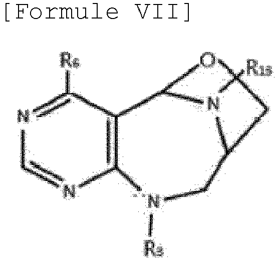
R₃ étant hydrogène, un groupe C₁₋₃-alkyle, un groupe allyle, -Tf, un groupe C₁₋₃-alkylbenzyle, ou 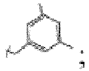
R₆ étant 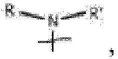 R et R' étant chacun indépendamment un groupe benzyle ou un groupe C₁₋₃-alkyle ; et
R₁₃ étant -Boc, -CONHBn, -m-Ns ; 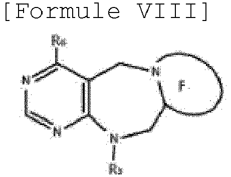
F étant -X-(CH₂)n-(O-CH₂)ₘ- et formant un cycle hétérocyclique à 3 à 6 chaînons conjointement avec des atomes N et C adjacents, X étant 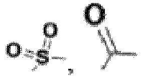 ou -CH₂-, et n et m étant respectivement un entier de 0 à 2 ;
R₃ étant hydrogène, un groupe C₁₋₃-alkyle, un groupe allyle, -Tf, un groupe C₁₋₃-alkylbenzyle, ou 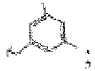
R₆ étant 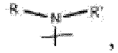 R et R' étant chacun indépendamment un groupe benzyle ou un groupe C₁₋₃-alkyle ; 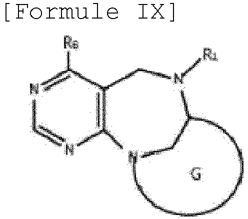
G formant un cycle hétérocyclique à 6 à 9 chaînons avec des atomes N et C adjacents, le cycle hétérocyclique étant un cycle hétérocyclique insaturé qui contient 2 à 3 atomes d'azote ;
R₁ étant -Boc ou un groupe benzyle ; et
R₆ étant 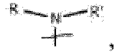 R et R' étant chacun indépendamment un groupe benzyle ou un groupe C₁₋₃-alkyle ;
ou sel pharmaceutiquement acceptable correspondant.

2. Composé selon la revendication 1, le composé étant représenté par l'une quelconque parmi les formules I-1 à IX-1 : 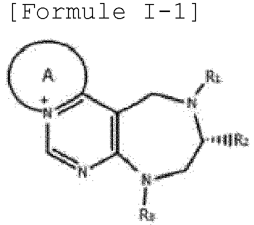
A formant un cycle hétérobicyclique conjointement avec des atomes N et C adjacents, le cycle hétérobicyclique étant un cycle bicyclique à 8 à 9 chaînons qui peut contenir 1 à 3 atomes d'azote ;
R₁ étant -Boc ou un groupe benzyle ;
R₂ étant -CH₂-O-TIPS ; et
R₃ étant hydrogène, un groupe C₁₋₃-alkyle, un groupe allyle, -Tf, un C₁₋₃-alkylbenzyle, ou 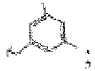 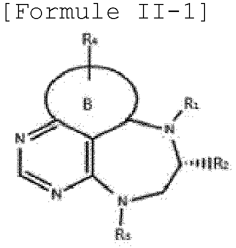
B formant un cycle à 6 à 10 chaînons conjointement avec 2 à 3 atomes C adjacents, le cycle pouvant contenir 1 à 3 atomes d'azote ;
R₁ étant -Boc ou un groupe benzyle ;
R₂ étant -CH₂-O-TIPS ; et
R₃ étant hydrogène, un groupe C₁₋₃-alkyle, un groupe allyle, -Tf, un groupe C₁₋₃-alkylbenzyle, ou 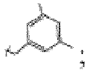 et
R₄ étant un groupe C₁₋₃-alkyle ou -CH₂-PMB ; 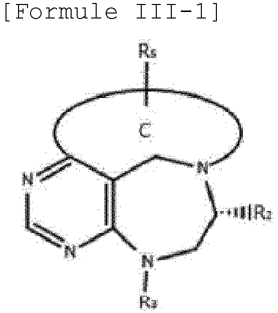
C formant un cycle à 10 à 14 chaînons conjointement avec les atomes N et C adjacents, le cycle pouvant contenir 1 à 3 atomes d'azote ;
R₂ étant -CH₂-O-TIPS ;
R₃ étant hydrogène, un groupe C₁₋₃-alkyle, un groupe allyle, -Tf, un groupe C₁₋₃-alkylbenzyle, ou 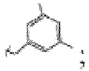 et
R₅ étant un groupe C₁₋₃-alkyle ou -CH₂-PMB ; 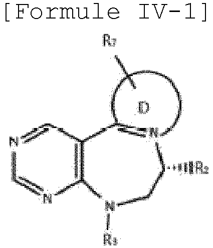
D formant un imide cyclique à 4 à 6 chaînons conjointement avec les atomes N et C adjacents, le cycle imide étant un cycle qui peut contenir 1 à 3 atomes d'azote ;
R₂ étant -CH₂-O-TIPS ;
R₃ étant hydrogène, un groupe C₁₋₃-alkyle, un groupe allyle, -Tf, un groupe C₁₋₃-alkylbenzyle, ou 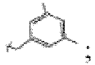
R₆ étant 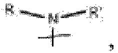 R et R' étant chacun indépendamment un groupe benzyle ou un groupe C₁₋₃-alkyle ;
R₇ étant un groupe benzyle, un groupe phényle, ou 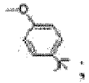 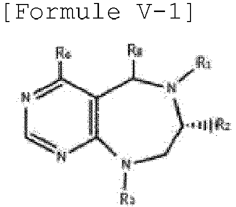
R₁ étant -Boc ou un groupe benzyle ;
R₂ étant -CH₂-O-TIPS ; et
R₃ étant hydrogène, un groupe C₁₋₃-alkyle, un groupe allyle, -Tf, un groupe C₁₋₃-alkylbenzyle, ou 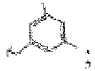
R₆ étant 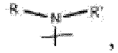 R et R' étant chacun indépendamment un groupe benzyle ou un groupe C₁₋₃-alkyle ; et
R₈ étant un groupe acétylène ; 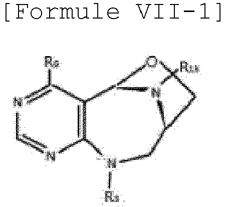
R₃ étant hydrogène, un groupe C₁₋₃-alkyle, un groupe allyle, -Tf, un groupe C₁₋₃-alkylbenzyle, ou 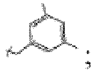
R₆ étant 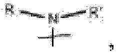 R et R' étant chacun indépendamment un groupe benzyle ou un groupe C₁₋₃-alkyle ; et
R₁₃ étant -Boc, -CONHBn, -m-Ns ; 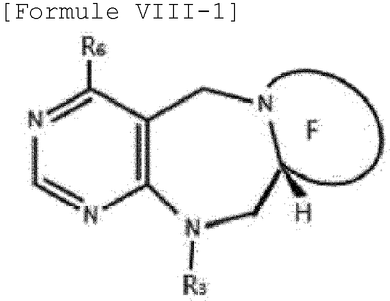
F étant -X-(CH₂)n-(O-CH₂)m- et formant un cycle hétérocyclique à 3 à 6 chaînons conjointement avec des atomes N et C adjacents, X étant 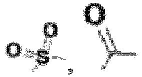 ou -CH₂-, et n et m étant respectivement un entier de 0 à 2 ;
R₃ étant hydrogène, un groupe C₁₋₃-alkyle, un groupe allyle, -Tf, un groupe C₁₋₃-alkylbenzyle, ou 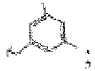
R₆ étant 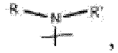 R et R' étant chacun indépendamment un groupe benzyle ou un groupe C₁₋₃-alkyle ; 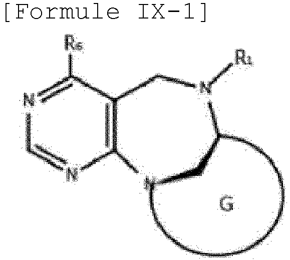
G formant un cycle hétérocyclique à 6 à 9 chaînons avec des atomes N et C adjacents, le cycle hétérocyclique étant un cycle hétérocyclique insaturé qui contient 2 à 3 atomes d'azote ;
R₁ étant -Boc ou un groupe benzyle ; et
R₆ étant 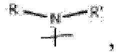 R et R' étant chacun indépendamment un groupe benzyle ou un groupe C₁₋₃-alkyle ;
ou sel pharmaceutiquement acceptable correspondant.

3. Composé selon la revendication 1, le composé étant représenté par l'une quelconque parmi les formules : 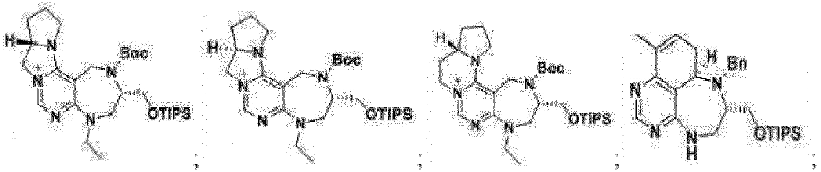 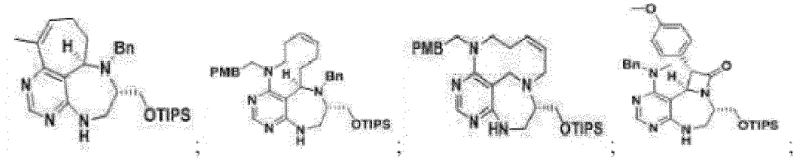 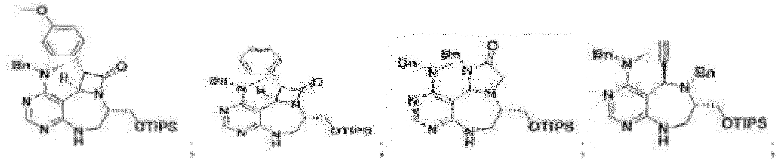 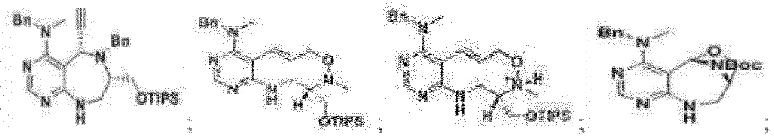 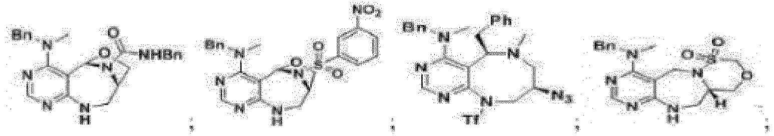 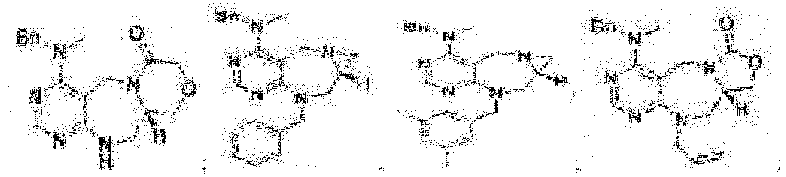 et 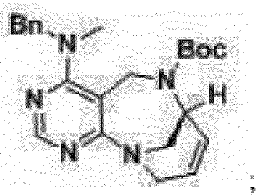 ou sel pharmaceutiquement acceptable correspondant.

4. Procédé pour la préparation du composé de type dérivé de pyrimidine selon la revendication 1 ou d'un sel pharmaceutiquement acceptable correspondant, **caractérisé en ce qu'**il comprend :
une étape 1 pour la préparation d'une substance intermédiaire, le tert-(S)-(1-(6-chloro-5-formylpyridine-4-yl)amino)-3-((triisopropylsilyl)oxy)propan-2-yl)carbamate ;
une étape la pour la préparation d'un produit de départ représenté par la formule 1 par mise en réaction de la substance intermédiaire préparée dans l'étape 1 avec une amine cyclique ou du tétrakis palladium ; et 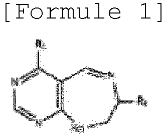
R₁ étant 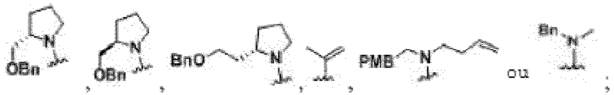
R₂ étant -CH₂-O-TIPS ;
une étape 2 pour la préparation d'un composé souhaité à partir du produit de départ préparé dans l'étape la.

5. Procédé pour la préparation selon la revendication 4, l'étape 2 comprenant au moins l'une des réactions suivantes sur le produit de départ de l'étape la : N-alkylation, débenzylation, réaction de substitution intramoléculaire, métathèse avec fermeture de cycle, réaction d'addition nucléophile, réaction d'allylation d'aminé, réaction de condensation avec le catalyseur de Grubbs, réaction de déprotection d'alcool, réaction d'addition nucléophile intramoléculaire, réaction de formation d'intermédiaire de type aziridium, réaction d'expansion de cycle nucléophile, et réaction de formation de cycle de tête de pont.

6. Composition pour une utilisation dans le traitement du cancer comprenant le composé de type dérivé de pyrimidine selon la revendication 1 ou un sel pharmaceutiquement acceptable correspondant en tant qu'un ingrédient actif.

7. Composition pour une utilisation selon la revendication 6, **caractérisé en ce que** le cancer est au moins l'un choisi dans le groupe constitué par un cancer du col de l'utérus, un cancer du poumon, un cancer pancréatique, un cancer du poumon non à petites cellules, un cancer du foie, un cancer du côlon, un cancer des os, un cancer de la peau, un cancer de la tête et du cou, un mélanome dans la peau ou dans l'œil, un cancer de l'utérus, un cancer de l'ovaire, un cancer rectal, un cancer gastrique, un cancer autour de l'anus, un cancer du côlon, un cancer du sein, un carcinome de la trompe de Fallope, un carcinome endométrial, un carcinome vaginal, un carcinome vulvaire, un cancer de l'œsophage, un cancer du petit intestin, un cancer de la glande endocrine, un cancer de la thyroïde, un cancer de la parathyroïde, un cancer adrénal, un sarcome des tissus mous, un cancer urétral, un cancer du pénis, un cancer de la prostate, un cancer de la vessie, un cancer du rein ou de l'uretère, un carcinome de cellules rénales, et un carcinome du pelvis rénal.

8. Composition pour une utilisation selon la revendication 6, **caractérisé en ce que** le cancer est un cancer du col de l'utérus.
